# EUROPEAN PATENT APPLICATION

(11) **EP 4 008 788 A1**
(43) Date of publication of application: **08.06.2022**
(21) Application number: 20211616.6
(22) Date of filing: 03.12.2020
(51) Int. Cl.: C12N 15/86, A61K 48/00

(54) **LENTIVIRAL VECTOR TECHNOLOGY FOR INNER EAR GENE THERAPY**

(71) Applicant: Medizinische Hochschule Hannover, 30625 Hannover (DE)
(72) Inventor: SCHAMBACH, Axel, 30625 Hannover (DE); MORGAN, Michael, 30855 Langenhagen (DE); SCHOTT, Juliane, 30625 Hannover (DE); BÜNING, Hildegard, 30625 Hannover (DE); STAECKER, Hinrich, Leawood, Kansas 66209 (US); WARNECKE, Athanasia, 30938 Burgwedel (DE)
(74) Representative: Moré, Solveig Helga

(57) **Abstract**

The present invention relates to the field of gene therapy for the treatment of sensorineural hearing loss. In particular, the invention discloses a composition comprising a 3^{rd} generation lentiviral vector pseudotyped with a viral envelope glycoprotein capable of binding to a receptor expressed in a cell of the inner ear for use in the treatment or prevention of sensorineural hearing loss, wherein said composition has a viral titer of at least 10⁷TU/mL and is administered to the inner ear of a subject suffering from or in danger to undergo sensorineural hearing loss. The viral envelope glycoprotein is capable of binding to a receptor selected from the group consisting of the LDL-receptor and LDL-R family members, the SLC1A5-receptor, the Pit1/2-receptor and the PIRYV-G-receptor. Preferably, the viral glycoprotein is MARAV-G, COCV-G, VSV-G, VSV-G ts or PIRYV-G, most preferably, MARAV-G. The lentiviral vector further comprises a cargo sequence comprising, e.g. a protein-coding gene, a miRNA, an shRNA, a IncRNA or an sgRNA, wherein expression of said cargo sequence in the cell of the inner ear is capable of reducing, eliminating or preventing at least a symptom of sensorineural hearing loss in the subject. The invention further discloses a method for treating sensorineural hearing loss in a subject in need thereof.

SEQUENCE LISTING

<110> Medizinische Hochschule Hannover

<120> Lentiviral vector technology for inner ear gene therapy

<130> 12747 P 6576 EP

<160> 27

<170> BiSSAP 1.3.6

<210> 1
<211> 4471
<212> DNA
<213> Artificial Sequence

<220>
<223> lentiviral vector (RRL.PPT.CAGs.dTomato.pre) (nucleic acid
      sequence)

<220>
<221> promoter
<222> 1..232
<223> /function="RSV promoter"

<220>
<221> 5'UTR
<222> 233..414
<223> /note="R/U5"

<220>
<221> 5'UTR
<222> 415..432
<223> /note="Primer bindings site (PBS)"

<220>
<221> 5'UTR
<222> 569..933
<223> /note="dGag"

<220>
<221> 5'UTR
<222> 1079..1320
<223> /note="Rev-responsive element (RRE)"

<220>
<221> 5'UTR
<222> 1804..1927
<223> /note="polypurine tract (PPT)"

<220>
<221> enhancer
<222> 1959..2338
<223> /note="Human Cytomegalovirus Immediate-Early (CMVie)"

<220>
<221> promoter
<222> 1962..2817
<223> /note="CAGs"

<220>

<221> promoter
<222> 2340..2617
<223> /note="Chicken beta-Actin promoter"

<220>
<221> gene
<222> 2843..3547
<223> /note="Transgene dTomato"

<220>
<221> 3'UTR
<222> 3548..4156
<223> /note="Postranscriptional Regulatory Element (PRE)"

<220>
<221> LTR
<222> 4238..4471
<223> /note="self-inactivating (SIN) LTR"

<400> 1
ttaatgtagt cttatgcaat actcttgtag tcttgcaaca tggtaacgat gagttagcaa 60

catgccttac aaggagagaa aaagcaccgt gcatgccgat tggtggaagt aaggtggtac 120

gatcgtgcct tattaggaag gcaacagacg ggtctgacat ggattggacg aaccactgaa 180

ttgccgcatt gcagagatat tgtatttaag tgcctagctc gatacaataa acgggtctct 240

ctggttagac cagatctgag cctgggagct ctctggctaa ctagggaacc cactgcttaa 300

gcctcaataa agcttgcctt gagtgcttca agtagtgtgt gcccgtctgt tgtgtgactc 360

tggtaactag agatccctca gaccctttta gtcagtgtgg aaaatctcta gcagtggcgc 420

ccgaacaggg acttgaaagc gaaagggaaa ccagaggagc tctctcgacg caggactcgg 480

cttgctgaag cgcgcacggc aagaggcgag gggcggcgac tggtgagtac gccaaaaatt 540

ttgactagcg gaggctagaa ggagagagat gggtgcgaga gcgtcagtat taagcggggg 600

agaattagat cgcgatggga aaaaattcgg ttaaggccag ggggaaagaa aaaatataaa 660

ttaaaacata tagtatgggc aagcagggag ctagaacgat tcgcagttaa tcctggcctg 720

ttagaaacat cagaaggctg tagacaaata ctgggacagc tacaaccatc ccttcagaca 780

ggatcagaag aacttagatc attatataat acagtagcaa ccctctattg tgtgcatcaa 840

aggatagaga taaaagacac caaggaagct ttagacaaga tagaggaaga gcaaaacaaa 900

agtaagacca ccgcacagca agcggccgct gatcttcaga cctggaggag gagatatgag 960

ggacaattgg agaagtgaat tatataaata taaagtagta aaaattgaac cattaggagt 1020

agcacccacc aaggcaaaga gaagagtggt gcagagagaa aaaagagcag tgggaatagg 1080

agctttgttc cttgggttct tgggagcagc aggaagcact atgggcgcag cctcaatgac 1140

gctgacggta caggccagac aattattgtc tggtatagtg cagcagcaga acaatttgct 1200

gagggctatt gaggcgcaac agcatctgtt gcaactcaca gtctggggca tcaagcagct 1260

ccaggcaaga atcctggctg tggaaagata cctaaaggat caacagctcc tggggatttg 1320

gggttgctct ggaaaactca tttgcaccac tgctgtgcct tggaatgcta gttggagtaa 1380

taaatctctg gaacagattt ggaatcacac gacctggatg gagtgggaca gagaaattaa 1440

caattacaca agcttaatac actccttaat tgaagaatcg caaaaccagc aagaaaagaa 1500

tgaacaagaa ttattggaat tagataaatg ggcaagtttg tggaattggt ttaacataac 1560

aaattggctg tggtatataa aattattcat aatgatagta ggaggcttgg taggtttaag 1620

aatagttttt gctgtacttt ctatagtgaa tagagttagg cagggatatt caccattatc 1680

gtttcagacc cacctcccaa ccccgagggg acccgacagg cccgaaggaa tagaagaaga 1740

aggtggagag agagacagag acagatccat tcgattagtg aacggatctc gacggtatcg 1800

gttaactttt aaaagaaaag gggggattgg ggggtacagt gcaggggaaa gaatagtaga 1860

cataatagca acagacatac aaactaaaga attacaaaaa caaattacaa aaattcaaaa 1920

ttttatcgat cacgagacta gcctcgagct agcccgtcga cattgattat tgactagtta 1980

ttaatagtaa tcaattacgg ggtcattagt tcatagccca tatatggagt tccgcgttac 2040

ataacttacg gtaaatggcc cgcctggctg accgcccaac gacccccgcc cattgacgtc 2100

aataatgacg tatgttccca tagtaacgcc aatagggact ttccattgac gtcaatgggt 2160

ggagtattta cggtaaactg cccacttggc agtacatcaa gtgtatcata tgccaagtac 2220

gccccctatt gacgtcaatg acggtaaatg gcccgcctgg cattatgccc agtacatgac 2280

cttatgggac tttcctactt ggcagtacat ctacgtatta gtcatcgcta ttaccatggt 2340

cgaggtgagc cccacgttct gcttcactct ccccatctcc cccccctccc cacccccaat 2400

tttgtattta tttatttttt aattattttg tgcagcgatg ggggcggggg gggggggggg 2460

gcgcgcgcca ggcggggcgg ggcggggcga ggggcggggc ggggcgaggc ggagaggtgc 2520

ggcggcagcc aatcagagcg gcgcgctccg aaagtttcct tttatggcga ggcggcggcg 2580

gcggcggccc tataaaaagc gaagcgcgcg gcgggcggga gtcgctgcgc gctgccttcg 2640

ccccgtgccc cgctccgcgc cgcctcgcgc cgcccgcccc ggctctgact gaccgcgtta 2700

ctcccacagg tgagcgggcg ggacggccct tctcctccgg gctgtaatta gcgcttggtt 2760

taatgacggc ttgtttcttt tctgtggctg cgtgaaagcc ttgaggggct ccgggaggac 2820

cggtgaattc ggatccgcca ccatggtgag caagggcgag gaggtcatca aagagttcat 2880

gcgcttcaag gtgcgcatgg agggctccat gaacggccac gagttcgaga tcgagggcga 2940

gggcgagggc cgcccctacg agggcaccca gaccgccaag ctgaaggtga ccaagggcgg 3000

ccccctgccc ttcgcctggg acatcctgtc cccccagttc atgtacggct ccaaggcgta 3060

cgtgaagcac cccgccgaca tccccgatta caagaagctg tccttccccg agggcttcaa 3120

gtgggagcgc gtgatgaact tcgaggacgg cggtctggtg accgtgaccc aggactcctc 3180

cctgcaggac ggcacgctga tctacaaggt gaagatgcgc ggcaccaact tcccccccga 3240

cggccccgta atgcagaaga agaccatggg ctgggaggcc tccaccgagc gcctgtaccc 3300

ccgcgacggc gtgctgaagg gcgagatcca ccaggccctg aagctgaagg acggcggcca 3360

ctacctggtg gagttcaaga ccatctacat ggccaagaag cccgtgcaac tgcccggcta 3420

ctactacgtg gacaccaagc tggacatcac ctcccacaac gaggactaca ccatcgtgga 3480

acagtacgag cgctccgagg gccgccacca cctgttcctg tacggcatgg acgagctgta 3540

caagtaaagc ggccgcgtcg acaatcaacc tctggattac aaaatttgtg aaagattgac 3600

tggtattctt aactatgttg ctccttttac gctatgtgga tacgctgctt taatgccttt 3660

gtatcatgct attgcttccc gtatggcttt cattttctcc tccttgtata aatcctggtt 3720

gctgtctctt tatgaggagt tgtggcccgt tgtcaggcaa cgtggcgtgg tgtgcactgt 3780

gtttgctgac gcaaccccca ctggttgggg cattgccacc acctgtcagc tcctttccgg 3840

gactttcgct ttccccctcc ctattgccac ggcggaactc atcgccgcct gccttgcccg 3900

ctgctggaca ggggctcggc tgttgggcac tgacaattcc gtggtgttgt cggggaagct 3960

gacgtccttt ccatggctgc tcgcctgtgt tgccacctgg attctgcgcg ggacgtcctt 4020

ctgctacgtc ccttcggccc tcaatccagc ggaccttcct tcccgcggcc tgctgccggc 4080

tctgcggcct cttccgcgtc ttcgccttcg ccctcagacg agtcggatct ccctttgggc 4140

cgcctccccg cctggaattc gagctcggta cctttaagac caatgactta caaggcagct 4200

gtagatctta gccacttttt aaaagaaaag gggggactgg aagggctaat tcactcccaa 4260

cgaagacaag atctgctttt tgcttgtact gggtctctct ggttagacca gatctgagcc 4320

tgggagctct ctggctaact agggaaccca ctgcttaagc ctcaataaag cttgccttga 4380

gtgcttcaag tagtgtgtgc ccgtctgttg tgtgactctg gtaactagag atccctcaga 4440

cccttttagt cagtgtggaa aatctctagc a 4471

<210> 2
<211> 1536
<212> DNA
<213> Artificial Sequence

<220>
<223> Vesicular stomatitis virus (VSV)-G ts (nucleic acid sequence)

<400> 2
atgaagtgcc ttttgtactt agccttttta ttcattgggg tgaattgcaa gttcaccata 60

gtttttccac acaaccaaaa aggaaactgg aaaaatgttc cttctaatta ccattattgc 120

ccgtcaagct cagatttaaa ttggcataat gacttaatag gcacagcctt acaagtcaaa 180

atgcccaaga gtcacaaggc tattcaagca gacggttgga tgtgtcatgc ttccaaatgg 240

gtcactactt gtgatttccg ctggtatgga ccgaagtata taacacattc catccgatcc 300

ttcactccat ctgtagaaca atgcaaggaa agcattgaac aaacgaaaca aggaacttgg 360

ctgaatccag gcttccctcc tcaaagttgt ggatatgcaa ctgtgacgga tgccgaagca 420

gtgattgtcc aggtgactcc tcaccatgtg ctggttgatg aatacacagg agaatgggtt 480

gattcacagt tcatcaacgg aaaatgcagc aattacatat gccccactgt ccataactct 540

acaacctggc attctgacta taaggtcaaa gggctatgtg attctaacct catttccatg 600

gacatcacct tcttctcaga ggacggagag ctatcatccc tgggaaagga gggcacaggg 660

ttcagaagta actactttgc ttatgaaaat ggaggcaagg cctgcaaaat gcaatactgc 720

aagcattggg gagtcagact cccatcaggt gtctggttcg agatggctga taaggatctc 780

tttgctgcag ccagattccc tgaatgccca gaagggtcaa gtatctctgc tccatctcag 840

acctcagtgg atgtaagtct aattcaggac gttgagagga tcttggatta ttccctctgc 900

caagaaacct ggagcaaaat cagagcgggt cttccaatct ctccagtgga tctcagctat 960

cttgctccta aaaacccagg aaccggtcct gctttcacca taatcaatgg taccctaaaa 1020

tactttgaga ccagatacat cagagtcgat attgctgctc caatcctctc aagaatggtc 1080

ggaatgatca gtggaactac cgcagaaagg gaactgtggg atgactgggc accatatgaa 1140

gacgtggaaa ttggacccaa tggagttctg aggaccagtt caggatataa gtttccttta 1200

tacatgattg gacatggtat gttggactcc gatcttcatc ttagctcaaa ggctcaggtg 1260

ttcgaacatc ctcacattca agacgctgct tcgcaacttc ctgatgatga gagtttattt 1320

tttggtgata ctgggctatc caaaaatcca atcgagcttg tagaaggttg gttcagtagt 1380

tggaaaagct ctattgcctc ttttttcttt atcatagggt taatcattgg actattcttg 1440

gttctccgag ttggtatcca tctttgcatt aaattaaagc acaccaagaa aagacagatt 1500

tatacagaca tagagatgaa ccgacttgga aagtaa 1536

<210> 3
<211> 511
<212> PRT
<213> Artificial Sequence

<220>
<223> Vesicular stomatitis virus (VSV)-G ts (amino acid sequence)

<400> 3
Met Lys Cys Leu Leu Tyr Leu Ala Phe Leu Phe Ile Gly Val Asn Cys
1               5                   10                  15
Lys Phe Thr Ile Val Phe Pro His Asn Gln Lys Gly Asn Trp Lys Asn
            20                  25                  30
Val Pro Ser Asn Tyr His Tyr Cys Pro Ser Ser Ser Asp Leu Asn Trp
        35                  40                  45
His Asn Asp Leu Ile Gly Thr Ala Leu Gln Val Lys Met Pro Lys Ser
    50                  55                  60

His Lys Ala Ile Gln Ala Asp Gly Trp Met Cys His Ala Ser Lys Trp
65                  70                  75                  80
Val Thr Thr Cys Asp Phe Arg Trp Tyr Gly Pro Lys Tyr Ile Thr His
                85                  90                  95
Ser Ile Arg Ser Phe Thr Pro Ser Val Glu Gln Cys Lys Glu Ser Ile
            100                 105                 110
Glu Gln Thr Lys Gln Gly Thr Trp Leu Asn Pro Gly Phe Pro Pro Gln
        115                 120                 125
Ser Cys Gly Tyr Ala Thr Val Thr Asp Ala Glu Ala Val Ile Val Gln
    130                 135                 140
Val Thr Pro His His Val Leu Val Asp Glu Tyr Thr Gly Glu Trp Val
145                 150                 155                 160
Asp Ser Gln Phe Ile Asn Gly Lys Cys Ser Asn Tyr Ile Cys Pro Thr
                165                 170                 175
Val His Asn Ser Thr Thr Trp His Ser Asp Tyr Lys Val Lys Gly Leu
            180                 185                 190
Cys Asp Ser Asn Leu Ile Ser Met Asp Ile Thr Phe Phe Ser Glu Asp
        195                 200                 205
Gly Glu Leu Ser Ser Leu Gly Lys Glu Gly Thr Gly Phe Arg Ser Asn
    210                 215                 220
Tyr Phe Ala Tyr Glu Asn Gly Gly Lys Ala Cys Lys Met Gln Tyr Cys
225                 230                 235                 240
Lys His Trp Gly Val Arg Leu Pro Ser Gly Val Trp Phe Glu Met Ala
                245                 250                 255
Asp Lys Asp Leu Phe Ala Ala Ala Arg Phe Pro Glu Cys Pro Glu Gly
            260                 265                 270
Ser Ser Ile Ser Ala Pro Ser Gln Thr Ser Val Asp Val Ser Leu Ile
        275                 280                 285
Gln Asp Val Glu Arg Ile Leu Asp Tyr Ser Leu Cys Gln Glu Thr Trp
    290                 295                 300
Ser Lys Ile Arg Ala Gly Leu Pro Ile Ser Pro Val Asp Leu Ser Tyr
305                 310                 315                 320
Leu Ala Pro Lys Asn Pro Gly Thr Gly Pro Ala Phe Thr Ile Ile Asn
                325                 330                 335
Gly Thr Leu Lys Tyr Phe Glu Thr Arg Tyr Ile Arg Val Asp Ile Ala
            340                 345                 350
Ala Pro Ile Leu Ser Arg Met Val Gly Met Ile Ser Gly Thr Thr Ala
        355                 360                 365
Glu Arg Glu Leu Trp Asp Asp Trp Ala Pro Tyr Glu Asp Val Glu Ile
    370                 375                 380
Gly Pro Asn Gly Val Leu Arg Thr Ser Ser Gly Tyr Lys Phe Pro Leu
385                 390                 395                 400
Tyr Met Ile Gly His Gly Met Leu Asp Ser Asp Leu His Leu Ser Ser
                405                 410                 415
Lys Ala Gln Val Phe Glu His Pro His Ile Gln Asp Ala Ala Ser Gln
            420                 425                 430
Leu Pro Asp Asp Glu Ser Leu Phe Phe Gly Asp Thr Gly Leu Ser Lys
        435                 440                 445
Asn Pro Ile Glu Leu Val Glu Gly Trp Phe Ser Ser Trp Lys Ser Ser
    450                 455                 460
Ile Ala Ser Phe Phe Phe Ile Ile Gly Leu Ile Ile Gly Leu Phe Leu
465                 470                 475                 480
Val Leu Arg Val Gly Ile His Leu Cys Ile Lys Leu Lys His Thr Lys
                485                 490                 495
Lys Arg Gln Ile Tyr Thr Asp Ile Glu Met Asn Arg Leu Gly Lys
            500                 505                 510

<210> 4
<211> 1539
<212> DNA
<213> Cocal virus

<220>
<223> Cocal virus (COCV)-G (nucleic acid sequence)

<400> 4
atgaatttcc tactcttgac atttattgtg ttgccgttgt gcagccacgc caagttctcc 60

attgtattcc ctcaaagcca aaaaggcaat tggaagaatg taccatcatc ttaccattac 120

tgcccttcaa gttcggatca aaactggcac aatgatttgc ttggaatcac aatgaaagtc 180

aaaatgccca aaacacacaa agctattcaa gcagacgggt ggatgtgtca tgctgccaaa 240

tggatcacta cctgtgactt tcgctggtac ggacccaaat acatcactca ctccattcat 300

tccatccagc ctacttcaga gcagtgtaaa gaaagcatca agcaaacaaa acaaggtact 360

tggatgagtc ctggcttccc tccacagaac tgcgggtatg caacagtaac agactctgtc 420

gctgttgtcg tccaagccac tcctcatcat gtcttggttg atgaatatac tggagaatgg 480

atcgactctc aattccccaa cgggaaatgt gaaaccgaag agtgcgagac cgtccacaac 540

tctaccgtat ggtactctga ctacaaagta actggattat gtgacgcaac tctggtagac 600

acagagatca ccttcttctc tgaagatggc aaaaaagaat ctatcgggaa gcccaacaca 660

ggctatagga gcaactactt cgcttatgag aaaggggaca aagtatgtaa aatgaactac 720

tgcaagcatg cgggtgtgag gttgccttcc ggggtttggt ttgagtttgt ggatcaggat 780

gtctacgccg ccgccaaact tccagaatgc cccgttggtg ccactatctc cgctccgaca 840

cagacctctg ttgacgtaag tctcattcta gatgtagaga gaattttaga ttactctctg 900

tgtcaagaga catggagcaa gatccggtcc aaacagccag tatcccctgt tgaccttagt 960

tacttggccc ccaagaatcc tgggaccgga ccggcattca caatcatcaa tggcactctg 1020

aagtactttg agaccagata cattcggatt gatatagaca atccaatcat ctccaagatg 1080

gtggggaaaa taagtggcag tcaaacagaa cgagaattgt ggacagagtg gttcccctac 1140

gagggtgtcg agatagggcc aaatgggatt ctcaaaaccc ctacaggata caaattccca 1200

ctcttcatga taggacacgg gatgctagat tccgacttgc acaagacgtc ccaagcagag 1260

gtctttgaac atcctcacct tgcagaagca ccaaagcagt tgccggagga ggagacttta 1320

ttttttggtg acacaggaat ctccaaaaat ccggtcgaac tgattgaagg gtggtttagt 1380

agttggaaga gcactgtagt cacctttttc tttgccatag gagtatttat actactgtat 1440

gtagtggcca gaattgtgat cgcagtgaga tacagatatc aaggctcaaa taacaaaaga 1500

atttacaatg atattgagat gagcagattt agaaaatga 1539

<210> 5
<211> 512
<212> PRT
<213> Cocal virus

<220>
<223> Cocal virus (COCV)-G (amino acid sequence)

<400> 5
Met Asn Phe Leu Leu Leu Thr Phe Ile Val Leu Pro Leu Cys Ser His
1               5                   10                  15
Ala Lys Phe Ser Ile Val Phe Pro Gln Ser Gln Lys Gly Asn Trp Lys
            20                  25                  30
Asn Val Pro Ser Ser Tyr His Tyr Cys Pro Ser Ser Ser Asp Gln Asn
        35                  40                  45
Trp His Asn Asp Leu Leu Gly Ile Thr Met Lys Val Lys Met Pro Lys
    50                  55                  60
Thr His Lys Ala Ile Gln Ala Asp Gly Trp Met Cys His Ala Ala Lys
65                  70                  75                  80
Trp Ile Thr Thr Cys Asp Phe Arg Trp Tyr Gly Pro Lys Tyr Ile Thr
                85                  90                  95
His Ser Ile His Ser Ile Gln Pro Thr Ser Glu Gln Cys Lys Glu Ser
            100                 105                 110
Ile Lys Gln Thr Lys Gln Gly Thr Trp Met Ser Pro Gly Phe Pro Pro
        115                 120                 125
Gln Asn Cys Gly Tyr Ala Thr Val Thr Asp Ser Val Ala Val Val Val
    130                 135                 140
Gln Ala Thr Pro His His Val Leu Val Asp Glu Tyr Thr Gly Glu Trp
145                 150                 155                 160
Ile Asp Ser Gln Phe Pro Asn Gly Lys Cys Glu Thr Glu Glu Cys Glu
                165                 170                 175
Thr Val His Asn Ser Thr Val Trp Tyr Ser Asp Tyr Lys Val Thr Gly
            180                 185                 190
Leu Cys Asp Ala Thr Leu Val Asp Thr Glu Ile Thr Phe Phe Ser Glu
        195                 200                 205
Asp Gly Lys Lys Glu Ser Ile Gly Lys Pro Asn Thr Gly Tyr Arg Ser
    210                 215                 220
Asn Tyr Phe Ala Tyr Glu Lys Gly Asp Lys Val Cys Lys Met Asn Tyr
225                 230                 235                 240
Cys Lys His Ala Gly Val Arg Leu Pro Ser Gly Val Trp Phe Glu Phe
                245                 250                 255
Val Asp Gln Asp Val Tyr Ala Ala Ala Lys Leu Pro Glu Cys Pro Val
            260                 265                 270
Gly Ala Thr Ile Ser Ala Pro Thr Gln Thr Ser Val Asp Val Ser Leu
        275                 280                 285
Ile Leu Asp Val Glu Arg Ile Leu Asp Tyr Ser Leu Cys Gln Glu Thr
    290                 295                 300
Trp Ser Lys Ile Arg Ser Lys Gln Pro Val Ser Pro Val Asp Leu Ser
305                 310                 315                 320
Tyr Leu Ala Pro Lys Asn Pro Gly Thr Gly Pro Ala Phe Thr Ile Ile
                325                 330                 335
Asn Gly Thr Leu Lys Tyr Phe Glu Thr Arg Tyr Ile Arg Ile Asp Ile
            340                 345                 350
Asp Asn Pro Ile Ile Ser Lys Met Val Gly Lys Ile Ser Gly Ser Gln
        355                 360                 365
Thr Glu Arg Glu Leu Trp Thr Glu Trp Phe Pro Tyr Glu Gly Val Glu
    370                 375                 380
Ile Gly Pro Asn Gly Ile Leu Lys Thr Pro Thr Gly Tyr Lys Phe Pro
385                 390                 395                 400
Leu Phe Met Ile Gly His Gly Met Leu Asp Ser Asp Leu His Lys Thr
                405                 410                 415
Ser Gln Ala Glu Val Phe Glu His Pro His Leu Ala Glu Ala Pro Lys
            420                 425                 430
Gln Leu Pro Glu Glu Glu Thr Leu Phe Phe Gly Asp Thr Gly Ile Ser
        435                 440                 445
Lys Asn Pro Val Glu Leu Ile Glu Gly Trp Phe Ser Ser Trp Lys Ser
    450                 455                 460
Thr Val Val Thr Phe Phe Phe Ala Ile Gly Val Phe Ile Leu Leu Tyr

465                 470                 475                 480
Val Val Ala Arg Ile Val Ile Ala Val Arg Tyr Arg Tyr Gln Gly Ser
                485                 490                 495
Asn Asn Lys Arg Ile Tyr Asn Asp Ile Glu Met Ser Arg Phe Arg Lys
            500                 505                 510

<210> 6
<211> 1539
<212> DNA
<213> Maraba virus

<220>
<223> Maraba virus (MARAV)-G (nucleic acid sequence)

<400> 6
atgttgagac tttttctctt ttgtttcttg gccttaggag cccactccaa atttactata      60

gtattccctc atcatcaaaa agggaattgg aagaatgtgc cttccacata tcattattgc     120

ccttctagtt ctgaccagaa ttggcataat gatttgactg gagttagtct tcatgtgaaa     180

attcccaaaa gtcacaaagc tatacaagca gatggctgga tgtgccacgc tgctaaatgg     240

gtgactactt gtgacttcag atggtacgga cccaaataca tcacgcattc catacactct     300

atgtcaccca ccctagaaca gtgcaagacc agtattgagc agacaaagca aggagtttgg     360

attaatccag gctttccccc tcaaagctgc ggatatgcta cagtgacgga tgcagaggtg     420

gttgttgtac aagcaacacc tcatcatgtg ttggttgatg agtacacagg agaatggatt     480

gactcacaat tggtgggggg caaatgttcc aaggaggttt gtcaaacggt tcacaactcg     540

accgtgtggc atgctgatta caagattaca gggctgtgcg agtcaaatct ggcatcagtg     600

gatatcacct tcttctctga ggatggtcaa aagacgtctt tgggaaaacc gaacactgga     660

ttcaggagta atcactttgc ttacgaaagt ggagagaagg catgccgtat gcagtactgc     720

acacaatggg ggatccgact accttctgga gtatggtttg aattagtgga caaagatctc     780

ttccaggcgg caaaattgcc tgaatgtcct agaggatcca gtatctcagc tccttctcag     840

acttctgtgg atgttagttt gatacaagac gtagagagga tcttagatta ctctctatgc     900

caggagacgt ggagtaagat acgagccaag cttcctgtat ctccagtaga tctgagttat     960

ctcgccccaa aaaatccagg gagcggaccg gccttcacta tcattaatgg cactttgaaa    1020

tatttcgaaa caagatacat cagagttgac ataagtaatc ccatcatccc tcacatggtg    1080

ggaacaatga gtggaaccac gactgagcgt gaattgtgga atgattggta tccatatgaa    1140

gacgtagaga ttggtccaaa tggggtgttg aaaactccca ctggtttcaa gtttccgctg    1200

tacatgattg ggcacggaat gttggattcc gatctccaca aatcctccca ggctcaagtc    1260

ttcgaacatc cacacgcaaa ggacgctgca tcacagcttc ctgatgatga gactttattt    1320

tttggtgaca caggactatc aaaaaaccca gtagagttag tagaaggctg gttcagtagc    1380

tggaagagca cattggcatc gttctttctg attataggct tgggggttgc attaatcttc 1440

atcattcgaa ttattgttgc gattcgctat aaatacaagg ggaggaagac ccaaaaaatt 1500

tacaatgatg tcgagatgag tcgattggga aataaataa 1539

<210> 7
<211> 512
<212> PRT
<213> Maraba virus

<220>
<223> Maraba virus (MARAV)-G (amino acid sequence)

<400> 7
   Met Leu Arg Leu Phe Leu Phe Cys Phe Leu Ala Leu Gly Ala His Ser
   1               5                   10                  15
   Lys Phe Thr Ile Val Phe Pro His His Gln Lys Gly Asn Trp Lys Asn
               20                  25                  30
   Val Pro Ser Thr Tyr His Tyr Cys Pro Ser Ser Ser Asp Gln Asn Trp
           35                  40                  45
   His Asn Asp Leu Thr Gly Val Ser Leu His Val Lys Ile Pro Lys Ser
       50                  55                  60
   His Lys Ala Ile Gln Ala Asp Gly Trp Met Cys His Ala Ala Lys Trp
   65                  70                  75                  80
   Val Thr Thr Cys Asp Phe Arg Trp Tyr Gly Pro Lys Tyr Ile Thr His
                   85                  90                  95
   Ser Ile His Ser Met Ser Pro Thr Leu Glu Gln Cys Lys Thr Ser Ile
               100                 105                 110
   Glu Gln Thr Lys Gln Gly Val Trp Ile Asn Pro Gly Phe Pro Pro Gln
           115                 120                 125
   Ser Cys Gly Tyr Ala Thr Val Thr Asp Ala Glu Val Val Val Val Gln
       130                 135                 140
   Ala Thr Pro His His Val Leu Val Asp Glu Tyr Thr Gly Glu Trp Ile
   145                 150                 155                 160
   Asp Ser Gln Leu Val Gly Gly Lys Cys Ser Lys Glu Val Cys Gln Thr
                   165                 170                 175
   Val His Asn Ser Thr Val Trp His Ala Asp Tyr Lys Ile Thr Gly Leu
               180                 185                 190
   Cys Glu Ser Asn Leu Ala Ser Val Asp Ile Thr Phe Phe Ser Glu Asp
           195                 200                 205
   Gly Gln Lys Thr Ser Leu Gly Lys Pro Asn Thr Gly Phe Arg Ser Asn
       210                 215                 220
   His Phe Ala Tyr Glu Ser Gly Glu Lys Ala Cys Arg Met Gln Tyr Cys
   225                 230                 235                 240
   Thr Gln Trp Gly Ile Arg Leu Pro Ser Gly Val Trp Phe Glu Leu Val
                   245                 250                 255
   Asp Lys Asp Leu Phe Gln Ala Ala Lys Leu Pro Glu Cys Pro Arg Gly
               260                 265                 270
   Ser Ser Ile Ser Ala Pro Ser Gln Thr Ser Val Asp Val Ser Leu Ile
           275                 280                 285
   Gln Asp Val Glu Arg Ile Leu Asp Tyr Ser Leu Cys Gln Glu Thr Trp
       290                 295                 300
   Ser Lys Ile Arg Ala Lys Leu Pro Val Ser Pro Val Asp Leu Ser Tyr
   305                 310                 315                 320
   Leu Ala Pro Lys Asn Pro Gly Ser Gly Pro Ala Phe Thr Ile Ile Asn
                   325                 330                 335
   Gly Thr Leu Lys Tyr Phe Glu Thr Arg Tyr Ile Arg Val Asp Ile Ser
               340                 345                 350
   Asn Pro Ile Ile Pro His Met Val Gly Thr Met Ser Gly Thr Thr Thr
   355                 360                 365
   Glu Arg Glu Leu Trp Asn Asp Trp Tyr Pro Tyr Glu Asp Val Glu Ile
       370                 375                 380
   Gly Pro Asn Gly Val Leu Lys Thr Pro Thr Gly Phe Lys Phe Pro Leu
   385                 390                 395                 400
   Tyr Met Ile Gly His Gly Met Leu Asp Ser Asp Leu His Lys Ser Ser
                   405                 410                 415
   Gln Ala Gln Val Phe Glu His Pro His Ala Lys Asp Ala Ala Ser Gln
               420                 425                 430
   Leu Pro Asp Asp Glu Thr Leu Phe Phe Gly Asp Thr Gly Leu Ser Lys
           435                 440                 445
   Asn Pro Val Glu Leu Val Glu Gly Trp Phe Ser Ser Trp Lys Ser Thr
       450                 455                 460
   Leu Ala Ser Phe Phe Leu Ile Ile Gly Leu Gly Val Ala Leu Ile Phe
   465                 470                 475                 480
   Ile Ile Arg Ile Ile Val Ala Ile Arg Tyr Lys Tyr Lys Gly Arg Lys
                   485                 490                 495
   Thr Gln Lys Ile Tyr Asn Asp Val Glu Met Ser Arg Leu Gly Asn Lys
               500                 505                 510

<210> 8
<211> 1698
<212> DNA
<213> RD114 retrovirus

<220>
<223> RD114 glycoprotein (nucleic acid sequence)

<400> 8
atgaaactcc caacaggaat ggtcatttta tgtagcctaa taatagttcg ggcagggttt      60

gacgaccccc gcaaggctat cgcattagta caaaaacaac atggtaaacc atgcgaatgc     120

agcggagggc aggtatccga ggccccaccg aactccatcc aacaggtaac ttgcccaggc     180

aagacggcct acttaatgac caaccaaaaa tggaaatgca gagtcactcc aaaaaatctc     240

acccctagcg ggggagaact ccagaactgc ccctgtaaca ctttccagga ctcgatgcac     300

agttcttgtt atactgaata ccggcaatgc agggcgaata ataagacata ctacacggcc     360

accttgctta aaatacggtc tgggagcctc aacgaggtac agatattaca aaaccccaat     420

cagctcctac agtccccttg taggggctct ataaatcagc ccgtttgctg gagtgccaca     480

gcccccatcc atatctccga tggtggagga cccctcgata ctaagagagt gtggacagtc     540

caaaaaaggc tagaacaaat tcataaggct atgcatcctg aacttcaata ccacccctta     600

gccctgccca aagtcagaga tgaccttagc cttgatgcac ggacttttga tatcctgaat     660

accactttta ggttactcca gatgtccaat tttagccttg cccaagattg ttggctctgt     720

ttaaaactag gtacccctac ccctcttgcg atacccactc cctctttaac ctactcccta     780

gcagactccc tagcgaatgc ctcctgtcag attatacctc ccctcttggt tcaaccgatg     840

cagttctcca actcgtcctg tttatcttcc cctttcatta acgatacgga acaaatagac     900

ttaggtgcag tcacctttac taactgcacc tctgtagcca atgtcagtag tcctttatgt     960

gccctaaacg ggtcagtctt cctctgtgga aataacatgg catacaccta tttaccccaa 1020

aactggacag gactttgcgt ccaagcctcc ctcctccccg acattgacat catcccgggg 1080

gatgagccag tccccattcc tgccattgat cattatatac atagacctaa acgagctgta 1140

cagttcatcc ctttactagc tggactggga atcaccgcag cattcaccac cggagctaca 1200

ggcctaggtg tctccgtcac ccagtataca aaattatccc atcagttaat atctgatgtc 1260

caagtcttat ccggtaccat acaagattta caagaccagg tagactcgtt agctgaagta 1320

gttctccaaa ataggagggg actggaccta ctaacggcag aacaaggagg aatttgttta 1380

gccttacaag aaaaatgctg tttttatgct aacaagtcag gaattgtgag aaacaaaata 1440

agaaccctac aagaagaatt acaaaaacgc agggaaagcc tggcatccaa ccctctctgg 1500

accgggctgc agggctttct tccgtacctc ctacctctcc tgggacccct actcaccctc 1560

ctactcatac taaccattgg gccatgcgtt ttcagtcgcc tcatggcctt cattaatgat 1620

agacttaatg ttgtacatgc catggtgctg gcccagcaat accaagcact caaagctgag 1680

gaagaagctc aggattga 1698

<210> 9
<211> 565
<212> PRT
<213> RD114 retrovirus

<220>
<223> RD114 glycoprotein (amino acid sequence)

<400> 9
Met Lys Leu Pro Thr Gly Met Val Ile Leu Cys Ser Leu Ile Ile Val
1               5                   10                  15
Arg Ala Gly Phe Asp Asp Pro Arg Lys Ala Ile Ala Leu Val Gln Lys
            20                  25                  30
Gln His Gly Lys Pro Cys Glu Cys Ser Gly Gly Gln Val Ser Glu Ala
        35                  40                  45
Pro Pro Asn Ser Ile Gln Gln Val Thr Cys Pro Gly Lys Thr Ala Tyr
    50                  55                  60
Leu Met Thr Asn Gln Lys Trp Lys Cys Arg Val Thr Pro Lys Asn Leu
65                  70                  75                  80
Thr Pro Ser Gly Gly Glu Leu Gln Asn Cys Pro Cys Asn Thr Phe Gln
                85                  90                  95
Asp Ser Met His Ser Ser Cys Tyr Thr Glu Tyr Arg Gln Cys Arg Ala
            100                 105                 110
Asn Asn Lys Thr Tyr Tyr Thr Ala Thr Leu Leu Lys Ile Arg Ser Gly
        115                 120                 125
Ser Leu Asn Glu Val Gln Ile Leu Gln Asn Pro Asn Gln Leu Leu Gln
    130                 135                 140
Ser Pro Cys Arg Gly Ser Ile Asn Gln Pro Val Cys Trp Ser Ala Thr
145                 150                 155                 160
Ala Pro Ile His Ile Ser Asp Gly Gly Gly Pro Leu Asp Thr Lys Arg
                165                 170                 175
Val Trp Thr Val Gln Lys Arg Leu Glu Gln Ile His Lys Ala Met His
            180                 185                 190
Pro Glu Leu Gln Tyr His Pro Leu Ala Leu Pro Lys Val Arg Asp Asp

195                     200                     205
Leu Ser Leu Asp Ala Arg Thr Phe Asp Ile Leu Asn Thr Thr Phe Arg
    210                     215                     220
Leu Leu Gln Met Ser Asn Phe Ser Leu Ala Gln Asp Cys Trp Leu Cys
225                     230                     235                     240
Leu Lys Leu Gly Thr Pro Thr Pro Leu Ala Ile Pro Thr Pro Ser Leu
                245                     250                     255
Thr Tyr Ser Leu Ala Asp Ser Leu Ala Asn Ala Ser Cys Gln Ile Ile
            260                     265                     270
Pro Pro Leu Leu Val Gln Pro Met Gln Phe Ser Asn Ser Ser Cys Leu
        275                     280                     285
Ser Ser Pro Phe Ile Asn Asp Thr Glu Gln Ile Asp Leu Gly Ala Val
    290                     295                     300
Thr Phe Thr Asn Cys Thr Ser Val Ala Asn Val Ser Ser Pro Leu Cys
305                     310                     315                     320
Ala Leu Asn Gly Ser Val Phe Leu Cys Gly Asn Asn Met Ala Tyr Thr
                325                     330                     335
Tyr Leu Pro Gln Asn Trp Thr Gly Leu Cys Val Gln Ala Ser Leu Leu
            340                     345                     350
Pro Asp Ile Asp Ile Ile Pro Gly Asp Glu Pro Val Pro Ile Pro Ala
        355                     360                     365
Ile Asp His Tyr Ile His Arg Pro Lys Arg Ala Val Gln Phe Ile Pro
    370                     375                     380
Leu Leu Ala Gly Leu Gly Ile Thr Ala Ala Phe Thr Thr Gly Ala Thr
385                     390                     395                     400
Gly Leu Gly Val Ser Val Thr Gln Tyr Thr Lys Leu Ser His Gln Leu
                405                     410                     415
Ile Ser Asp Val Gln Val Leu Ser Gly Thr Ile Gln Asp Leu Gln Asp
            420                     425                     430
Gln Val Asp Ser Leu Ala Glu Val Val Leu Gln Asn Arg Arg Gly Leu
        435                     440                     445
Asp Leu Leu Thr Ala Glu Gln Gly Gly Ile Cys Leu Ala Leu Gln Glu
    450                     455                     460
Lys Cys Cys Phe Tyr Ala Asn Lys Ser Gly Ile Val Arg Asn Lys Ile
465                     470                     475                     480
Arg Thr Leu Gln Glu Glu Leu Gln Lys Arg Arg Glu Ser Leu Ala Ser
                485                     490                     495
Asn Pro Leu Trp Thr Gly Leu Gln Gly Phe Leu Pro Tyr Leu Leu Pro
            500                     505                     510
Leu Leu Gly Pro Leu Leu Thr Leu Leu Leu Ile Leu Thr Ile Gly Pro
        515                     520                     525
Cys Val Phe Ser Arg Leu Met Ala Phe Ile Asn Asp Arg Leu Asn Val
    530                     535                     540
Val His Ala Met Val Leu Ala Gln Gln Tyr Gln Ala Leu Lys Ala Glu
545                     550                     555                     560
Glu Glu Ala Gln Asp
                565

<210> 10
<211> 1692
<212> DNA
<213> Baboon endogenous virus strain M7

<220>
<223> Baboon endogenous virus strain M7 (BaEV)-GP (nucleic acid
      sequence)

<400> 10
atgggattca caacaaagat aatcttctta tacaacctag tactggtcta cgcggggttt      60

gacgaccctc gcaaagccat agaactagta caaaagcgat atggccgacc atgcgattgc     120

agcggaggac aagtgtccga gcccccgtca gacagggtca gtcaagtgac ttgctcaggc 180

aagacagctt acttaatgcc cgaccaaaga tggaaatgta agtcaattcc aaaagacacc 240

tccccaagcg ggccactcca agagtgcccc tgtaattctt accagtcctc agtacacagt 300

tcttgttata cctcatacca acaatgcaga tcaggcaata agacatatta tacggctact 360

ctgctaaaaa cacaaactgg gggcaccagt gatgtacaag tattaggatc caccaacaaa 420

cttatacaat ctccctgtaa tggcataaaa gggcagtcta tttgctggag cactacagct 480

cctatccacg tctctgatgg aggaggtcca ttagacacca caagaattaa aagtgttcag 540

agaaaactgg aagaaattca taaagcccta tatcctgaac ttcagtatca ccctttggcc 600

atacctaagg ttagagataa cctcatggtc gatgcccaga ctttaaacat tctcaatgcc 660

acttacaact tactcctaat gtccaacacg agcctagtgg acgactgttg gctttgttta 720

aaattaggtc cccctactcc cctcgcaata cctaacttcc tattatccta cgtgactcgc 780

tcctcggata atatctcttg tttaataatt cccccccttc tagttcaacc gatgcagttt 840

tccaattcat cttgcctctt ttcccctcc tacaacagta cagaagaaat agatctaggc 900

catgttgcct tcagcaactg tacctccata accaatgtca ccggtcccat atgcgctgta 960

aatggttcgg tctttctctg tggcaataac atggcataca cttatctacc cacgaactgg 1020

acggggcttt gcgtcctagc aactctcctc cccgacattg acatcattcc cggagatgaa 1080

ccggtcccca tccctgctat tgatcatttt atatatagac ctaaacgggc catacagttt 1140

attcctttac tagcagggct agggatcacc gcagccttca caacaggagc tacaggccta 1200

ggtgtctctg tgacccaata tacaaaatta tctaatcagc taatttctga tgtacaaatc 1260

ttatctagca ccatacaaga tctgcaagat caagtagact cattagccga agtggttctc 1320

cagaacagaa gggggctaga tctacttaca gcagaacaag gaggaatctg tttagccctg 1380

caagaaaaat gctgctttta tgttaacaag tcagggattg tgagagacaa aataaaaacc 1440

ttacaagaag aactagaaag acgtagaaaa gatctagctt ccaacccact ttggactggg 1500

cttcaagggc tcctccctta cctcctgccc tttcttggcc ctctacttac cctcctgctc 1560

ttactcacca ttgggccgtg catttttaac cgtctaaccg cttttattaa tgataagtta 1620

aacataatac acgctatggt gctaacccaa cagtatcagg tgctcagaac cgatgaagaa 1680

gctcaagatt ga 1692

<210> 11
<211> 563
<212> PRT
<213> Baboon endogenous virus strain M7

<220>
<223> Baboon endogenous virus strain M7 (BaEV)-GP (amino acid sequence)

<400> 11
Met Gly Phe Thr Thr Lys Ile Ile Phe Leu Tyr Asn Leu Val Leu Val
1               5                   10                  15
Tyr Ala Gly Phe Asp Asp Pro Arg Lys Ala Ile Glu Leu Val Gln Lys
            20                  25                  30
Arg Tyr Gly Arg Pro Cys Asp Cys Ser Gly Gly Gln Val Ser Glu Pro
        35                  40                  45
Pro Ser Asp Arg Val Ser Gln Val Thr Cys Ser Gly Lys Thr Ala Tyr
    50                  55                  60
Leu Met Pro Asp Gln Arg Trp Lys Cys Lys Ser Ile Pro Lys Asp Thr
65                  70                  75                  80
Ser Pro Ser Gly Pro Leu Gln Glu Cys Pro Cys Asn Ser Tyr Gln Ser
                85                  90                  95
Ser Val His Ser Ser Cys Tyr Thr Ser Tyr Gln Gln Cys Arg Ser Gly
            100                 105                 110
Asn Lys Thr Tyr Tyr Thr Ala Thr Leu Leu Lys Thr Gln Thr Gly Gly
        115                 120                 125
Thr Ser Asp Val Gln Val Leu Gly Ser Thr Asn Lys Leu Ile Gln Ser
    130                 135                 140
Pro Cys Asn Gly Ile Lys Gly Gln Ser Ile Cys Trp Ser Thr Thr Ala
145                 150                 155                 160
Pro Ile His Val Ser Asp Gly Gly Gly Pro Leu Asp Thr Thr Arg Ile
                165                 170                 175
Lys Ser Val Gln Arg Lys Leu Glu Glu Ile His Lys Ala Leu Tyr Pro
            180                 185                 190
Glu Leu Gln Tyr His Pro Leu Ala Ile Pro Lys Val Arg Asp Asn Leu
        195                 200                 205
Met Val Asp Ala Gln Thr Leu Asn Ile Leu Asn Ala Thr Tyr Asn Leu
    210                 215                 220
Leu Leu Met Ser Asn Thr Ser Leu Val Asp Asp Cys Trp Leu Cys Leu
225                 230                 235                 240
Lys Leu Gly Pro Pro Thr Pro Leu Ala Ile Pro Asn Phe Leu Leu Ser
                245                 250                 255
Tyr Val Thr Arg Ser Ser Asp Asn Ile Ser Cys Leu Ile Ile Pro Pro
            260                 265                 270
Leu Leu Val Gln Pro Met Gln Phe Ser Asn Ser Ser Cys Leu Phe Ser
        275                 280                 285
Pro Ser Tyr Asn Ser Thr Glu Glu Ile Asp Leu Gly His Val Ala Phe
    290                 295                 300
Ser Asn Cys Thr Ser Ile Thr Asn Val Thr Gly Pro Ile Cys Ala Val
305                 310                 315                 320
Asn Gly Ser Val Phe Leu Cys Gly Asn Asn Met Ala Tyr Thr Tyr Leu
                325                 330                 335
Pro Thr Asn Trp Thr Gly Leu Cys Val Leu Ala Thr Leu Leu Pro Asp
            340                 345                 350
Ile Asp Ile Ile Pro Gly Asp Glu Pro Val Pro Ile Pro Ala Ile Asp
        355                 360                 365
His Phe Ile Tyr Arg Pro Lys Arg Ala Ile Gln Phe Ile Pro Leu Leu
    370                 375                 380
Ala Gly Leu Gly Ile Thr Ala Ala Phe Thr Thr Gly Ala Thr Gly Leu
385                 390                 395                 400
Gly Val Ser Val Thr Gln Tyr Thr Lys Leu Ser Asn Gln Leu Ile Ser
                405                 410                 415
Asp Val Gln Ile Leu Ser Ser Thr Ile Gln Asp Leu Gln Asp Gln Val
            420                 425                 430
Asp Ser Leu Ala Glu Val Val Leu Gln Asn Arg Arg Gly Leu Asp Leu
        435                 440                 445
Leu Thr Ala Glu Gln Gly Gly Ile Cys Leu Ala Leu Gln Glu Lys Cys
    450                 455                 460
Cys Phe Tyr Val Asn Lys Ser Gly Ile Val Arg Asp Lys Ile Lys Thr
465                 470                 475                 480
Leu Gln Glu Glu Leu Glu Arg Arg Arg Lys Asp Leu Ala Ser Asn Pro

485                 490                 495
Leu Trp Thr Gly Leu Gln Gly Leu Leu Pro Tyr Leu Leu Pro Phe Leu
            500                 505                 510
Gly Pro Leu Leu Thr Leu Leu Leu Leu Thr Ile Gly Pro Cys Ile
        515                 520                 525
Phe Asn Arg Leu Thr Ala Phe Ile Asn Asp Lys Leu Asn Ile Ile His
    530                 535                 540
Ala Met Val Leu Thr Gln Gln Tyr Gln Val Leu Arg Thr Asp Glu Glu
545                 550                 555                 560
Ala Gln Asp

<210> 12
<211> 2058
<212> DNA
<213> Gibbon ape leukemia virus

<220>
<223> Gibbon ape leukemia virus strain SEATO (GALV)-GP (nucleic acid
      sequence)

<400> 12
atggtattgc tgcctgggtc catgcttctc acctcaaacc tgcaccacct tcggcaccag      60

atgagtcctg ggagctggaa aagactgatc atcctcttaa gctgcgtatt cggcggcggc     120

gggacgagtc tgcaaaataa gaacccccac cagcccatga ccctcacttg gcaggtactg     180

tcccaaactg gagacgttgt ctgggataca aaggcagtcc agcccccttg gacttggtgg      240

cccacactta aacctgatgt atgtgccttg gcggctagtc ttgagtcctg ggatatcccg     300

ggaaccgatg tctcgtcctc taaacgagtc agacctccgg actcagacta tactgccgct     360

tataagcaaa tcacctgggg agccataggg tgcagctacc ctcgggctag gactagaatg     420

gcaagctcta ccttctacgt atgtccccgg gatggccgga ccctttcaga agctagaagg     480

tgcggggggc tagaatccct atactgtaaa gaatgggatt gtgagaccac ggggaccggt      540

tattggctat ctaaatcctc aaaagacctc ataactgtaa aatgggacca aaatagcgaa     600

tggactcaaa aatttcaaca gtgtcaccag accggctggt gtaaccccct taaaatagat      660

ttcacagaca aaggaaaatt atccaaggac tggataacgg gaaaaacctg gggattaaga     720

ttctatgtgt ctggacatcc aggcgtacag ttcaccattc gcttaaaaat caccaacatg     780

ccagctgtgg cagtaggtcc tgacctcgtc cttgtggaac aaggacctcc tagaacgtcc     840

ctcgctctcc cacctcctct tcccccaagg gaagcgccac cgccatctct ccccgactct     900

aactccacag ccctggcgac tagtgcacaa actcccacgg tgagaaaaac aattgttacc     960

ctaaacactc cgcctcccac cacaggcgac agactttttg atcttgtgca gggggccttc     1020

ctaaccttaa atgctaccaa cccaggggcc actgagtctt gctggctttg tttggccatg    1080

ggcccccctt attatgaagc aatagcctca tcaggagagg tcgcctactc caccgacctt     1140

gaccggtgcc gctgggggac ccaaggaaag ctcaccctca ctgaggtctc aggacacggg    1200

ttgtgcatag gaaaggtgcc ctttacccat cagcatctct gcaatcagac cctatccatc 1260

aattcctccg gagaccatca gtatctgctc ccctccaacc atagctggtg ggcttgcagc 1320

actggcctca ccccttgcct ctccacctca gtttttaatc agactagaga tttctgtatc 1380

caggtccagc tgattcctcg catctattac tatcctgaag aagttttgtt acaggcctat 1440

gacaattctc accccaggac taaaagagag gctgtctcac ttaccctagc tgttttactg 1500

gggttgggaa tcacggcggg aataggtact ggttcaactg ccttaattaa aggacctata 1560

gacctccagc aaggcctgac aagcctccag atcgccatag atgctgacct ccgggccctc 1620

caagactcag tcagcaagtt agaggactca ctgacttccc tgtccgaggt agtgctccaa 1680

aataggagag gccttgactt gctgtttcta aaagaaggtg gcctctgtgc ggccctaaag 1740

gaagagtgct gtttttacat agaccactca ggtgcagtac gggactccat gaaaaaactc 1800

aaagaaaaac tggataaaag acagttagag cgccagaaaa gccaaaactg gtatgaagga 1860

tggttcaata actccccttg gttcactacc ctgctatcaa ccatcgctgg gcccctatta 1920

ctcctccttc tgttgctcat cctcgggcca tgcatcatca ataagttagt tcaattcatc 1980

aatgatagga taagtgcagt taaaattctg gtccttagac aaaaatatca ggccctagag 2040

aacgaaggta acctttaa 2058

<210> 13
<211> 685
<212> PRT
<213> Gibbon ape leukemia virus

<220>
<223> Gibbon ape leukemia virus strain SEATO (GALV)-GP (amino acid
      sequence)

<400> 13
Met Val Leu Leu Pro Gly Ser Met Leu Leu Thr Ser Asn Leu His His
1               5                   10                  15
Leu Arg His Gln Met Ser Pro Gly Ser Trp Lys Arg Leu Ile Ile Leu
            20                  25                  30
Leu Ser Cys Val Phe Gly Gly Gly Gly Thr Ser Leu Gln Asn Lys Asn
        35                  40                  45
Pro His Gln Pro Met Thr Leu Thr Trp Gln Val Leu Ser Gln Thr Gly
    50                  55                  60
Asp Val Val Trp Asp Thr Lys Ala Val Gln Pro Pro Trp Thr Trp Trp
65                  70                  75                  80
Pro Thr Leu Lys Pro Asp Val Cys Ala Leu Ala Ala Ser Leu Glu Ser
                85                  90                  95
Trp Asp Ile Pro Gly Thr Asp Val Ser Ser Ser Lys Arg Val Arg Pro
            100                 105                 110
Pro Asp Ser Asp Tyr Thr Ala Ala Tyr Lys Gln Ile Thr Trp Gly Ala
        115                 120                 125
Ile Gly Cys Ser Tyr Pro Arg Ala Arg Thr Arg Met Ala Ser Ser Thr
    130                 135                 140
Phe Tyr Val Cys Pro Arg Asp Gly Arg Thr Leu Ser Glu Ala Arg Arg
145                 150                 155                 160
Cys Gly Gly Leu Glu Ser Leu Tyr Cys Lys Glu Trp Asp Cys Glu Thr

                165                 170                 175
Thr Gly Thr Gly Tyr Trp Leu Ser Lys Ser Ser Lys Asp Leu Ile Thr
            180                 185                 190
Val Lys Trp Asp Gln Asn Ser Glu Trp Thr Gln Lys Phe Gln Gln Cys
        195                 200                 205
His Gln Thr Gly Trp Cys Asn Pro Leu Lys Ile Asp Phe Thr Asp Lys
    210                 215                 220
Gly Lys Leu Ser Lys Asp Trp Ile Thr Gly Lys Thr Trp Gly Leu Arg
225                 230                 235                 240
Phe Tyr Val Ser Gly His Pro Gly Val Gln Phe Thr Ile Arg Leu Lys
                245                 250                 255
Ile Thr Asn Met Pro Ala Val Ala Val Gly Pro Asp Leu Val Leu Val
            260                 265                 270
Glu Gln Gly Pro Pro Arg Thr Ser Leu Ala Leu Pro Pro Pro Leu Pro
        275                 280                 285
Pro Arg Glu Ala Pro Pro Pro Ser Leu Pro Asp Ser Asn Ser Thr Ala
    290                 295                 300
Leu Ala Thr Ser Ala Gln Thr Pro Thr Val Arg Lys Thr Ile Val Thr
305                 310                 315                 320
Leu Asn Thr Pro Pro Pro Thr Thr Gly Asp Arg Leu Phe Asp Leu Val
                325                 330                 335
Gln Gly Ala Phe Leu Thr Leu Asn Ala Thr Asn Pro Gly Ala Thr Glu
            340                 345                 350
Ser Cys Trp Leu Cys Leu Ala Met Gly Pro Pro Tyr Tyr Glu Ala Ile
        355                 360                 365
Ala Ser Ser Gly Glu Val Ala Tyr Ser Thr Asp Leu Asp Arg Cys Arg
    370                 375                 380
Trp Gly Thr Gln Gly Lys Leu Thr Leu Thr Glu Val Ser Gly His Gly
385                 390                 395                 400
Leu Cys Ile Gly Lys Val Pro Phe Thr His Gln His Leu Cys Asn Gln
                405                 410                 415
Thr Leu Ser Ile Asn Ser Ser Gly Asp His Gln Tyr Leu Leu Pro Ser
            420                 425                 430
Asn His Ser Trp Trp Ala Cys Ser Thr Gly Leu Thr Pro Cys Leu Ser
        435                 440                 445
Thr Ser Val Phe Asn Gln Thr Arg Asp Phe Cys Ile Gln Val Gln Leu
    450                 455                 460
Ile Pro Arg Ile Tyr Tyr Tyr Pro Glu Glu Val Leu Leu Gln Ala Tyr
465                 470                 475                 480
Asp Asn Ser His Pro Arg Thr Lys Arg Glu Ala Val Ser Leu Thr Leu
                485                 490                 495
Ala Val Leu Leu Gly Leu Gly Ile Thr Ala Gly Ile Gly Thr Gly Ser
            500                 505                 510
Thr Ala Leu Ile Lys Gly Pro Ile Asp Leu Gln Gln Gly Leu Thr Ser
        515                 520                 525
Leu Gln Ile Ala Ile Asp Ala Asp Leu Arg Ala Leu Gln Asp Ser Val
    530                 535                 540
Ser Lys Leu Glu Asp Ser Leu Thr Ser Leu Ser Glu Val Val Leu Gln
545                 550                 555                 560
Asn Arg Arg Gly Leu Asp Leu Leu Phe Leu Lys Glu Gly Gly Leu Cys
                565                 570                 575
Ala Ala Leu Lys Glu Glu Cys Cys Phe Tyr Ile Asp His Ser Gly Ala
            580                 585                 590
Val Arg Asp Ser Met Lys Lys Leu Lys Glu Lys Leu Asp Lys Arg Gln
        595                 600                 605
Leu Glu Arg Gln Lys Ser Gln Asn Trp Tyr Glu Gly Trp Phe Asn Asn
    610                 615                 620
Ser Pro Trp Phe Thr Thr Leu Leu Ser Thr Ile Ala Gly Pro Leu Leu
625                 630                 635                 640
Leu Leu Leu Leu Leu Leu Ile Leu Gly Pro Cys Ile Ile Asn Lys Leu
                645                 650                 655
Val Gln Phe Ile Asn Asp Arg Ile Ser Ala Val Lys Ile Leu Val Leu
            660                 665                 670

Arg Gln Lys Tyr Gln Ala Leu Glu Asn Glu Gly Asn Leu
        675                 680                 685

<210> 14
<211> 1965
<212> DNA
<213> Amphotropic murine leukemia virus


<220>
<223> 4070A Amphotropic Murine leukemia virus (Ampho)-GP (nucleic acid
      sequence)

<400> 14
atggcgcgtt caacgctctc aaaaccccct caagataaga ttaacccgtg gaagccctta      60

atagtcatgg gagtcctgtt aggagtaggg atggcagaga gcccccatca ggtctttaat     120

gtaacctgga gagtcaccaa cctgatgact gggcgtaccg ccaatgccac ctccctcctg     180

ggaactgtac aagatgcctt cccaaaatta tattttgatc tatgtgatct ggtcggagag     240

gagtgggacc cttcagacca ggaaccgtat gtcgggtatg gctgcaagta ccccgcaggg     300

agacagcgga cccggacttt tgacttttac gtgtgccctg ggcataccgt aaagtcgggg     360

tgtgggggac caggagaggg ctactgtggt aaatgggggt gtgaaaccac cggacaggct     420

tactggaagc ccacatcatc gtgggaccta atctccctta agcgcggtaa caccccctgg      480

gacacgggat gctctaaagt tgcctgtggc ccctgctacg acctctccaa agtatccaat     540

tccttccaag gggctactcg agggggcaga tgcaaccctc tagtcctaga attcactgat     600

gcaggaaaaa aggctaactg ggacgggccc aaatcgtggg gactgagact gtaccggaca     660

ggaacagatc ctattaccat gttctccctg acccggcagg tccttaatgt gggaccccga     720

gtccccatag ggcccaaccc agtattaccc gaccaaagac tcccttcctc accaatagag     780

attgtaccgg ctccacagcc acctagcccc ctcaatacca gttacccccc ttccactacc     840

agtacaccct caacctcccc tacaagtcca agtgtcccac agccaccccc aggaactgga     900

gatagactac tagctctagt caaaggagcc tatcaggcgc ttaacctcac caatcccgac     960

aagacccaag aatgttggct gtgcttagtg tcgggacctc cttattacga aggagtagcg    1020

gtcgtgggca cttataccaa tcattccacc gctccggcca actgtacggc cacttcccaa    1080

cataagctta ccctatctga agtgacagga cagggcctat gcatgggggc agtacctaaa    1140

actcaccagg ccttatgtaa caccacccaa agcgccggct caggctccta ctaccttgca    1200

gcacccgccg gaacaatgtg ggcttgcagc actggattga ctccctgctt gtccaccacg    1260

gtgctcaatc taaccacaga ttattgtgta ttagttgaac tctggcccag agtaatttac    1320

cactcccccg attatatgta tggtcagctt gaacagcgta ccaaatataa aagagagcca    1380

gtatcattga ccctggccct tctactagga ggattaacca tgggagggat tgcagctgga    1440

atagggacgg ggaccactgc cttaattaaa acccagcagt ttgagcagct tcatgccgct    1500

atccagacag acctcaacga agtcgaaaag tcaattacca acctagaaaa gtcactgacc 1560

tcgttgtctg aagtagtcct acagaaccgc agaggcctag atttgctatt cctaaaggag 1620

ggaggtctct gcgcagccct aaaagaagaa tgttgttttt atgcagacca cacggggcta 1680

gtgagagaca gcatggccaa attaagagaa aggcttaatc agagacaaaa actatttgag 1740

acaggccaag gatggttcga agggctgttt aatagatccc cctggtttac caccttaatc 1800

tccaccatca tgggacctct aatagtactc ttactgatct tactctttgg accttgcatt 1860

ctcaatcgat tggtccaatt tgttaaagac aggatctcag tggtccaggc tctggttttg 1920

actcagcaat atcaccagct aaaacccata gagtacgagc catga 1965

<210> 15
<211> 654
<212> PRT
<213> Amphotropic murine leukemia virus

<220>
<223> 4070A Amphotropic Murine leukemia virus (Ampho)-GP (amino acid
      sequence)

<400> 15
Met Ala Arg Ser Thr Leu Ser Lys Pro Pro Gln Asp Lys Ile Asn Pro
1               5                   10                  15
Trp Lys Pro Leu Ile Val Met Gly Val Leu Leu Gly Val Gly Met Ala
            20                  25                  30
Glu Ser Pro His Gln Val Phe Asn Val Thr Trp Arg Val Thr Asn Leu
        35                  40                  45
Met Thr Gly Arg Thr Ala Asn Ala Thr Ser Leu Leu Gly Thr Val Gln
    50                  55                  60
Asp Ala Phe Pro Lys Leu Tyr Phe Asp Leu Cys Asp Leu Val Gly Glu
65                  70                  75                  80
Glu Trp Asp Pro Ser Asp Gln Glu Pro Tyr Val Gly Tyr Gly Cys Lys
                85                  90                  95
Tyr Pro Ala Gly Arg Gln Arg Thr Arg Thr Phe Asp Phe Tyr Val Cys
            100                 105                 110
Pro Gly His Thr Val Lys Ser Gly Cys Gly Gly Pro Gly Glu Gly Tyr
        115                 120                 125
Cys Gly Lys Trp Gly Cys Glu Thr Thr Gly Gln Ala Tyr Trp Lys Pro
    130                 135                 140
Thr Ser Ser Trp Asp Leu Ile Ser Leu Lys Arg Gly Asn Thr Pro Trp
145                 150                 155                 160
Asp Thr Gly Cys Ser Lys Val Ala Cys Gly Pro Cys Tyr Asp Leu Ser
                165                 170                 175
Lys Val Ser Asn Ser Phe Gln Gly Ala Thr Arg Gly Gly Arg Cys Asn
            180                 185                 190
Pro Leu Val Leu Glu Phe Thr Asp Ala Gly Lys Lys Ala Asn Trp Asp
        195                 200                 205
Gly Pro Lys Ser Trp Gly Leu Arg Leu Tyr Arg Thr Gly Thr Asp Pro
    210                 215                 220
Ile Thr Met Phe Ser Leu Thr Arg Gln Val Leu Asn Val Gly Pro Arg
225                 230                 235                 240
Val Pro Ile Gly Pro Asn Pro Val Leu Pro Asp Gln Arg Leu Pro Ser
                245                 250                 255
Ser Pro Ile Glu Ile Val Pro Ala Pro Gln Pro Pro Ser Pro Leu Asn
            260                 265                 270

Thr Ser Tyr Pro Pro Ser Thr Thr Ser Thr Pro Ser Thr Ser Pro Thr
        275                 280                 285
Ser Pro Ser Val Pro Gln Pro Pro Pro Gly Thr Gly Asp Arg Leu Leu
    290                 295                 300
Ala Leu Val Lys Gly Ala Tyr Gln Ala Leu Asn Leu Thr Asn Pro Asp
305                 310                 315                 320
Lys Thr Gln Glu Cys Trp Leu Cys Leu Val Ser Gly Pro Pro Tyr Tyr
                325                 330                 335
Glu Gly Val Ala Val Val Gly Thr Tyr Thr Asn His Ser Thr Ala Pro
            340                 345                 350
Ala Asn Cys Thr Ala Thr Ser Gln His Lys Leu Thr Leu Ser Glu Val
        355                 360                 365
Thr Gly Gln Gly Leu Cys Met Gly Ala Val Pro Lys Thr His Gln Ala
    370                 375                 380
Leu Cys Asn Thr Thr Gln Ser Ala Gly Ser Gly Ser Tyr Tyr Leu Ala
385                 390                 395                 400
Ala Pro Ala Gly Thr Met Trp Ala Cys Ser Thr Gly Leu Thr Pro Cys
                405                 410                 415
Leu Ser Thr Thr Val Leu Asn Leu Thr Thr Asp Tyr Cys Val Leu Val
            420                 425                 430
Glu Leu Trp Pro Arg Val Ile Tyr His Ser Pro Asp Tyr Met Tyr Gly
        435                 440                 445
Gln Leu Glu Gln Arg Thr Lys Tyr Lys Arg Glu Pro Val Ser Leu Thr
    450                 455                 460
Leu Ala Leu Leu Leu Gly Gly Leu Thr Met Gly Gly Ile Ala Ala Gly
465                 470                 475                 480
Ile Gly Thr Gly Thr Thr Ala Leu Ile Lys Thr Gln Gln Phe Glu Gln
                485                 490                 495
Leu His Ala Ala Ile Gln Thr Asp Leu Asn Glu Val Glu Lys Ser Ile
            500                 505                 510
Thr Asn Leu Glu Lys Ser Leu Thr Ser Leu Ser Glu Val Val Leu Gln
        515                 520                 525
Asn Arg Arg Gly Leu Asp Leu Leu Phe Leu Lys Glu Gly Gly Leu Cys
    530                 535                 540
Ala Ala Leu Lys Glu Glu Cys Cys Phe Tyr Ala Asp His Thr Gly Leu
545                 550                 555                 560
Val Arg Asp Ser Met Ala Lys Leu Arg Glu Arg Leu Asn Gln Arg Gln
                565                 570                 575
Lys Leu Phe Glu Thr Gly Gln Gly Trp Phe Glu Gly Leu Phe Asn Arg
            580                 585                 590
Ser Pro Trp Phe Thr Thr Leu Ile Ser Thr Ile Met Gly Pro Leu Ile
        595                 600                 605
Val Leu Leu Leu Ile Leu Leu Phe Gly Pro Cys Ile Leu Asn Arg Leu
    610                 615                 620
Val Gln Phe Val Lys Asp Arg Ile Ser Val Val Gln Ala Leu Val Leu
625                 630                 635                 640
Thr Gln Gln Tyr His Gln Leu Lys Pro Ile Glu Tyr Glu Pro
                645                 650

<210> 16
<211> 1938
<212> DNA
<213> Murine leukemia virus

<220>
<223> 10A1 Murine leukemia virus (MLV)-GP (nucleic acid sequence)

<400> 16
   atggaaggtc cagcgttctc aaaacccctt aaagataaga ttaacccgtg gaagtcctta      60
   atggtcatgg gggtctattt aagagtaggg atggcagaga gcccccatca ggtctttaat     120
   gtaacctgga gagtcaccaa cctgatgact gggcgtaccg ccaatgccac ctccctttta 180
   ggaactgtac aagatgcctt cccaagatta tattttgatc tatgtgatct ggtcggagaa 240
   gagtgggacc cttcagacca ggaaccatat gtcgggtatg gctgcaaata ccccggaggg 300
   agaaagcgga cccggacttt tgacttttac gtgtgccctg ggcataccgt aaaatcgggg 360
   tgtggggggc caagagaggg ctactgtggt gaatggggtt gtgaaaccac cggacaggct 420
   tactggaagc ccacatcatc atggacctaa atctccctta agcgcggtaa caccccctgg 480
   gacacgggat gctccaaaat ggcttgtggc ccctgctacg acctctccaa agtatccaat 540
   tccttccaag gggctactcg agggggcaga tgcaaccctc tagtcctaga attcactgat 600
   gcaggaaaaa aggctaattg ggacgggccc aaatcgtggg gactgagact gtaccggaca 660
   ggaacagatc ctattaccat gttctccctg acccgccagg tcctcaatat agggccccgc 720
   atccccattg ggcctaatcc cgtgatcact ggtcaactac ccccctcccg acccgtgcag 780
   atcaggctcc ccaggcctcc tcagcctcct cctacaggcg cagcctctat agtccctgag 840
   actgccccac cttctcaaca acctgggacg ggagacaggc tgctaaacct ggtagaagga 900
   gcctatcagg cgcttaacct caccaatccc gacaagaccc aagaatgttg gctgtgctta 960
   gtgtcgggac ctccttatta cgaaggagta gcggtcgtgg gcacttatac caatcattct 1020
   accgccccgg ccagctgtac ggccacttcc caacataagc ttaccctatc tgaagtgaca 1080
   ggacagggcc tatgcatggg agcactacct aaaactcacc aggccttatg taacaccacc 1140
   caaagtgccg gctcaggatc ctactacctt gcagcacccg ctggaacaat gtgggcttgt 1200
   agcactggat tgactccctg cttgtccacc acgatgctca atctaaccac agactattgt 1260
   gtattagttg agctctggcc cagaataatt taccactccc ccgattatat gtatggtcag 1320
   cttgaacagc gtaccaaata taagagggag ccagtatcgt tgaccctggc ccttctgcta 1380
   ggaggattaa ccatgggagg gattgcagct ggaataggga cggggaccac tgccctaatc 1440
   aaaacccagc agtttgagca gcttcacgcc gctatccaga cagacctcaa cgaagtcgaa 1500
   aaatcaatta ccaacctaga aaagtcactg acctcgttgt ctgaagtagt cctacagaac 1560
   cgaagaggcc tagatttgct cttcctaaaa gagggaggtc tctgcgcagc cctaaaagaa 1620
   gaatgttgtt tttatgcaga ccacacggga ctagtgagag acagcatggc caaactaagg 1680
   gaaaggctta atcagagaca aaaactattt gagtcaggcc aaggttggtt cgaagggcag 1740
   tttaatagat ccccctggtt taccacctta atctccacca tcatgggacc tctaatagta 1800
   ctcttactga tcttactctt tggaccctgc attctcaatc gattggtcca atttgttaaa 1860
   gacaggatct cagtggtcca ggctctggtt ttgactcaac aatatcacca gctaaaacct 1920
   atagagtacg agccatga 1938

<210> 17
<211> 645
<212> PRT
<213> Murine leukemia virus

<220>
<223> 10A1 Murine leukemia virus (MLV)-GP (amino acid sequence)

<400> 17
Met Glu Gly Pro Ala Phe Ser Lys Pro Leu Lys Asp Lys Ile Asn Pro
1               5                   10                  15
Trp Lys Ser Leu Met Val Met Gly Val Tyr Leu Arg Val Gly Met Ala
            20                  25                  30
Glu Ser Pro His Gln Val Phe Asn Val Thr Trp Arg Val Thr Asn Leu
        35                  40                  45
Met Thr Gly Arg Thr Ala Asn Ala Thr Ser Leu Leu Gly Thr Val Gln
    50                  55                  60
Asp Ala Phe Pro Arg Leu Tyr Phe Asp Leu Cys Asp Leu Val Gly Glu
65                  70                  75                  80
Glu Trp Asp Pro Ser Asp Gln Glu Pro Tyr Val Gly Tyr Gly Cys Lys
                85                  90                  95
Tyr Pro Gly Gly Arg Lys Arg Thr Arg Thr Phe Asp Phe Tyr Val Cys
            100                 105                 110
Pro Gly His Thr Val Lys Ser Gly Cys Gly Gly Pro Arg Glu Gly Tyr
        115                 120                 125
Cys Gly Glu Trp Gly Cys Glu Thr Thr Gly Gln Ala Tyr Trp Lys Pro
    130                 135                 140
Thr Ser Ser Trp Asp Leu Ile Ser Leu Lys Arg Gly Asn Thr Pro Trp
145                 150                 155                 160
Asp Thr Gly Cys Ser Lys Met Ala Cys Gly Pro Cys Tyr Asp Leu Ser
                165                 170                 175
Lys Val Ser Asn Ser Phe Gln Gly Ala Thr Arg Gly Gly Arg Cys Asn
            180                 185                 190
Pro Leu Val Leu Glu Phe Thr Asp Ala Gly Lys Lys Ala Asn Trp Asp
        195                 200                 205
Gly Pro Lys Ser Trp Gly Leu Arg Leu Tyr Arg Thr Gly Thr Asp Pro
    210                 215                 220
Ile Thr Met Phe Ser Leu Thr Arg Gln Val Leu Asn Ile Gly Pro Arg
225                 230                 235                 240
Ile Pro Ile Gly Pro Asn Pro Val Ile Thr Gly Gln Leu Pro Pro Ser
                245                 250                 255
Arg Pro Val Gln Ile Arg Leu Pro Arg Pro Pro Gln Pro Pro Pro Thr
            260                 265                 270
Gly Ala Ala Ser Ile Val Pro Glu Thr Ala Pro Pro Ser Gln Gln Pro
        275                 280                 285
Gly Thr Gly Asp Arg Leu Leu Asn Leu Val Glu Gly Ala Tyr Gln Ala
    290                 295                 300
Leu Asn Leu Thr Asn Pro Asp Lys Thr Gln Glu Cys Trp Leu Cys Leu
305                 310                 315                 320
Val Ser Gly Pro Pro Tyr Tyr Glu Gly Val Ala Val Val Gly Thr Tyr
                325                 330                 335
Thr Asn His Ser Thr Ala Pro Ala Ser Cys Thr Ala Thr Ser Gln His
            340                 345                 350
Lys Leu Thr Leu Ser Glu Val Thr Gly Gln Gly Leu Cys Met Gly Ala
        355                 360                 365
Leu Pro Lys Thr His Gln Ala Leu Cys Asn Thr Thr Gln Ser Ala Gly
    370                 375                 380
Ser Gly Ser Tyr Tyr Leu Ala Ala Pro Ala Gly Thr Met Trp Ala Cys
385                 390                 395                 400
Ser Thr Gly Leu Thr Pro Cys Leu Ser Thr Thr Met Leu Asn Leu Thr
                405                 410                 415
Thr Asp Tyr Cys Val Leu Val Glu Leu Trp Pro Arg Ile Ile Tyr His

            420                 425                 430
Ser Pro Asp Tyr Met Tyr Gly Gln Leu Glu Gln Arg Thr Lys Tyr Lys
        435                 440                 445
Arg Glu Pro Val Ser Leu Thr Leu Ala Leu Leu Leu Gly Gly Leu Thr
    450                 455                 460
Met Gly Gly Ile Ala Ala Gly Ile Gly Thr Gly Thr Thr Ala Leu Ile
465                 470                 475                 480
Lys Thr Gln Gln Phe Glu Gln Leu His Ala Ala Ile Gln Thr Asp Leu
                485                 490                 495
Asn Glu Val Glu Lys Ser Ile Thr Asn Leu Glu Lys Ser Leu Thr Ser
            500                 505                 510
Leu Ser Glu Val Val Leu Gln Asn Arg Arg Gly Leu Asp Leu Leu Phe
        515                 520                 525
Leu Lys Glu Gly Gly Leu Cys Ala Ala Leu Lys Glu Glu Cys Cys Phe
    530                 535                 540
Tyr Ala Asp His Thr Gly Leu Val Arg Asp Ser Met Ala Lys Leu Arg
545                 550                 555                 560
Glu Arg Leu Asn Gln Arg Gln Lys Leu Phe Glu Ser Gly Gln Gly Trp
                565                 570                 575
Phe Glu Gly Gln Phe Asn Arg Ser Pro Trp Phe Thr Thr Leu Ile Ser
            580                 585                 590
Thr Ile Met Gly Pro Leu Ile Val Leu Leu Leu Ile Leu Leu Phe Gly
        595                 600                 605
Pro Cys Ile Leu Asn Arg Leu Val Gln Phe Val Lys Asp Arg Ile Ser
    610                 615                 620
Val Val Gln Ala Leu Val Leu Thr Gln Gln Tyr His Gln Leu Lys Pro
625                 630                 635                 640
Ile Glu Tyr Glu Pro
                645

<210> 18
<211> 1590
<212> DNA
<213> Piry virus

<220>
<223> Piry virus (PIRYV)-G (nucleic acid sequence)

<400> 18
atggatctct ttcccatttt ggtcgtggtg ctcatgacag atactgtctt agggaagttt 60

caaattgtct tcccggatca gaatgaactg gagtggagac cagttgtggg tgactctcgg 120

cattgcccac agtcatcaga aatgcaattc gatggaagca gatcccagac catactgact 180

gggaaagctc ccgtggggat cacgccctct aaatcagatg gatttatctg ccatgccgca 240

aaatgggtga caacatgtga tttcaggtgg tatgggccga aatacatcac tcattcaata 300

catcatctga gaccgacaac atcagactgt gagacagctc tccaaaggta taaagatggg 360

agcttaatca atcttggatt ccccccagaa tcctgcggtt atgcaacagt cacagattct 420

gaggcaatgt tggtccaagt gactccccac cacgttgggg tggatgatta tagaggtcac 480

tggatcgacc cactatttcc aggaggagaa tgctccacca atttttgtga tacagtccac 540

aattcatcgg tgtggatccc caagagtcaa aagactgaca tctgtgccca gtctttcaaa 600

aatatcaaga tgaccgcatc ttacccctca gaaggagcat tggtgagtga cagatttgcc 660

ttccacagtg catatcatcc aaatatgccg gggtcaactg tttgcataat ggacttttgc 720

gaacaaaagg ggttgagatt cacaaatgga gagtggatgg gtctcaatgt ggagcaatcc 780

atccgagaga agaagataag tgccatcttc ccaaattgtg ttgcagggac tgaaatccga 840

gccacactag aatcagaagg ggcaagaact ttgacgtggg agactcaaag aatgctagat 900

tactctttgt gtcagaacac ctgggacaaa gtttccagga aagaacctct cagtccgctt 960

gacttgagct atctgtcacc aagggctcca gggaaaggca tggcctatac cgtcataaac 1020

ggaaccctgc attcggctca tgctaaatac attagaacct ggattgatta tggagaaatg 1080

aaggaaatta aaggtggacg tggagaatat tccaaggctc ctgagctcct ctggtcccag 1140

tggttcgatt ttggaccgtt caaaattgga ccgaatggac tcctgcacac agggaaaacc 1200

tttaaattcc ctctttattt gatcggagca ggcataattg acgaagatct gcatgaacta 1260

gatgaggctg ctcccattga tcacccacaa atgcctgacg cgaaaagcgt tcttccagaa 1320

gatgaagaga tattcttcgg agacacaggt gtatccaaaa accctatcga gttgattcaa 1380

ggatggttct caaattggag agagagtgta atggcaatag tcggaattgt tctactcatc 1440

gttgtgacat ttctggcgat caagacggtc cgggtgctta attgtctctg gagacccaga 1500

aagaaaagaa tcgtcagaca agaagtagat gttgaatccc gactaaacca ttttgagatg 1560

agaggctttc ctgaatatgt taagagataa 1590

<210> 19
<211> 529
<212> PRT
<213> Piry virus

<220>
<223> Piry virus (PIRYV)-G (amino acid sequence)

<400> 19
   Met Asp Leu Phe Pro Ile Leu Val Val Val Leu Met Thr Asp Thr Val
   1 5 10 15
   Leu Gly Lys Phe Gln Ile Val Phe Pro Asp Gln Asn Glu Leu Glu Trp
   20 25 30
   Arg Pro Val Val Gly Asp Ser Arg His Cys Pro Gln Ser Ser Glu Met
   35 40 45
   Gln Phe Asp Gly Ser Arg Ser Gln Thr Ile Leu Thr Gly Lys Ala Pro
   50 55 60
   Val Gly Ile Thr Pro Ser Lys Ser Asp Gly Phe Ile Cys His Ala Ala
   65 70 75 80
   Lys Trp Val Thr Thr Cys Asp Phe Arg Trp Tyr Gly Pro Lys Tyr Ile
   85 90 95
   Thr His Ser Ile His His Leu Arg Pro Thr Thr Ser Asp Cys Glu Thr
   100 105 110
   Ala Leu Gln Arg Tyr Lys Asp Gly Ser Leu Ile Asn Leu Gly Phe Pro
   115 120 125
   Pro Glu Ser Cys Gly Tyr Ala Thr Val Thr Asp Ser Glu Ala Met Leu
   130 135 140
   Val Gln Val Thr Pro His His Val Gly Val Asp Asp Tyr Arg Gly His
   145 150 155 160
   Trp Ile Asp Pro Leu Phe Pro Gly Gly Glu Cys Ser Thr Asn Phe Cys
   165 170 175
   Asp Thr Val His Asn Ser Ser Val Trp Ile Pro Lys Ser Gln Lys Thr
   180 185 190
   Asp Ile Cys Ala Gln Ser Phe Lys Asn Ile Lys Met Thr Ala Ser Tyr
   195 200 205
   Pro Ser Glu Gly Ala Leu Val Ser Asp Arg Phe Ala Phe His Ser Ala
   210 215 220
   Tyr His Pro Asn Met Pro Gly Ser Thr Val Cys Ile Met Asp Phe Cys
   225 230 235 240
   Glu Gln Lys Gly Leu Arg Phe Thr Asn Gly Glu Trp Met Gly Leu Asn
   245 250 255
   Val Glu Gln Ser Ile Arg Glu Lys Lys Ile Ser Ala Ile Phe Pro Asn
   260 265 270
   Cys Val Ala Gly Thr Glu Ile Arg Ala Thr Leu Glu Ser Glu Gly Ala
   275 280 285
   Arg Thr Leu Thr Trp Glu Thr Gln Arg Met Leu Asp Tyr Ser Leu Cys
   290 295 300
   Gln Asn Thr Trp Asp Lys Val Ser Arg Lys Glu Pro Leu Ser Pro Leu
   305 310 315 320
   Asp Leu Ser Tyr Leu Ser Pro Arg Ala Pro Gly Lys Gly Met Ala Tyr
   325 330 335
   Thr Val Ile Asn Gly Thr Leu His Ser Ala His Ala Lys Tyr Ile Arg
   340 345 350
   Thr Trp Ile Asp Tyr Gly Glu Met Lys Glu Ile Lys Gly Gly Arg Gly
   355 360 365
   Glu Tyr Ser Lys Ala Pro Glu Leu Leu Trp Ser Gln Trp Phe Asp Phe
   370 375 380
   Gly Pro Phe Lys Ile Gly Pro Asn Gly Leu Leu His Thr Gly Lys Thr
   385 390 395 400
   Phe Lys Phe Pro Leu Tyr Leu Ile Gly Ala Gly Ile Ile Asp Glu Asp
   405 410 415
   Leu His Glu Leu Asp Glu Ala Ala Pro Ile Asp His Pro Gln Met Pro
   420 425 430
   Asp Ala Lys Ser Val Leu Pro Glu Asp Glu Glu Ile Phe Phe Gly Asp
   435 440 445
   Thr Gly Val Ser Lys Asn Pro Ile Glu Leu Ile Gln Gly Trp Phe Ser
   450 455 460
   Asn Trp Arg Glu Ser Val Met Ala Ile Val Gly Ile Val Leu Leu Ile
   465 470 475 480
   Val Val Thr Phe Leu Ala Ile Lys Thr Val Arg Val Leu Asn Cys Leu
   485 490 495
   Trp Arg Pro Arg Lys Lys Arg Ile Val Arg Gln Glu Val Asp Val Glu
   500 505 510
   Ser Arg Leu Asn His Phe Glu Met Arg Gly Phe Pro Glu Tyr Val Lys
   515 520 525
   Arg

<210> 20
<211> 1536
<212> DNA
<213> Vesicular stomatitis virus

<220>
<223> Vesicular stomatitis virus (VSV)-G serotype Indiana (nucleic acid
      sequence)

<400> 20
atgaagtgcc ttttgtactt agccttttta ttcattgggg tgaattgcaa gttcaccata      60

gtttttccac acaaccaaaa aggaaactgg aaaaatgttc cttctaatta ccattattgc     120

ccgtcaagct cagatttaaa ttggcataat gacttaatag gcacagcctt acaagtcaaa 180

atgcccaaga gtcacaaggc tattcaagca gacggttgga tgtgtcatgc ttccaaatgg 240

gtcactactt gtgatttccg ctggtatgga ccgaagtata taacacattc catccgatcc 300

ttcactccat ctgtagaaca atgcaaggaa agcattgaac aaacgaaaca aggaacttgg 360

ctgaatccag gcttccctcc tcaaagttgt ggatatgcaa ctgtgacgga tgccgaagca 420

gtgattgtcc aggtgactcc tcaccatgtg ctggttgatg aatacacagg agaatgggtt 480

gattcacagt tcatcaacgg aaaatgcagc aattacatat gccccactgt ccataactct 540

acaacctggc attctgacta taaggtcaaa gggctatgtg attctaacct catttccatg 600

gacatcacct tcttctcaga ggacggagag ctatcatccc tgggaaagga gggcacaggg 660

ttcagaagta actactttgc ttatgaaact ggaggcaagg cctgcaaaat gcaatactgc 720

aagcattggg gagtcagact cccatcaggt gtctggttcg agatggctga taaggatctc 780

tttgctgcag ccagattccc tgaatgccca gaagggtcaa gtatctctgc tccatctcag 840

acctcagtgg atgtaagtct aattcaggac gttgagagga tcttggatta ttccctctgc 900

caagaaacct ggagcaaaat cagagcgggt cttccaatct ctccagtgga tctcagctat 960

cttgctccta aaaacccagg aaccggtcct gctttcacca taatcaatgg taccctaaaa 1020

tactttgaga ccagatacat cagagtcgat attgctgctc caatcctctc aagaatggtc 1080

ggaatgatca gtggaactac cacagaaagg gaactgtggg atgactgggc accatatgaa 1140

gacgtggaaa ttggacccaa tggagttctg aggaccagtt caggatataa gtttccttta 1200

tacatgattg gacatggtat gttggactcc gatcttcatc ttagctcaaa ggctcaggtg 1260

ttcgaacatc ctcacattca agacgctgct tcgcaacttc ctgatgatga gagtttattt 1320

tttggtgata ctgggctatc caaaaatcca atcgagcttg tagaaggttg gttcagtagt 1380

tggaaaagct ctattgcctc ttttttcttt atcatagggt taatcattgg actattcttg 1440

gttctccgag ttggtatcca tctttgcatt aaattaaagc acaccaagaa aagacagatt 1500

tatacagaca tagagatgaa ccgacttgga aagtaa 1536

<210> 21
<211> 511
<212> PRT
<213> Vesicular stomatitis virus

<220>
<223> Vesicular stomatitis virus (VSV)-G serotype Indiana (amino acid
      sequence)

<400> 21
Met Lys Cys Leu Leu Tyr Leu Ala Phe Leu Phe Ile Gly Val Asn Cys
1               5                   10                  15
Lys Phe Thr Ile Val Phe Pro His Asn Gln Lys Gly Asn Trp Lys Asn

                20                  25                  30
Val Pro Ser Asn Tyr His Tyr Cys Pro Ser Ser Ser Asp Leu Asn Trp
        35                  40                  45
His Asn Asp Leu Ile Gly Thr Ala Leu Gln Val Lys Met Pro Lys Ser
    50                  55                  60
His Lys Ala Ile Gln Ala Asp Gly Trp Met Cys His Ala Ser Lys Trp
65                  70                  75                  80
Val Thr Thr Cys Asp Phe Arg Trp Tyr Gly Pro Lys Tyr Ile Thr His
                85                  90                  95
Ser Ile Arg Ser Phe Thr Pro Ser Val Glu Gln Cys Lys Glu Ser Ile
            100                 105                 110
Glu Gln Thr Lys Gln Gly Thr Trp Leu Asn Pro Gly Phe Pro Pro Gln
        115                 120                 125
Ser Cys Gly Tyr Ala Thr Val Thr Asp Ala Glu Ala Val Ile Val Gln
    130                 135                 140
Val Thr Pro His His Val Leu Val Asp Glu Tyr Thr Gly Glu Trp Val
145                 150                 155                 160
Asp Ser Gln Phe Ile Asn Gly Lys Cys Ser Asn Tyr Ile Cys Pro Thr
                165                 170                 175
Val His Asn Ser Thr Thr Trp His Ser Asp Tyr Lys Val Lys Gly Leu
            180                 185                 190
Cys Asp Ser Asn Leu Ile Ser Met Asp Ile Thr Phe Phe Ser Glu Asp
        195                 200                 205
Gly Glu Leu Ser Ser Leu Gly Lys Glu Gly Thr Gly Phe Arg Ser Asn
    210                 215                 220
Tyr Phe Ala Tyr Glu Thr Gly Gly Lys Ala Cys Lys Met Gln Tyr Cys
225                 230                 235                 240
Lys His Trp Gly Val Arg Leu Pro Ser Gly Val Trp Phe Glu Met Ala
                245                 250                 255
Asp Lys Asp Leu Phe Ala Ala Ala Arg Phe Pro Glu Cys Pro Glu Gly
            260                 265                 270
Ser Ser Ile Ser Ala Pro Ser Gln Thr Ser Val Asp Val Ser Leu Ile
        275                 280                 285
Gln Asp Val Glu Arg Ile Leu Asp Tyr Ser Leu Cys Gln Glu Thr Trp
    290                 295                 300
Ser Lys Ile Arg Ala Gly Leu Pro Ile Ser Pro Val Asp Leu Ser Tyr
305                 310                 315                 320
Leu Ala Pro Lys Asn Pro Gly Thr Gly Pro Ala Phe Thr Ile Ile Asn
                325                 330                 335
Gly Thr Leu Lys Tyr Phe Glu Thr Arg Tyr Ile Arg Val Asp Ile Ala
            340                 345                 350
Ala Pro Ile Leu Ser Arg Met Val Gly Met Ile Ser Gly Thr Thr Thr
        355                 360                 365
Glu Arg Glu Leu Trp Asp Asp Trp Ala Pro Tyr Glu Asp Val Glu Ile
    370                 375                 380
Gly Pro Asn Gly Val Leu Arg Thr Ser Ser Gly Tyr Lys Phe Pro Leu
385                 390                 395                 400
Tyr Met Ile Gly His Gly Met Leu Asp Ser Asp Leu His Leu Ser Ser
                405                 410                 415
Lys Ala Gln Val Phe Glu His Pro His Ile Gln Asp Ala Ala Ser Gln
            420                 425                 430
Leu Pro Asp Asp Glu Ser Leu Phe Phe Gly Asp Thr Gly Leu Ser Lys
        435                 440                 445
Asn Pro Ile Glu Leu Val Glu Gly Trp Phe Ser Ser Trp Lys Ser Ser
    450                 455                 460
Ile Ala Ser Phe Phe Phe Ile Ile Gly Leu Ile Ile Gly Leu Phe Leu
465                 470                 475                 480
Val Leu Arg Val Gly Ile His Leu Cys Ile Lys Leu Lys His Thr Lys
                485                 490                 495
Lys Arg Gln Ile Tyr Thr Asp Ile Glu Met Asn Arg Leu Gly Lys
            500                 505                 510

<210> 22

<211> 1554
<212> DNA
<213> Vesicular stomatitis virus

<220>
<223> Vesicular stomatitis virus (VSV)-G serotype New Jersey (nucleic
      acid sequence)

<400> 22
atgttgtctt atctaatctt tgcacttgcc gtttcgccca ttttgggcaa aattgaaatt      60

gtgtttcctc aacataccac tggggattgg aagagagttc cccatgaata taattattgc     120

cctaccagcg cagacaagaa ctcacatggg actcaaacag gaatccctgt tgagttaaca     180

atgccaaaag gactaacaac ccatcaagtt gaaggattta tgtgtcactc agccttgtgg     240

atgaccactt gtgacttcag atggtatggg ccaaaataca taacccattc catacataat     300

gaagagccta cagattatca atgtttggag gccattaagt catacaaaga tggagtcagt     360

ttcaatccag ggtttcctcc tcagagctgc gggtatggca cagttaccga tgccgaagcc     420

catattgtga cagttactcc ccactctgtc aaagtggacg agtacacggg ggaatggatc     480

gatccacatt tcatcggagg aaggtgcaaa ggacaaattt gtgaaacagt ccataattcc     540

acaaaatggt ttacgtcctc tgatggagaa agtgtctgca gtcaattgtt tactttggtt     600

ggaggaattt ttttctctga ttcagaagag attacctcca tggggttacc agaaacagga     660

atcagaagta attacttccc ctacatatct acagagggaa tttgcaaaat gccgttttgc     720

agaaaacagg ggtacaagct taaaaatgac ctctggttcc agatcatgga cccagacctg     780

gataaaacgg ttagagatct ccctcatatt aaggactgtg acctctcctc gtccataatc     840

acaccaggag aacatgctac agacatctca ctgatatcag atgttgaaag gatcctggac     900

tatgctcttt gtcagaatac atggagtaaa attgaatcgg gagaaccaat tactccggta     960

gatctcagct atcttgggcc aaaaaaccca ggggttgggc cggtcttcac catcattaac    1020

ggttccctgc attattttac atcgaagtat ctgcgagtcg aattagaaag tcctgtcata    1080

cccagaatgg aaggaaaagt tgcaggaact aggattgtac ggcaattgtg ggatcagtgg    1140

tttcctttcg gagaagttga gattggaccc aatggtgtgt tgaaaacgaa gcaagggtat     1200

aaattcccac tacacatcat tggaactgga gaagtagaca gtgacatcaa aatggaaagg    1260

gttgtcaagc actgggaaca cccccatatt gaggccgctc agacattttt aaaaaaagat    1320

gacacaggag aagtccttta ttatggcgac accggagtgt cgaaaaatcc agttgaatta    1380

gtcgagggat ggtttagtgg atggaggagc tccctcatgg gagtgctggc tgtgattata    1440

ggatttgtga ttttaatgtt tttaattaaa ttgattggag tcttatctag ccttttcaga    1500

cctaaacgca ggccaatcta caaatcagac gtggaaatgg ctcatttccg ttaa          1554

<210> 23
<211> 517
<212> PRT
<213> Vesicular stomatitis virus

<220>
<223> Vesicular stomatitis virus (VSV)-G serotype New Jersey (amino
      acid sequence)

<400> 23
Met Leu Ser Tyr Leu Ile Phe Ala Leu Ala Val Ser Pro Ile Leu Gly
1               5                   10                  15
Lys Ile Glu Ile Val Phe Pro Gln His Thr Thr Gly Asp Trp Lys Arg
            20                  25                  30
Val Pro His Glu Tyr Asn Tyr Cys Pro Thr Ser Ala Asp Lys Asn Ser
        35                  40                  45
His Gly Thr Gln Thr Gly Ile Pro Val Glu Leu Thr Met Pro Lys Gly
    50                  55                  60
Leu Thr Thr His Gln Val Glu Gly Phe Met Cys His Ser Ala Leu Trp
65                  70                  75                  80
Met Thr Thr Cys Asp Phe Arg Trp Tyr Gly Pro Lys Tyr Ile Thr His
                85                  90                  95
Ser Ile His Asn Glu Glu Pro Thr Asp Tyr Gln Cys Leu Glu Ala Ile
            100                 105                 110
Lys Ser Tyr Lys Asp Gly Val Ser Phe Asn Pro Gly Phe Pro Pro Gln
        115                 120                 125
Ser Cys Gly Tyr Gly Thr Val Thr Asp Ala Glu Ala His Ile Val Thr
    130                 135                 140
Val Thr Pro His Ser Val Lys Val Asp Glu Tyr Thr Gly Glu Trp Ile
145                 150                 155                 160
Asp Pro His Phe Ile Gly Gly Arg Cys Lys Gly Gln Ile Cys Glu Thr
                165                 170                 175
Val His Asn Ser Thr Lys Trp Phe Thr Ser Ser Asp Gly Glu Ser Val
            180                 185                 190
Cys Ser Gln Leu Phe Thr Leu Val Gly Gly Ile Phe Phe Ser Asp Ser
        195                 200                 205
Glu Glu Ile Thr Ser Met Gly Leu Pro Glu Thr Gly Ile Arg Ser Asn
    210                 215                 220
Tyr Phe Pro Tyr Ile Ser Thr Glu Gly Ile Cys Lys Met Pro Phe Cys
225                 230                 235                 240
Arg Lys Gln Gly Tyr Lys Leu Lys Asn Asp Leu Trp Phe Gln Ile Met
                245                 250                 255
Asp Pro Asp Leu Asp Lys Thr Val Arg Asp Leu Pro His Ile Lys Asp
            260                 265                 270
Cys Asp Leu Ser Ser Ser Ile Ile Thr Pro Gly Glu His Ala Thr Asp
        275                 280                 285
Ile Ser Leu Ile Ser Asp Val Glu Arg Ile Leu Asp Tyr Ala Leu Cys
    290                 295                 300
Gln Asn Thr Trp Ser Lys Ile Glu Ser Gly Glu Pro Ile Thr Pro Val
305                 310                 315                 320
Asp Leu Ser Tyr Leu Gly Pro Lys Asn Pro Gly Val Gly Pro Val Phe
                325                 330                 335
Thr Ile Ile Asn Gly Ser Leu His Tyr Phe Thr Ser Lys Tyr Leu Arg
            340                 345                 350
Val Glu Leu Glu Ser Pro Val Ile Pro Arg Met Glu Gly Lys Val Ala
        355                 360                 365
Gly Thr Arg Ile Val Arg Gln Leu Trp Asp Gln Trp Phe Pro Phe Gly
    370                 375                 380
Glu Val Glu Ile Gly Pro Asn Gly Val Leu Lys Thr Lys Gln Gly Tyr
385                 390                 395                 400
Lys Phe Pro Leu His Ile Ile Gly Thr Gly Glu Val Asp Ser Asp Ile
                405                 410                 415

Lys Met Glu Arg Val Val Lys His Trp Glu His Pro His Ile Glu Ala
            420                 425                 430
Ala Gln Thr Phe Leu Lys Lys Asp Asp Thr Gly Glu Val Leu Tyr Tyr
        435                 440                 445
Gly Asp Thr Gly Val Ser Lys Asn Pro Val Glu Leu Val Glu Gly Trp
    450                 455                 460
Phe Ser Gly Trp Arg Ser Ser Leu Met Gly Val Leu Ala Val Ile Ile
465                 470                 475                 480
Gly Phe Val Ile Leu Met Phe Leu Ile Lys Leu Ile Gly Val Leu Ser
                485                 490                 495
Ser Leu Phe Arg Pro Lys Arg Arg Pro Ile Tyr Lys Ser Asp Val Glu
            500                 505                 510
Met Ala His Phe Arg
        515

<210> 24
<211> 7712
<212> DNA
<213> Artificial Sequence

<220>
<223> Prototypical transfer vector plasmid encoding the lentiviral
      vector genome (pRRL.PPT.CAGs.dTomato.pre nosal) (nucleic acid
      sequence)

<220>
<221> promoter
<222> 4..235
<223> /note="RSV promoter"

<220>
<221> LTR
<222> 236..417
<223> /note="R/U5"

<220>
<221> 5'UTR
<222> 418..435
<223> /note="Primer Binding Site (PBS)"

<220>
<221> 5'UTR
<222> 572..936
<223> /note="dGag"

<220>
<221> 5'UTR
<222> 1082..1323
<223> /note="Rev-responsive element (RRE)"

<220>
<221> 5'UTR
<222> 1807..1930
<223> /note="Polypurine tract (PPT)"

<220>
<221> enhancer
<222> 1965..2341
<223> /note="Human Cytomegalovirus Immediate-Early (CMVie)"

<220>
<221> promoter

<222> 1965..2820
<223> /note="CAGs"

<220>
<221> promoter
<222> 2343..2620
<223> /note="Chicken beta-Actin promoter"

<220>
<221> gene
<222> 2846..3551
<223> /note="Transgene dTomato"

<220>
<221> 3'UTR
<222> 3551..4159
<223> /note="Posttranscriptional Regulatory Element (PRE)"

<220>
<221> LTR
<222> 4241..4474
<223> /note="Self-inactivating (SIN) LTR"

<220>
<221> polyA_signal
<222> 4675..4881
<223> /note="SV40 pA/Ori"

<220>
<221> rep_origin
<222> 5042..5500
<223> /note="F1 ORI"

<220>
<221> gene
<222> 5632..6489
<223> /note="Ampicillin resistance gene"

<400> 24
agcttaatgt agtcttatgc aatactcttg tagtcttgca acatggtaac gatgagttag      60

caacatgcct tacaaggaga gaaaaagcac cgtgcatgcc gattggtgga agtaaggtgg     120

tacgatcgtg ccttattagg aaggcaacag acgggtctga catggattgg acgaaccact     180

gaattgccgc attgcagaga tattgtattt aagtgcctag ctcgatacaa taaacgggtc     240

tctctggtta gaccagatct gagcctggga gctctctggc taactaggga acccactgct     300

taagcctcaa taaagcttgc cttgagtgct tcaagtagtg tgtgcccgtc tgttgtgtga     360

ctctggtaac tagagatccc tcagaccctt ttagtcagtg tggaaaatct ctagcagtgg     420

cgcccgaaca gggacttgaa agcgaaaggg aaaccagagg agctctctcg acgcaggact     480

cggcttgctg aagcgcgcac ggcaagaggc gaggggcggc gactggtgag tacgccaaaa     540

attttgacta gcggaggcta gaaggagaga gatgggtgcg agagcgtcag tattaagcgg     600

gggagaatta gatcgcgatg ggaaaaaatt cggttaaggc cagggggaaa gaaaaaatat     660

aaattaaaac atatagtatg ggcaagcagg gagctagaac gattcgcagt taatcctggc     720

ctgttagaaa catcagaagg ctgtagacaa atactgggac agctacaacc atcccttcag 780

acaggatcag aagaacttag atcattatat aatacagtag caaccctcta ttgtgtgcat 840

caaaggatag agataaaaga caccaaggaa gctttagaca agatagagga agagcaaaac 900

aaaagtaaga ccaccgcaca gcaagcggcc gctgatcttc agacctggag gaggagatat 960

gagggacaat tggagaagtg aattatataa atataaagta gtaaaaattg aaccattagg 1020

agtagcaccc accaaggcaa agagaagagt ggtgcagaga gaaaaaagag cagtgggaat 1080

aggagctttg ttccttgggt tcttgggagc agcaggaagc actatgggcg cagcctcaat 1140

gacgctgacg gtacaggcca gacaattatt gtctggtata gtgcagcagc agaacaattt 1200

gctgagggct attgaggcgc aacagcatct gttgcaactc acagtctggg gcatcaagca 1260

gctccaggca agaatcctgg ctgtggaaag atacctaaag gatcaacagc tcctggggat 1320

ttggggttgc tctggaaaac tcatttgcac cactgctgtg ccttggaatg ctagttggag 1380

taataaatct ctggaacaga tttggaatca cacgacctgg atggagtggg acagagaaat 1440

taacaattac acaagcttaa tacactcctt aattgaagaa tcgcaaaacc agcaagaaaa 1500

gaatgaacaa gaattattgg aattagataa atgggcaagt ttgtggaatt ggtttaacat 1560

aacaaattgg ctgtggtata taaaattatt cataatgata gtaggaggct tggtaggttt 1620

aagaatagtt tttgctgtac tttctatagt gaatagagtt aggcagggat attcaccatt 1680

atcgtttcag acccacctcc caaccccgag gggacccgac aggcccgaag gaatagaaga 1740

agaaggtgga gagagagaca gagacagatc cattcgatta gtgaacggat ctcgacggta 1800

tcggttaact tttaaaagaa aaggggggat tggggggtac agtgcagggg aaagaatagt 1860

agacataata gcaacagaca tacaaactaa agaattacaa aaacaaatta caaaaattca 1920

aaattttatc gatcacgaga ctagcctcga gctagcccgt cgacattgat tattgactag 1980

ttattaatag taatcaatta cggggtcatt agttcatagc ccatatatgg agttccgcgt 2040

tacataactt acggtaaatg gcccgcctgg ctgaccgccc aacgaccccc gcccattgac 2100

gtcaataatg acgtatgttc ccatagtaac gccaataggg actttccatt gacgtcaatg 2160

ggtggagtat ttacggtaaa ctgcccactt ggcagtacat caagtgtatc atatgccaag 2220

tacgccccct attgacgtca atgacggtaa atggcccgcc tggcattatg cccagtacat 2280

gaccttatgg gactttccta cttggcagta catctacgta ttagtcatcg ctattaccat 2340

ggtcgaggtg agccccacgt tctgcttcac tctccccatc tcccccccct ccccaccccc 2400

aattttgtat ttatttattt tttaattatt ttgtgcagcg atgggggcgg gggggggggg 2460

ggggcgcgcg ccaggcgggg cggggcgggg cgaggggcgg ggcggggcga ggcggagagg 2520

tgcggcggca gccaatcaga gcggcgcgct ccgaaagttt ccttttatgg cgaggcggcg 2580

gcggcggcgg ccctataaaa agcgaagcgc gcggcgggcg ggagtcgctg cgcgctgcct 2640

tcgccccgtg ccccgctccg cgccgcctcg cgccgcccgc cccggctctg actgaccgcg 2700

ttactcccac aggtgagcgg gcgggacggc ccttctcctc cgggctgtaa ttagcgcttg 2760

gtttaatgac ggcttgtttc ttttctgtgg ctgcgtgaaa gccttgaggg gctccgggag 2820

gaccggtgaa ttcggatccg ccaccatggt gagcaagggc gaggaggtca tcaaagagtt 2880

catgcgcttc aaggtgcgca tggagggctc catgaacggc cacgagttcg agatcgaggg 2940

cgagggcgag ggccgcccct acgagggcac ccagaccgcc aagctgaagg tgaccaaggg 3000

cggccccctg cccttcgcct gggacatcct gtccccccag ttcatgtacg gctccaaggc 3060

gtacgtgaag caccccgccg acatccccga ttacaagaag ctgtccttcc ccgagggctt 3120

caagtgggag cgcgtgatga acttcgagga cggcggtctg gtgaccgtga cccaggactc 3180

ctccctgcag gacggcacgc tgatctacaa ggtgaagatg cgcggcacca acttcccccc 3240

cgacggcccc gtaatgcaga agaagaccat gggctgggag gcctccaccg agcgcctgta 3300

cccccgcgac ggcgtgctga agggcgagat ccaccaggcc ctgaagctga aggacggcgg 3360

ccactacctg gtggagttca agaccatcta catggccaag aagcccgtgc aactgcccgg 3420

ctactactac gtggacacca agctggacat cacctcccac aacgaggact acaccatcgt 3480

ggaacagtac gagcgctccg agggccgcca ccacctgttc ctgtacggca tggacgagct 3540

gtacaagtaa agcggccgcg tcgacaatca acctctggat tacaaaattt gtgaaagatt 3600

gactggtatt cttaactatg ttgctccttt tacgctatgt ggatacgctg ctttaatgcc 3660

tttgtatcat gctattgctt cccgtatggc tttcattttc tcctccttgt ataaatcctg 3720

gttgctgtct ctttatgagg agttgtggcc cgttgtcagg caacgtggcg tggtgtgcac 3780

tgtgtttgct gacgcaaccc ccactggttg gggcattgcc accacctgtc agctcctttc 3840

cgggactttc gctttccccc tccctattgc cacggcggaa ctcatcgccg cctgccttgc 3900

ccgctgctgg acaggggctc ggctgttggg cactgacaat tccgtggtgt tgtcggggaa 3960

gctgacgtcc tttccatggc tgctcgcctg tgttgccacc tggattctgc gcgggacgtc 4020

cttctgctac gtcccttcgg ccctcaatcc agcggacctt ccttcccgcg gcctgctgcc 4080

ggctctgcgg cctcttccgc gtcttcgcct tcgccctcag acgagtcgga tctccctttg 4140

ggccgcctcc ccgcctggaa ttcgagctcg gtacctttaa gaccaatgac ttacaaggca 4200

gctgtagatc ttagccactt tttaaaagaa aaggggggac tggaagggct aattcactcc 4260

caacgaagac aagatctgct ttttgcttgt actgggtctc tctggttaga ccagatctga 4320

gcctgggagc tctctggcta actagggaac ccactgctta agcctcaata aagcttgcct 4380

tgagtgcttc aagtagtgtg tgcccgtctg ttgtgtgact ctggtaacta gagatccctc 4440

agaccctttt agtcagtgtg gaaaatctct agcagtagta gttcatgtca tcttattatt 4500

cagtatttat aacttgcaaa gaaatgaata tcagagagtg agaggaactt gtttattgca 4560

gcttataatg gttacaaata aagcaatagc atcacaaatt tcacaaataa agcatttttt 4620

tcactgcatt ctagttgtgg tttgtccaaa ctcatcaatg tatcttatca tgtctggctc 4680

tagctatccc gcccctaact ccgcccagtt ccgcccattc tccgccccat ggctgactaa 4740

ttttttttat ttatgcagag gccgaggccg cctcggcctc tgagctattc cagaagtagt 4800

gaggaggctt ttttggaggc ctaggctttt gcgtcgagac gtacccaatt cgccctatag 4860

tgagtcgtat tacgcgcgct cactggccgt cgttttacaa cgtcgtgact gggaaaaccc 4920

tggcgttacc caacttaatc gccttgcagc acatccccct ttcgccagct ggcgtaatag 4980

cgaagaggcc cgcaccgatc gcccttccca acagttgcgc agcctgaatg gcgaatggcg 5040

cgacgcgccc tgtagcggcg cattaagcgc ggcgggtgtg gtggttacgc gcagcgtgac 5100

cgctacactt gccagcgccc tagcgcccgc tcctttcgct ttcttccctt cctttctcgc 5160

cacgttcgcc ggctttcccc gtcaagctct aaatcggggg ctccctttag ggttccgatt 5220

tagtgcttta cggcacctcg accccaaaaa acttgattag ggtgatggtt cacgtagtgg 5280

gccatcgccc tgatagacgg tttttcgccc tttgacgttg gagtccacgt tctttaatag 5340

tggactcttg ttccaaactg gaacaacact caaccctatc tcggtctatt cttttgattt 5400

ataagggatt ttgccgattt cggcctattg gttaaaaaat gagctgattt aacaaaaatt 5460

taacgcgaat tttaacaaaa tattaacgtt tacaatttcc caggtggcac ttttcgggga 5520

aatgtgcgcg gaacccctat ttgtttattt ttctaaatac attcaaatat gtatccgctc 5580

atgagacaat aaccctgata aatgcttcaa taatattgaa aaaggaagag tatgagtatt 5640

caacatttcc gtgtcgccct tattcccttt tttgcggcat tttgccttcc tgtttttgct 5700

cacccagaaa cgctggtgaa agtaaaagat gctgaagatc agttgggtgc acgagtgggt 5760

tacatcgaac tggatctcaa cagcggtaag atccttgaga gttttcgccc cgaagaacgt 5820

tttccaatga tgagcacttt taaagttctg ctatgtggcg cggtattatc ccgtattgac 5880

gccgggcaag agcaactcgg tcgccgcata cactattctc agaatgactt ggttgagtac 5940

tcaccagtca cagaaaagca tcttacggat ggcatgacag taagagaatt atgcagtgct 6000

gccataacca tgagtgataa cactgcggcc aacttacttc tgacaacgat cggaggaccg 6060

aaggagctaa ccgctttttt gcacaacatg ggggatcatg taactcgcct tgatcgttgg 6120

gaaccggagc tgaatgaagc cataccaaac gacgagcgtg acaccacgat gcctgtagca 6180

atggcaacaa cgttgcgcaa actattaact ggcgaactac ttactctagc ttcccggcaa 6240

caattaatag actggatgga ggcggataaa gttgcaggac cacttctgcg ctcggccctt 6300

ccggctggct ggtttattgc tgataaatct ggagccggtg agcgtgggtc tcgcggtatc 6360

attgcagcac tggggccaga tggtaagccc tcccgtatcg tagttatcta cacgacgggg 6420

agtcaggcaa ctatggatga acgaaataga cagatcgctg agataggtgc ctcactgatt 6480

aagcattggt aactgtcaga ccaagtttac tcatatatac tttagattga tttaaaactt 6540

catttttaat ttaaaaggat ctaggtgaag atcctttttg ataatctcat gaccaaaatc 6600

ccttaacgtg agttttcgtt ccactgagcg tcagaccccg tagaaaagat caaaggatct 6660

tcttgagatc ctttttttct gcgcgtaatc tgctgcttgc aaacaaaaaa accaccgcta 6720

ccagcggtgg tttgtttgcc ggatcaagag ctaccaactc tttttccgaa ggtaactggc 6780

ttcagcagag cgcagatacc aaatactgtc cttctagtgt agccgtagtt aggccaccac 6840

ttcaagaact ctgtagcacc gcctacatac ctcgctctgc taatcctgtt accagtggct 6900

gctgccagtg gcgataagtc gtgtcttacc gggttggact caagacgata gttaccggat 6960

aaggcgcagc ggtcgggctg aacggggggt tcgtgcacac agcccagctt ggagcgaacg 7020

acctacaccg aactgagata cctacagcgt gagctatgag aaagcgccac gcttcccgaa 7080

gggagaaagg cggacaggta tccggtaagc ggcagggtcg gaacaggaga gcgcacgagg 7140

gagcttccag ggggaaacgc ctggtatctt tatagtcctg tcgggtttcg ccacctctga 7200

cttgagcgtc gatttttgtg atgctcgtca ggggggcgga gcctatggaa aaacgccagc 7260

aacgcggcct ttttacggtt cctggccttt tgctggcctt ttgctcacat gttctttcct 7320

gcgttatccc ctgattctgt ggataaccgt attaccgcct ttgagtgagc tgataccgct 7380

cgccgcagcc gaacgaccga gcgcagcgag tcagtgagcg aggaagcgga agagcgccca 7440

atacgcaaac cgcctctccc cgcgcgttgg ccgattcatt aatgcagctg gcacgacagg 7500

tttcccgact ggaaagcggg cagtgagcgc aacgcaatta atgtgagtta gctcactcat 7560

taggcacccc aggctttaca ctttatgctt ccggctcgta tgttgtgtgg aattgtgagc 7620

ggataacaat ttcacacagg aaacagctat gaccatgatt acgccaagcg cgcaattaac 7680

cctcactaaa gggaacaaaa gctggagctg ca 7712

<210> 25
<211> 11035
<212> DNA
<213> Artificial Sequence

<220>
<223> Packaging plasmid (pCDNA3.g/p.4xCTE) (nucleic acid sequence)

<220>
<221> misc_feature
<222> 2..769
<223> /note="4x Constitutive Transport Element (CTE)"

<220>
<221> polyA_signal
<222> 847..1071

<223> /note="bGH pA"

<220>
<221> promoter
<222> 5482..6069
<223> /note="CMV promoter"

<220>
<221> intron
<222> 6157..6700
<223> /note="Beta Globin Intron"

<220>
<221> gene
<222> 6722..11028
<223> /note="HIV-1 gag/pol"

<400> 25
aattcctgca gcccgggatc cactagttct agaccacctc ccctgtgagc tagactggac 60

agccaatgac gggtaagaga gtgacatttc tcactaacct aagacaggag ggccgtcaaa 120

gctactgcct aatccaatga cgggtaatag tgacaagaaa tgtatcactc caacctaaga 180

caggcgcagc ctccgaggga tgtgttgatc agctagacca cctccctgtg agctagactg 240

gacagccaat gacgggtaag agagtgacat ttctcactaa cctaagacag gagggccgtc 300

aaagctactg cctaatccaa tgacgggtaa tagtgacaag aaatgtatca ctccaaccta 360

agacaggcgc agcctccgag ggatgtgttg atcagctaga ccacctcccc tgtgagctag 420

actggacagc caatgacggg taagagagtg acatttctca ctaacctaag acaggagggc 480

cgtcaaagct actgcctaat ccaatgacgg gtaatagtga caagaaatgt atcactccaa 540

cctaagacag gcgcagcctc cgagggatgt gttgatcagc tagaccacct cccctgtgag 600

ctagactgga cagccaatga cgggtaagag agtgacattt ctcactaacc taagacagga 660

gggccgtcaa agctactgcc taatccaatg acgggtaata gtgacaagaa atgtatcact 720

ccaacctaag acaggcgcag cctccgaggg atgtgttgat cagctagagc ggccgctcga 780

gcatgcatct agagggccct attctatagt gtcacctaaa tgctagagct cgctgatcag 840

cctcgactgt gccttctagt tgccagccat ctgttgtttg cccctccccc gtgccttcct 900

tgaccctgga aggtgccact cccactgtcc tttcctaata aaatgaggaa attgcatcgc 960

attgtctgag taggtgtcat tctattctgg ggggtggggt ggggcaggac agcaaggggg 1020

aggattggga agacaatagc aggcatgctg gggatgcggt gggctctatg gcttctgagg 1080

cggaaagaac cagctggggc tctagggggt atccccacgc gccctgtagc ggcgcattaa 1140

gcgcggcggg tgtggtggtt acgcgcagcg tgaccgctac acttgccagc gccctagcgc 1200

ccgctccttt cgctttcttc ccttcctttc tcgccacgtt cgccggcttt ccccgtcaag 1260

ctctaaatcg ggggctccct ttagggttcc gatttagtgc tttacggcac ctcgacccca 1320

aaaaacttga ttagggtgat ggttcacgta gtgggccatc gccctgatag acggtttttc 1380

gccctttgac gttggagtcc acgttcttta atagtggact cttgttccaa actggaacaa 1440

cactcaaccc tatctcggtc tattcttttg atttataagg gattttgccg atttcggcct 1500

attggttaaa aaatgagctg atttaacaaa aatttaacgc gaattaattc tgtggaatgt 1560

gtgtcagtta gggtgtggaa agtccccagg ctccccagca ggcagaagta tgcaaagcat 1620

gcatctcaat tagtcagcaa ccaggtgtgg aaagtcccca ggctccccag caggcagaag 1680

tatgcaaagc atgcatctca attagtcagc aaccatagtc ccgcccctaa ctccgcccat 1740

cccgccccta actccgccca gttccgccca ttctccgccc catggctgac taattttttt 1800

tatttatgca gaggccgagg ccgcctctgc ctctgagcta ttccagaagt agtgaggagg 1860

cttttttgga ggcctaggct tttgcaaaaa gctcccggga gcttgtatat ccattttcgg 1920

atctgatcaa gagacaggat gaggatcgtt tcgcatgatt gaacaagatg gattgcacgc 1980

aggttctccg gccgcttggg tggagaggct attcggctat gactgggcac aacagacaat 2040

cggctgctct gatgccgccg tgttccggct gtcagcgcag gggcgcccgg ttctttttgt 2100

caagaccgac ctgtccggtg ccctgaatga actgcaggac gaggcagcgc ggctatcgtg 2160

gctggccacg acgggcgttc cttgcgcagc tgtgctcgac gttgtcactg aagcgggaag 2220

ggactggctg ctattgggcg aagtgccggg gcaggatctc ctgtcatctc accttgctcc 2280

tgccgagaaa gtatccatca tggctgatgc aatgcggcgg ctgcatacgc ttgatccggc 2340

tacctgccca ttcgaccacc aagcgaaaca tcgcatcgag cgagcacgta ctcggatgga 2400

agccggtctt gtcgatcagg atgatctgga cgaagagcat caggggctcg cgccagccga 2460

actgttcgcc aggctcaagg cgcgcatgcc cgacggcgag gatctcgtcg tgacccatgg 2520

cgatgcctgc ttgccgaata tcatggtgga aaatggccgc ttttctggat tcatcgactg 2580

tggccggctg ggtgtggcgg accgctatca ggacatagcg ttggctaccc gtgatattgc 2640

tgaagagctt ggcggcgaat gggctgaccg cttcctcgtg ctttacggta tcgccgctcc 2700

cgattcgcag cgcatcgcct tctatcgcct tcttgacgag ttcttctgag cgggactctg 2760

gggttcgaaa tgaccgacca agcgacgccc aacctgccat cacgagattt cgattccacc 2820

gccgccttct atgaaaggtt gggcttcgga atcgttttcc gggacgccgg ctggatgatc 2880

ctccagcgcg gggatctcat gctggagttc ttcgcccacc ccaacttgtt tattgcagct 2940

tataatggtt acaaataaag caatagcatc acaaatttca caaataaagc atttttttca 3000

ctgcattcta gttgtggttt gtccaaactc atcaatgtat cttatcatgt ctgtataccg 3060

tcgacctcta gctagagctt ggcgtaatca tggtcatagc tgtttcctgt gtgaaattgt 3120

tatccgctca caattccaca caacatacga gccggaagca taaagtgtaa agcctggggt 3180

gcctaatgag tgagctaact cacattaatt gcgttgcgct cactgcccgc tttccagtcg 3240

ggaaacctgt cgtgccagct gcattaatga atcggccaac gcgcggggag aggcggtttg 3300

cgtattgggc gctcttccgc ttcctcgctc actgactcgc tgcgctcggt cgttcggctg 3360

cggcgagcgg tatcagctca ctcaaaggcg gtaatacggt tatccacaga atcaggggat 3420

aacgcaggaa agaacatgtg agcaaaaggc cagcaaaagg ccaggaaccg taaaaaggcc 3480

gcgttgctgg cgtttttcca taggctccgc ccccctgacg agcatcacaa aaatcgacgc 3540

tcaagtcaga ggtggcgaaa cccgacagga ctataaagat accaggcgtt tccccctgga 3600

agctccctcg tgcgctctcc tgttccgacc ctgccgctta ccggatacct gtccgccttt 3660

ctcccttcgg gaagcgtggc gctttctcat agctcacgct gtaggtatct cagttcggtg 3720

taggtcgttc gctccaagct gggctgtgtg cacgaacccc ccgttcagcc cgaccgctgc 3780

gccttatccg gtaactatcg tcttgagtcc aacccggtaa gacacgactt atcgccactg 3840

gcagcagcca ctggtaacag gattagcaga gcgaggtatg taggcggtgc tacagagttc 3900

ttgaagtggt ggcctaacta cggctacact agaagaacag tatttggtat ctgcgctctg 3960

ctgaagccag ttaccttcgg aaaaagagtt ggtagctctt gatccggcaa acaaaccacc 4020

gctggtagcg gtggtttttt tgtttgcaag cagcagatta cgcgcagaaa aaaaggatct 4080

caagaagatc ctttgatctt ttctacgggg tctgacgctc agtggaacga aaactcacgt 4140

taagggattt tggtcatgag attatcaaaa aggatcttca cctagatcct tttaaattaa 4200

aaatgaagtt ttaaatcaat ctaaagtata tatgagtaaa cttggtctga cagttaccaa 4260

tgcttaatca gtgaggcacc tatctcagcg atctgtctat ttcgttcatc catagttgcc 4320

tgactccccg tcgtgtagat aactacgata cgggagggct taccatctgg ccccagtgct 4380

gcaatgatac cgcgagaccc acgctcaccg gctccagatt tatcagcaat aaaccagcca 4440

gccggaaggg ccgagcgcag aagtggtcct gcaactttat ccgcctccat ccagtctatt 4500

aattgttgcc gggaagctag agtaagtagt tcgccagtta atagtttgcg caacgttgtt 4560

gccattgcta caggcatcgt ggtgtcacgc tcgtcgtttg gtatggcttc attcagctcc 4620

ggttcccaac gatcaaggcg agttacatga tcccccatgt tgtgcaaaaa agcggttagc 4680

tccttcggtc ctccgatcgt tgtcagaagt aagttggccg cagtgttatc actcatggtt 4740

atggcagcac tgcataattc tcttactgtc atgccatccg taagatgctt ttctgtgact 4800

ggtgagtact caaccaagtc attctgagaa tagtgtatgc ggcgaccgag ttgctcttgc 4860

ccggcgtcaa tacgggataa taccgcgcca catagcagaa ctttaaaagt gctcatcatt 4920

ggaaaacgtt cttcggggcg aaaactctca aggatcttac cgctgttgag atccagttcg 4980

atgtaaccca ctcgtgcacc caactgatct tcagcatctt ttactttcac cagcgtttct 5040

gggtgagcaa aaacaggaag gcaaaatgcc gcaaaaaagg gaataagggc gacacggaaa 5100

tgttgaatac tcatactctt cctttttcaa tattattgaa gcatttatca gggttattgt 5160

ctcatgagcg gatacatatt tgaatgtatt tagaaaaata aacaaatagg ggttccgcgc 5220

acatttcccc gaaaagtgcc acctgacgtc gacggatcgg gagatctccc gatcccctat 5280

ggtgcactct cagtacaatc tgctctgatg ccgcatagtt aagccagtat ctgctccctg 5340

cttgtgtgtt ggaggtcgct gagtagtgcg cgagcaaaat ttaagctaca acaaggcaag 5400

gcttgaccga caattgcatg aagaatctgc ttagggttag gcgttttgcg ctgcttcgcg 5460

atgtacgggc cagatatacg cgttgacatt gattattgac tagttattaa tagtaatcaa 5520

ttacggggtc attagttcat agcccatata tggagttccg cgttacataa cttacggtaa 5580

atggcccgcc tggctgaccg cccaacgacc cccgcccatt gacgtcaata atgacgtatg 5640

ttcccatagt aacgccaata gggactttcc attgacgtca atgggtggag tatttacggt 5700

aaactgccca cttggcagta catcaagtgt atcatatgcc aagtacgccc cctattgacg 5760

tcaatgacgg taaatggccc gcctggcatt atgcccagta catgacctta tgggactttc 5820

ctacttggca gtacatctac gtattagtca tcgctattac catggtgatg cggttttggc 5880

agtacatcaa tgggcgtgga tagcggtttg actcacgggg atttccaagt ctccacccca 5940

ttgacgtcaa tgggagtttg ttttggcacc aaaatcaacg ggactttcca aaatgtcgta 6000

acaactccgc cccattgacg caaatgggcg gtaggcgtgt acggtgggag gtctatataa 6060

gcagagctct ctggctaact agagaaccca ctgcttactg gcttatcgaa attaatacga 6120

ctcactatag ggagacccaa gcttggtacc gagctcggat cctgagaact tcagggtgag 6180

tctatgggac ccttgatgtt ttctttcccc ttcttttcta tggttaagtt catgtcatag 6240

gaaggggaga agtaacaggg tacacatatt gaccaaatca gggtaatttt gcatttgtaa 6300

ttttaaaaaa tgctttcttc ttttaatata cttttttgtt tatcttattt ctaatacttt 6360

ccctaatctc tttctttcag ggcaataatg atacaatgta tcatgcctct ttgcaccatt 6420

ctaaagaata acagtgataa tttctgggtt aaggcaatag caatatttct gcatataaat 6480

atttctgcat ataaattgta actgatgtaa gaggtttcat attgctaata gcagctacaa 6540

tccagctacc attctgcttt tattttatgg ttgggataag gctggattat tctgagtcca 6600

agctaggccc ttttgctaat catgttcata cctcttatct tcctcccaca gctcctgggc 6660

aacgtgctgg tctgtgtgct ggcccatcac tttggcaaag aattcgagat ctgccgccgc 6720

catgggtgcg agagcgtcag tattaagcgg gggagaatta gatcgatggg aaaaaattcg 6780

gttaaggcca gggggaaaga aaaaatataa attaaaacat atagtatggg caagcaggga 6840

gctagaacga ttcgcagtta atcctggcct gttagaaaca tcagaaggct gtagacaaat 6900

actgggacag ctacaaccat cccttcagac aggatcagaa gaacttagat cattatataa 6960

tacagtagca accctctatt gtgtgcatca aaggatagag ataaaagaca ccaaggaagc 7020

tttagacaag atagaggaag agcaaaacaa aagtaagaaa aaagcacagc aagcagcagc 7080

tgacacagga cacagcaatc aggtcagcca aaattaccct atagtgcaga acatccaggg 7140

gcaaatggta catcaggcca tatcacctag aactttaaat gcatgggtaa aagtagtaga 7200

agagaaggct ttcagcccag aagtgatacc catgttttca gcattatcag aaggagccac 7260

cccacaagat ttaaacacca tgctaaacac agtgggggga catcaagcag ccatgcaaat 7320

gttaaaagag accatcaatg aggaagctgc agaatgggat agagtgcatc cagtgcatgc 7380

agggcctatt gcaccaggcc agatgagaga accaagggga agtgacatag caggaactac 7440

tagtaccctt caggaacaaa taggatggat gacacataat ccacctatcc cagtaggaga 7500

aatctataaa agatggataa tcctgggatt aaataaaata gtaagaatgt atagccctac 7560

cagcattctg gacataagac aaggaccaaa ggaacccttt agagactatg tagaccgatt 7620

ctataaaact ctaagagccg agcaagcttc acaagaggta aaaaattgga tgacagaaac 7680

cttgttggtc caaaatgcga acccagattg taagactatt ttaaaagcat tgggaccagg 7740

agcgacacta gaagaaatga tgacagcatg tcagggagtg gggggacccg gccataaagc 7800

aagagttttg gctgaagcaa tgagccaagt aacaaatcca gctaccataa tgatacagaa 7860

aggcaatttt aggaaccaaa gaaagactgt taagtgtttc aattgtggca aagaagggca 7920

catagccaaa aattgcaggg cccctaggaa aaagggctgt tggaaatgtg gaaaggaagg 7980

acaccaaatg aaagattgta ctgagagaca ggctaatttt ttagggaaga tctggccttc 8040

ccacaaggga aggccaggga attttcttca gagcagacca gagccaacag ccccaccaga 8100

agagagcttc aggtttgggg aagagacaac aactccctct cagaagcagg agccgataga 8160

caaggaactg tatcctttag cttccctcag atcactcttt ggcagcgacc cctcgtcaca 8220

ataaagatag gggggcaatt aaaggaagct ctattagata caggagcaga tgatacagta 8280

ttagaagaaa tgaatttgcc aggaagatgg aaaccaaaaa tgataggggg aattggaggt 8340

tttatcaaag taagacagta tgatcagata ctcatagaaa tctgcggaca taaagctata 8400

ggtacagtat tagtaggacc tacacctgtc aacataattg gaagaaatct gttgactcag 8460

attggctgca ctttaaattt tcccattagt cctattgaga ctgtaccagt aaaattaaag 8520

ccaggaatgg atggcccaaa agttaaacaa tggccattga cagaagaaaa aataaaagca 8580

ttagtagaaa tttgtacaga aatggaaaag gaaggaaaaa tttcaaaaat tgggcctgaa 8640

aatccataca atactccagt atttgccata aagaaaaaag acagtactaa atggagaaaa 8700

ttagtagatt tcagagaact taataagaga actcaagatt tctgggaagt tcaattagga 8760

ataccacatc ctgcagggtt aaaacagaaa aaatcagtaa cagtactgga tgtgggcgat 8820

gcatattttt cagttccctt agataaagac ttcaggaagt atactgcatt taccatacct 8880

agtataaaca atgagacacc agggattaga tatcagtaca atgtgcttcc acagggatgg 8940

aaaggatcac cagcaatatt ccagtgtagc atgacaaaaa tcttagagcc ttttagaaaa 9000

caaaatccag acatagtcat ctatcaatac atggatgatt tgtatgtagg atctgactta 9060

gaaatagggc agcatagaac aaaaatagag gaactgagac aacatctgtt gaggtgggga 9120

tttaccacac cagacaaaaa acatcagaaa gaacctccat tcctttggat gggttatgaa 9180

ctccatcctg ataaatggac agtacagcct atagtgctgc cagaaaagga cagctggact 9240

gtcaatgaca tacagaaatt agtgggaaaa ttgaattggg caagtcagat ttatgcaggg 9300

attaaagtaa ggcaattatg taaacttctt aggggaacca aagcactaac agaagtagta 9360

ccactaacag aagaagcaga gctagaactg gcagaaaaca gggagattct aaaagaaccg 9420

gtacatggag tgtattatga cccatcaaaa gacttaatag cagaaataca gaagcagggg 9480

caaggccaat ggacatatca aatttatcaa gagccattta aaaatctgaa aacaggaaag 9540

tatgcaagaa tgaagggtgc ccacactaat gatgtgaaac aattaacaga ggcagtacaa 9600

aaaatagcca cagaaagcat agtaatatgg ggaaagactc ctaaatttaa attacccata 9660

caaaaggaaa catgggaagc atggtggaca gagtattggc aagccacctg gattcctgag 9720

tgggagtttg tcaatacccc tcccttagtg aagttatggt accagttaga gaaagaaccc 9780

ataataggag cagaaacttt ctatgtagat ggggcagcca atagggaaac taaattagga 9840

aaagcaggat atgtaactga cagaggaaga caaaaagttg tccccctaac ggacacaaca 9900

aatcagaaga ctgagttaca agcaattcat ctagctttgc aggattcggg attagaagta 9960

aacatagtga cagactcaca atatgcattg ggaatcattc aagcacaacc agataagagt 10020

gaatcagagt tagtcagtca aataatagag cagttaataa aaaaggaaaa agtctacctg 10080

gcatgggtac cagcacacaa aggaattgga ggaaatgaac aagtagataa attggtcagt 10140

gctggaatca ggaaagtact atttttagat ggaatagata aggcccaaga agaacatgag 10200

aaatatcaca gtaattggag agcaatggct agtgatttta acctaccacc tgtagtagca 10260

aaagaaatag tagccagctg tgataaatgt cagctaaaag gggaagccat gcatggacaa 10320

gtagactgta gcccaggaat atggcagcta gattgtacac atttagaagg aaaagttatc 10380

ttggtagcag ttcatgtagc cagtggatat atagaagcag aagtaattcc agcagagaca 10440

gggcaagaaa cagcatactt cctcttaaaa ttagcaggaa gatggccagt aaaaacagta 10500

catacagaca atggcagcaa tttcaccagt actacagtta aggccgcctg ttggtgggcg 10560

gggatcaagc aggaatttgg cattccctac aatccccaaa gtcaaggagt aatagaatct 10620

atgaataaag aattaaagaa aattatagga caggtaagag atcaggctga acatcttaag 10680

acagcagtac aaatggcagt attcatccac aattttaaaa gaaaaggggg gattgggggg 10740

tacagtgcag gggaaagaat agtagacata atagcaacag acatacaaac taaagaatta 10800

caaaaacaaa ttacaaaaat tcaaaatttt cgggtttatt acagggacag cagagatcca 10860

gtttggaaag gaccagcaaa gctcctctgg aaaggtgaag gggcagtagt aatacaagat 10920

aatagtgaca taaaagtagt gccaagaaga aaagcaaaga tcatcaggga ttatggaaaa 10980

cagatggcag gtgatgattg tgtggcaagt agacaggatg aggattaaca catgg 11035

<210> 26
<211> 4180
<212> DNA
<213> Artificial Sequence

<220>
<223> Rev-encoding plasmid (pRSV.Rev) (nucleic acid sequence)

<220>
<221> promoter
<222> 238..635
<223> /note="RSV promoter"

<220>
<221> gene
<222> 756..1106
<223> /note="Rev"

<400> 26
agcgcccaat acgcaaaccg cctctccccg cgcgttggcc gattcattaa tgcagctggc 60

acgacaggtt tcccgactgg aaagcgggca gtgagcgcaa cgcaattaat gtgagttagc 120

tcactcatta ggcaccccag gctttacact ttatgcttcc ggctcgtatg ttgtgtggaa 180

ttgtgagcgg ataacaattt cacacaggaa acagctatga catgattacg aattcgatgt 240

acgggccaga tatacgcgta tctgagggga ctagggtgtg tttaggcgaa aagcggggct 300

tcggttgtac gcggttagga gtcccctcag gatatagtag tttcgctttt gcatagggag 360

ggggaaatgt agtcttatgc aatactcttg tagtcttgca acatggtaac gatgagttag 420

caacatgcct tacaaggaga gaaaaagcac cgtgcatgcc gattggtgga agtaaggtgg 480

tacgatcgtg ccttattagg aaggcaacag acgggtctga catggattgg acgaaccact 540

gaattccgca ttgcagagat attgtattta agtgcctagc tcgatacaat aaacgccatt 600

tgaccattca ccacattggt gtgcacctcc aagctcgagc tcgtttagtg aaccgtcaga 660

tcgcctggag acgccatcca cgctgttttg acctccatag aagacaccgg gaccgatcca 720

gcctcccctc gaagctagtc gattaggcat ctcctatggc aggaagaagc ggagacagcg 780

acgaagacct cctcaaggca gtcagactca tcaagtttct ctatcaaagc aacccacctc 840

ccaatcccga ggggacccga caggcccgaa ggaatagaag aagaaggtgg agagagagac 900

agagacagat ccattcgatt agtgaacgga tccttagcac ttatctggga cgatctgcgg 960

agcctgtgcc tcttcagcta ccaccgcttg agagacttac tcttgattgt aacgaggatt 1020

gtggaacttc tgggacgcag ggggtgggaa gccctcaaat attggtggaa tctcctacaa 1080

tattggagtc aggagctaaa gaatagtgct gttagcttgc tcaatgccac agctatagca 1140

gtagctgagg ggacagatag ggttatagaa gtagtacaag aagcttggca ctggccgtcg 1200

ttttacaacg tcgtgatctg agcctgggag atctctggct aactagggaa cccactgctt 1260

aagcctcaat aaagcttgcc ttgagtgctt caagtagtgt gtgcccgtct gttgtgtgac 1320

tctggtaact agagatcagg aaaaccctgg cgttacccaa cttaatcgcc ttgcagcaca 1380

tccccctttc gccagctggc gtaatagcga agaggcccgc accgatcgcc cttcccaaca 1440

gttgcgcagc ctgaatggcg aatggcgcct gatgcggtat tttctcctta cgcatctgtg 1500

cggtatttca caccgcatac gtcaaagcaa ccatagtacg cgccctgtag cggcgcatta 1560

agcgcggcgg gtgtggtggt tacgcgcagc gtgaccgcta cacttgccag cgccctagcg 1620

cccgctcctt tcgctttctt cccttccttt ctcgccacgt tcgccggctt tccccgtcaa 1680

gctctaaatc gggggctccc tttagggttc cgatttagtg ctttacggca cctcgacccc 1740

aaaaaacttg atttgggtga tggttcacgt agtgggccat cgccctgata gacggttttt 1800

cgccctttga cgttggagtc cacgttcttt aatagtggac tcttgttcca aactggaaca 1860

acactcaacc ctatctcggg ctattctttt gatttataag ggattttgcc gatttcggcc 1920

tattggttaa aaaatgagct gatttaacaa aaatttaacg cgaattttaa caaaatatta 1980

acgtttacaa ttttatggtg cactctcagt acaatctgct ctgatgccgc atagttaagc 2040

cagccccgac acccgccaac acccgctgac gcgccctgac gggcttgtct gctcccggca 2100

tccgcttaca gacaagctgt gaccgtctcc gggagctgca tgtgtcagag gttttcaccg 2160

tcatcaccga aacgcgcgag acgaaagggc ctcgtgatac gcctattttt ataggttaat 2220

gtcatgataa taatggtttc ttagacgtca ggtggcactt ttcggggaaa tgtgcgcgga 2280

acccctattt gtttattttt ctaaatacat tcaaatatgt atccgctcat gagacaataa 2340

ccctgataaa tgcttcaata atattgaaaa aggaagagta tgagtattca acatttccgt 2400

gtcgccctta ttcccttttt tgcggcattt tgccttcctg tttttgctca cccagaaacg 2460

ctggtgaaag taaaagatgc tgaagatcag ttgggtgcac gagtgggtta catcgaactg 2520

gatctcaaca gcggtaagat ccttgagagt tttcgccccg aagaacgttt tccaatgatg 2580

agcactttta aagttctgct atgtggcgcg gtattatccc gtattgacgc cgggcaagag 2640

caactcggtc gccgcataca ctattctcag aatgacttgg ttgagtactc accagtcaca 2700

gaaaagcatc ttacggatgg catgacagta agagaattat gcagtgctgc cataaccatg 2760

agtgataaca ctgcggccaa cttacttctg acaacgatcg gaggaccgaa ggagctaacc 2820

gcttttttgc acaacatggg ggatcatgta actcgccttg atcgttggga accggagctg 2880

aatgaagcca taccaaacga cgagcgtgac accacgatgc ctgtagcaat ggcaacaacg 2940

ttgcgcaaac tattaactgg cgaactactt actctagctt cccggcaaca attaatagac 3000

tggatggagg cggataaagt tgcaggacca cttctgcgct cggcccttcc ggctggctgg 3060

tttattgctg ataaatctgg agccggtgag cgtgggtctc gcggtatcat tgcagcactg 3120

gggccagatg gtaagccctc ccgtatcgta gttatctaca cgacggggag tcaggcaact 3180

atggatgaac gaaatagaca gatcgctgag ataggtgcct cactgattaa gcattggtaa 3240

ctgtcagacc aagtttactc atatatactt tagattgatt taaaacttca tttttaattt 3300

aaaaggatct aggtgaagat cctttttgat aatctcatga ccaaaatccc ttaacgtgag 3360

ttttcgttcc actgagcgtc agaccccgta gaaaagatca aaggatcttc ttgagatcct 3420

ttttttctgc gcgtaatctg ctgcttgcaa acaaaaaaac caccgctacc agcggtggtt 3480

tgtttgccgg atcaagagct accaactctt tttccgaagg taactggctt cagcagagcg 3540

cagataccaa atactgttct tctagtgtag ccgtagttag gccaccactt caagaactct 3600

gtagcaccgc ctacatacct cgctctgcta atcctgttac cagtggctgc tgccagtggc 3660

gataagtcgt gtcttaccgg gttggactca agacgatagt taccggataa ggcgcagcgg 3720

tcgggctgaa cggggggttc gtgcacacag cccagcttgg agcgaacgac ctacaccgaa 3780

ctgagatacc tacagcgtga gctatgagaa agcgccacgc ttcccgaagg gagaaaggcg 3840

gacaggtatc cggtaagcgg cagggtcgga acaggagagc gcacgaggga gcttccaggg 3900

ggaaacgcct ggtatcttta tagtcctgtc gggtttcgcc acctctgact tgagcgtcga 3960

tttttgtgat gctcgtcagg ggggcggagc ctatggaaaa acgccagcaa cgcggccttt 4020

ttacggttcc tggccttttg ctggcctttt gctcacatgt tctttcctgc gttatcccct 4080

gattctgtgg ataaccgtat taccgccttt gagtgagctg ataccgctcg ccgcagccga 4140

acgaccgagc gcagcgagtc agtgagcgag gaagcggaag 4180

<210> 27
<211> 4827
<212> DNA
<213> Artificial Sequence

<220>
<223> Env-encoding plasmid (pHCMV.Env GP) without insert Env GP
      (nucleic acid sequence)

<220>
<221> promoter
<222> 1..752
<223> /note="CMV promoter"

<220>
<221> intron
<222> 760..1404
<223> /note="Globin intron"

<220>
<221> gene
<222> 1419..1420
<223> /note="Transgene/Env GP Insert Site"

<220>
<221> polyA_signal
<222> 1435..1957
<223> /note="Globin polyA"

<220>
<221> rep_origin
<222> 2398..2986
<223> /note="ORI"

<220>
<221> gene
<222> 3157..4017
<223> /note="Ampicillin resistance (AmpR)"

<220>
<221> promoter
<222> 4018..4122
<223> /note="AmpR promoter"

<220>
<221> rep_origin
<222> 4148..4603
<223> /note="F1 ORI"

<400> 27
gagcttggcc cattgcatac gttgtatcca tatcataata tgtacattta tattggctca 60

tgtccaacat taccgccatg ttgacattga ttattgacta gttattaata gtaatcaatt 120

acggggtcat tagttcatag cccatatatg gagttccgcg ttacataact tacggtaaat 180

ggcccgcctg gctgaccgcc caacgacccc cgcccattga cgtcaataat gacgtatgtt 240

cccatagtaa cgccaatagg gactttccat tgacgtcaat gggtggagta tttacggtaa 300

actgcccact tggcagtaca tcaagtgtat catatgccaa gtacgccccc tattgacgtc 360

aatgacggta aatggcccgc ctggcattat gcccagtaca tgaccttatg ggactttcct 420

acttggcagt acatctacgt attagtcatc gctattacca tggtgatgcg gttttggcag 480

tacatcaatg ggcgtggata gcggtttgac tcacggggat ttccaagtct ccaccccatt 540

gacgtcaatg ggagtttgtt ttggcaccaa aatcaacggg actttccaaa atgtcgtaac 600

aactccgccc cattgacgca aatgggcggt aggcgtgtac ggtgggaggt ctatataagc 660

agagctcgtt tagtgaaccg tcagatcgcc tggagacgcc atccacgctg ttttgacctc 720

catagaagac accgggaccg atccagcctc cggtcgaccg atcctgagaa cttcagggtg 780

agtttgggga cccttgattg ttctttcttt ttcgctattg taaaattcat gttatatgga 840

gggggcaaag ttttcagggt gttgtttaga atgggaagat gtcccttgta tcaccatgga 900

ccctcatgat aattttgttt ctttcacttt ctactctgtt gacaaccatt gtctcctctt 960

attttctttt cattttctgt aactttttcg ttaaacttta gcttgcattt gtaacgaatt 1020

tttaaattca cttttgttta tttgtcagat tgtaagtact ttctctaatc actttttttt 1080

caaggcaatc agggtatatt atattgtact tcagcacagt tttagagaac aattgttata 1140

attaaatgat aaggtagaat atttctgcat ataaattctg gctggcgtgg aaatattctt 1200

attggtagaa acaactacac cctggtcatc atcctgcctt tctctttatg gttacaatga 1260

tatacactgt ttgagatgag gataaaatac tctgagtcca aaccgggccc ctctgctaac 1320

catgttcatg ccttcttctc tttcctacag ctcctgggca acgtgctggt tgttgtgctg 1380

tctcatcatt ttggcaaaga attcctcgac ggatccgcct aaggatccgt cgaggaattc 1440

actcctcagg tgcaggctgc ctatcagaag gtggtggctg gtgtggccaa tgccctggct 1500

cacaaatacc actgagatct ttttccctct gccaaaaatt atggggacat catgaagccc 1560

cttgagcatc tgacttctgg ctaataaagg aaatttattt tcattgcaat agtgtgttgg 1620

aattttttgt gtctctcact cggaaggaca tatgggaggg caaatcattt aaaacatcag 1680

aatgagtatt tggtttagag tttggcaaca tatgcccata tgctggctgc catgaacaaa 1740

ggttggctat aaagaggtca tcagtatatg aaacagcccc ctgctgtcca ttccttattc 1800

catagaaaag ccttgacttg aggttagatt ttttttatat tttgttttgt gttatttttt 1860

tctttaacat ccctaaaatt ttccttacat gttttactag ccagattttt cctcctctcc 1920

tgactactcc cagtcatagc tgtccctctt ctcttatgga gatccctcga cggatcggcc 1980

gcaattcgta atcatgtcat agctgtttcc tgtgtgaaat tgttatccgc tcacaattcc 2040

acacaacata cgagccggaa gcataaagtg taaagcctgg ggtgcctaat gagtgagcta 2100

actcacatta attgcgttgc gctcactgcc cgctttccag tcgggaaacc tgtcgtgcca 2160

gctgcattaa tgaatcggcc aacgcgcggg gagaggcggt ttgcgtattg ggcgctcttc 2220

cgcttcctcg ctcactgact cgctgcgctc ggtcgttcgg ctgcggcgag cggtatcagc 2280

tcactcaaag gcggtaatac ggttatccac agaatcaggg gataacgcag gaaagaacat 2340

gtgagcaaaa ggccagcaaa aggccaggaa ccgtaaaaag gccgcgttgc tggcgttttt 2400

ccataggctc cgcccccctg acgagcatca caaaaatcga cgctcaagtc agaggtggcg 2460

aaacccgaca ggactataaa gataccaggc gtttccccct ggaagctccc tcgtgcgctc 2520

tcctgttccg accctgccgc ttaccggata cctgtccgcc tttctccctt cgggaagcgt 2580

ggcgctttct catagctcac gctgtaggta tctcagttcg gtgtaggtcg ttcgctccaa 2640

gctgggctgt gtgcacgaac cccccgttca gcccgaccgc tgcgccttat ccggtaacta 2700

tcgtcttgag tccaacccgg taagacacga cttatcgcca ctggcagcag ccactggtaa 2760

caggattagc agagcgaggt atgtaggcgg tgctacagag ttcttgaagt ggtggcctaa 2820

ctacggctac actagaagaa cagtatttgg tatctgcgct ctgctgaagc cagttacctt 2880

cggaaaaaga gttggtagct cttgatccgg caaacaaacc accgctggta gcggtggttt 2940

ttttgtttgc aagcagcaga ttacgcgcag aaaaaaagga tctcaagaag atcctttgat 3000

cttttctacg gggtctgacg ctcagtggaa cgaaaactca cgttaaggga ttttggtcat 3060

gagattatca aaaaggatct tcacctagat ccttttaaat taaaaatgaa gttttaaatc 3120

aatctaaagt atatatgagt aaacttggtc tgacagttac caatgcttaa tcagtgaggc 3180

acctatctca gcgatctgtc tatttcgttc atccatagtt gcctgactcc ccgtcgtgta 3240

gataactacg atacgggagg gcttaccatc tggccccagt gctgcaatga taccgcgaga 3300

cccacgctca ccggctccag atttatcagc aataaaccag ccagccggaa gggccgagcg 3360

cagaagtggt cctgcaactt tatccgcctc catccagtct attaattgtt gccgggaagc 3420

tagagtaagt agttcgccag ttaatagttt gcgcaacgtt gttgccattg ctacaggcat 3480

cgtggtgtca cgctcgtcgt ttggtatggc ttcattcagc tccggttccc aacgatcaag 3540

gcgagttaca tgatccccca tgttgtgcaa aaaagcggtt agctccttcg gtcctccgat 3600

cgttgtcaga agtaagttgg ccgcagtgtt atcactcatg gttatggcag cactgcataa 3660

ttctcttact gtcatgccat ccgtaagatg cttttctgtg actggtgagt actcaaccaa 3720

gtcattctga gaatagtgta tgcggcgacc gagttgctct tgcccggcgt caatacggga 3780

taataccgcg ccacatagca gaactttaaa agtgctcatc attggaaaac gttcttcggg 3840

gcgaaaactc tcaaggatct taccgctgtt gagatccagt tcgatgtaac ccactcgtgc 3900

acccaactga tcttcagcat cttttacttt caccagcgtt tctgggtgag caaaaacagg 3960

aaggcaaaat gccgcaaaaa agggaataag ggcgacacgg aaatgttgaa tactcatact 4020

cttccttttt caatattatt gaagcattta tcagggttat tgtctcatga gcggatacat 4080

atttgaatgt atttagaaaa ataaacaaat aggggttccg cgcacatttc cccgaaaagt 4140

gccacctaaa ttgtaagcgt taatattttg ttaaaattcg cgttaaattt ttgttaaatc 4200

agctcatttt ttaaccaata ggccgaaatc ggcaaaatcc cttataaatc aaaagaatag 4260

accgagatag ggttgagtgt tgttccagtt tggaacaaga gtccactatt aaagaacgtg 4320

gactccaacg tcaaagggcg aaaaaccgtc tatcagggcg atggcccact acgtgaacca 4380

tcaccctaat caagtttttt ggggtcgagg tgccgtaaag cactaaatcg gaaccctaaa 4440

gggagccccc gatttagagc ttgacgggga aagccggcga acgtggcgag aaaggaaggg 4500

aagaaagcga aaggagcggg cgctagggcg ctggcaagtg tagcggtcac gctgcgcgta 4560

accaccacac ccgccgcgct taatgcgccg ctacagggcg cgtcccattc gccattcagg 4620

ctgcgcaact gttgggaagg gcgatcggtg cgggcctctt cgctattacg ccagctggcg 4680

aaagggggat gtgctgcaag gcgattaagt tgggtaacgc cagggttttc ccagtcacga 4740

cgttgtaaaa cgacggccag tgagcgcgcg taatacgact cactataggg cgaattggag 4800

ctccaccgcg gtggcggccg ctctaga 4827

## Description

The present invention relates to the field of gene therapy for the treatment of sensorineural hearing loss. In particular, the invention discloses a composition comprising a 3^{rd} generation lentiviral vector pseudotyped with a viral envelope glycoprotein capable of binding to a receptor expressed in a cell of the inner ear for use in the treatment or prevention of sensorineural hearing loss, wherein said composition has a viral titer of at least 10⁷TU/mL and is administered to the inner ear of a subject suffering from or in danger to undergo sensorineural hearing loss. The viral envelope glycoprotein is capable of binding to a receptor selected from the group consisting of the LDL-receptor (LDL-R) and LDL-R family members, the SLC1A5-receptor, the Pit1/2-receptor and the PIRYV-G-receptor. Preferably, the viral glycoprotein is MARAV-G, COCV-G, VSV-G, VSV-G ts, or PIRYV-G. The lentiviral vector further comprises a cargo sequence comprising, e.g. a protein-coding gene, a miRNA, an shRNA, a IncRNA or an sgRNA, wherein expression of said cargo sequence in the cell of the inner ear is capable of reducing, eliminating or preventing at least a symptom of sensorineural hearing loss in the subject. The invention further discloses a method for treating sensorineural hearing loss in a subject in need thereof.

Approximately 466 million people suffer from disabling hearing loss, with substantial social and economic consequences (Blanc et al., 2020, rAAV-Mediated Cochlear Gene Therapy: Prospects and Challenges for Clinical Application. J. Clin Med. 9(2), 589). Sensorineural hearing loss (SNHL) is caused by dysfunction or degeneration of structures of the inner ear, including hair cells (HC), spiral ganglion neurons (SGN), supporting cells, the tectorial membrane and the stria vascularis. Especially HC (inner and outer HC, residing in the Organ of Corti) and SGN, both of which are part of the sensory epithelium of the cochlea, play important roles in the transduction of an acoustic stimulus from a mechanical vibration into neural impulses and the transmission of the latter to the primary auditory cortex in the brain.

Current treatment options for SNHL are limited to the use of medical devices. These are based on physico-mechanical amplification of sound (hearing aid) or direct stimulation of SGN (cochlear implant). While these treatment options provide benefit to a great proportion of patients, they are not applicable to all patients, as their use is restricted if cell types relevant for transmission of the signal to the brain are too damaged or degenerated. Furthermore, hearing aids and cochlear implants do not result in a hearing quality comparable to unaffected individuals, and the resolution achieved with a cochlear implant is often only suboptimal (Blanc *et al.,* 2020). As a large percentage of SNHL cases are due to genetic aberrations, gene therapy to provide an intact copy of the defective gene in relevant cell types of the inner ear is a highly attractive alternative to current treatment options, or even the only treatment option in some cases. Moreover, genetic protection may be a suitable means to protect against or prevent the occurrence of acquired forms of SNHL, such as those caused by drug-induced, e.g., chemotherapeutic- or antibiotic-induced ototoxicity. Cell type-specific protein expression may be achieved by use of cell-specific promoters, specific viral subtypes, or a combination of both.

At present, vectors derived from adenovirus, adeno-associated virus (AAV), herpes simplex virus, vaccinia virus, helper-dependent virus and retrovirus have been tested for targeted gene delivery into the cochlea (Izumikawa et al., 2005, Auditory hair cell replacement and hearing improvement by Atoh1 gene therapy in deaf mammals. Nature Medicine 11(3), 271-276, Tao et al., 2018, Delivery of Adeno-Associated Virus Vectors in Adult Mammalian Inner-Ear Cell Subtypes Without Auditory Dysfunction. Human Gene Therapy 29(4), 492-506, Blanc *et al.,* 2020). In particular, initial proof-of-concept of successful *in vivo* delivery of marker and therapeutic genes into the inner ear has been provided with AAV vector technology, and a first in-human inner ear gene therapy clinical trial using an adenoviral vector expressing ATOH1 has been launched. As the inner ear is an enclosed organ system that is filled with endolymph and perilymph, thus enabling local restriction of the vector particles upon their injection, with no spread to other tissues expected, and a distribution of the vector within this fluid upon administration, it appears a well-suited target for gene therapy. Furthermore, the number of cells to be treated is comparatively low, so that minimal vector quantities are expected to achieve a significant effect. Nevertheless, AAV and adenoviral vector systems face the limitation of a restricted coding capacity (below 5 kb), which is too small for several genes identified to be mutated in SNHL (Dong et al., 1996, Quantitative Analysis of the Packaging Capacity of Recombinant Adeno-Associated Virus. Human Gene Therapy 7, 2101-2112).

As an alternative, scientists have turned to lentiviral vectors, which have already been successfully applied in the clinics for other, non-inner-ear-related disease entities (i.e., immuno-deficiencies) (Milone and O'Doherty, 2018, Clinical use of lentiviral vectors. Leukemia 32, 1529-1542). Lentiviral vectors are capable of mediating efficient gene deliveries to a variety of cell types in vitro and in vivo, and profit from increased cargo capacities of 10 kb or higher (Kumar et al., 2001, Systematic Determination of the Packaging Limit of Lentiviral Vectors. Human Gene Threapy 12, 1893-1905). Lentiviruses furthermore came into focus as potential gene vectors because of their ability to transduce non-dividing cells (Schambach et al., 2013, Biosafety Features of Lentiviral Vectors, Human Gene Therapy).

Hashimoto *et al.* demonstrated that lentiviral vectors may be used to treat visual symptoms of Usher syndrome in the shaker1 mouse model carrying a null mutation in the *MYO7A* gene. Usher syndrome is a rare genetic disorder that causes a combination of hearing loss and visual impairments in humans and may arise due to mutations in any one of at least 11 genes, amongst them *MYO7A. MYO7A*-deficient mice are deaf and exhibit defective melanosome localization and motility in the retinal pigment epithelium (RPE) as well as an excess of opsin in cilia of their photoreceptor cells. The authors of this study used HIV-1-derived vesicular stomatitis virus (VSV) -G-pseudotyped, 3^{rd} generation lentiviral vectors to successfully deliver cDNA of full length human *MYO7A* to the RPE and photoreceptor cells. At that time, the authors claimed that the 6962 bp *MYO7A* cDNA attached to an at least 600 bp promoter sequence constituted the largest transgene ever expressed by a viral vector in the retina. This study served as a proof-of-principle that lentiviral vectors are suitable for delivering large genes to particular cells of interest. However, the study did not address the question of whether these lentiviral vectors could also be successfully delivered to cells of the inner ear (Hashimoto et al., 2007, Lentiviral gene replacement therapy of retinas in a mouse model for Usher syndrome type 1B. Gene Therapy).

Indeed, due to the inaccessible location and the blood-labyrinth-barrier which separates the vasculature from fluids of the inner ear, successful delivery of lentiviral vectors to sensory cells of the inner ear has so far represented an insurmountable challenge.

In 1999, Han *et al.* attempted to deliver a second generation HIV-based, GFP-expressing and VSV-G-pseudotyped lentiviral vector via intracochlear perfusion into cells of the inner ear of guinea pigs. Their analysis of cochlear sections from these lentiviral vector-GFP-infused animals revealed fluorescence in cells bordering the periphery of the perilymphatic space into which the vector was infused as well as the spiral ligament, with fluorescence extending from the base of the cochlea to its apex with decreasing intensity. Importantly, however, the organ of Corti, which contains the sensory hair cells, and the spiral limbus were entirely free of GFP. Only upon *in vitro* infection of cochlear explants with lentiviral vector-GFP, did the authors observe the transduction of spiral ganglion neuros, glial cells as well as supporting cells surrounding sensory hair cells. Nevertheless, transduction of the sensory inner ear cells themselves was not detected. (Han et al., 1999, Transgene Expression in the Guinea Pig Cochlea Mediated by a Lentivirus-Derived Gene Transfer Vector. Human Gene Therapy).

Similarly, Pietola *et al.* injected second generation, self-inactivating, VSV-G-pseudotyped and GFP-expressing lentiviral vectors through the round window membrane (RMW) into the inner ear of mice and observed fluorescence in epithelial cells surrounding the scala vestibuli and scala tympani. Again, no GFP expression was observed in the organ of Corti and the tectorial membrane (Pietola et al., 2009, HOX-GFP and WOX-GFP lentivirus vectors for inner ear gene transfer. Acta Oto-Laryngologica).

Yan Wei *et al.* tested the feasibility of introducing EGFP-expressing lentiviral vectors via cochleostomy through the post-auricular route in rats. Using this delivery strategy, the authors for the first time observed EGFP fluorescence in marginal cells of the stria vascularis, the organ of Corti, the spiral nerve and spiral ganglion cells. However, successful transfection of the sensory hair cells located within the organ of Corti was not reported. In fact, injection via cochleostomy into the scala media resulted in a loss of outer hair cells at the sites of injection, which significantly impaired auditory function of the tested rats (Yan Wei et al., 2013, Effect of lentiviruses carrying enhanced green fluorescent protein injected into the scala media through a cochleostomy in rats).

Collectively, the transduction rates for inner ear cell types determined in the studies described above were not sufficient to recommend the lentiviral vector technology for gene therapy against SNHL. While the use of AAV vector technology in inner ear gene therapy has delivered promising results, the lack of corresponding data for lentiviral vectors therefore has so far prevented their testing in clinical trials. Therefore, the prior art documents do not teach a lentiviral vector system that is capable of reliably transducing cells of the sensory epithelium of the cochlea, in particular both inner and outer hair cells, and that may thus come into use in human gene therapies for treating symptoms of SNHL. This problem is solved by the present invention, in particular by the subject matter of the claims.

The present invention provides a composition for use in treating sensorineural hearing loss in a subject, wherein the composition comprises a 3^{rd} generation lentiviral vector pseudotyped with a viral envelope glycoprotein capable of binding to a receptor expressed in a cell of the inner ear, wherein said composition has a titer of at least 10⁷ TU/mL and is administered to the inner ear of the subject.

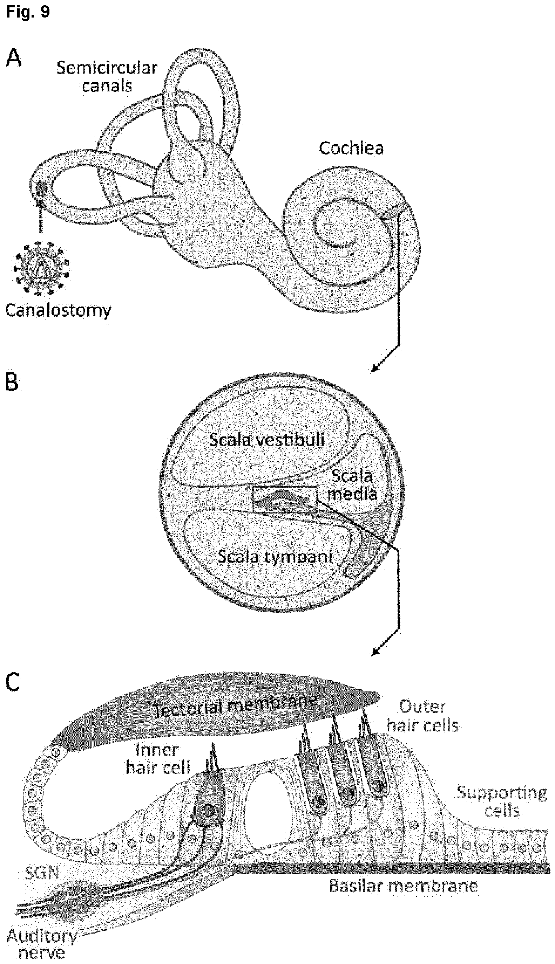

The inner ear refers to the innermost part of the vertebrate ear and is responsible for sound detection and balance. The mammalian inner ear consists of the bony labyrinth comprising the cochlea, which is required for hearing, and the vestibular system that is dedicated to balance (cf. Fig. 9). The cochlea is a spiral or snail shaped structure containing three fluid filled compartments or scalae. The scala vestibuli (vestibular duct) is filled with Na⁺-rich and K⁺-low perilymph and terminates at the oval window. The scala tympani (tympanic duct) is also filled with perilymph and abuts the round window. Located between the scala vestibule and the scala tympani is the scala media (cochlear duct). It contains endolymph, which is low in Na⁺ and high in K⁺. The stria vascularis, which forms the outer wall of the scala media, pumps Na⁺ and K⁺ against their concentration gradients to create an electrical potential between endo- and perilymph known as endocochlear potential. The scala media furthermore contains the organ of Corti that sits on top of the basilar membrane, which separates the scala media from the scala tympani. The organ of Corti consists of mechanosensory epithelial cells known as inner and outer hair cells as well as the rods of Corti and a variety of supporting cells (Morgan et al., 2020, Gene therapy as a possible option to treat hereditary hearing loss. Medizinische Genetik).

When sound waves reach the outer ear, air pressure pushes against the eardrum (tympanic membrane) and induces mechanical movement of the three ossicles malleus, incus and stapes, which in turn transmit vibrations to the oval window. The pressure applied to the oval membrane triggers movement of the perilymph and endolymph within the cochlea. Motion of these inner ear fluids results in the bending of the basilar membrane and causes a plurality of stereocilia on top of the hair cells to deflect. In consequence, mechanically gated K⁺ channels open to allow small positive ions to enter, thereby causing depolarization of the hair cells. The hair cells subsequently release neurotransmitters that bind to receptors of spiral ganglion neurons (SGN). The SGN in turn fire action potentials to the brain via the cochlear nerve (Morgan et al., 2020, Gene therapy as a possible option to treat hereditary hearing loss. Medizinische Genetik).

Sensorineural hearing loss (SNHL) is a type of hearing loss that is associated with defects in the inner ear, in particular the cochlea or the vestibulocochlear nerve. SNHL can be acquired or may originate from genetic causes. Recent estimates suggest that approximately 80% of all SNHL cases arise due to genetic factors, wherein merely 20% are due to environmental causes (Blanc *et al.,* 2020). More than 500 syndromic and non-syndromic genetic loci have been described to be associated with predispositions to hearing loss (ACMG Working Group on Update of Genetics Evaluation Guidelines for the Etiologic Diagnosis of Congenital Hearing Loss; for the Professional Practice and Guidelines Committee, 2014, American College of Medical Genetics and Genomics guideline for the clinical evaluation and etiologic diagnosis of hearing loss. Genetics in Medicine 16, 347-355). The most common form of congenital deafness in developed countries is Connexin 26 deafness (also known as GJB2-related deafness) that arises from mutations in the gene encoding Gap junction beta-2 protein (*GJB2*) (Cabanillas et al., 2018, Comprehensive genomic diagnosis of non-syndromic and syndromic hereditary loss in Spanish patients. BMC Medical Genomics 11, 58). Other syndromic forms of hearing impairment include Stickler syndrome, Waardenburg syndrome, Pendred syndrome or the aforementioned Usher syndrome. Congenital forms of hearing loss may also arise due to infections with rubella virus, cytomegalovirus or *toxoplasma gondii.* Furthermore, the process of ageing can significantly contribute to SNHL, in particular in industrialized societies. Age-related hearing loss or Presbycusis results from the degeneration of the cochlea or associated structures of the inner ear or auditory nerves and arises due to a combination of genetic and environmental factors. The main environmental stimulus for inducing hearing loss is continuous exposure to loud noise, which gradually damages the sensory and neuronal apparatus of hearing in the inner ear. Other environmental factors that may impair hearing include the use of ototoxic drugs, including cancer chemotherapy and aminoglycoside antibiotics.

The composition of the invention may be used to effectively treat SNHL that has arisen from genetic causes. The hearing loss to be treated with the composition of the invention may for instance be caused by a mutation in a gene selected from the group comprising *MYO7A, GJB2, OTOF, ATOH1, SLC26A4, TMC1, TMPRSS3, GIPC3, USH1C, RDX, CLDN14, WHRN, ESPN, ILDR1, SERPINB6, KCNQ4, POU4F3, SIX1, CCDC50, MIR96, POU3F4, EYA1, KCNQ1, GJB6, STRC, TECTA, GJB3, PRPS1, CDH23, GRXCR2, CABP2, GRXCR1, SYNE4, COL11A2, LRTOMT, BSND, PJVK, CIB2, CEACAM16, TMIE, NARS2, LHFPL5, MARVELD2, SLC22A4, KARS, SLC26A5, TPRN, ELMOD3, GPSM2, COCH, ESRRB, ADCY1, EPS8L2, CD164, OTOA, S1PR2, LOXHD1, TSPEAR, EPS8, CDC14A, MSRB3, DCDC2, PNPT1, TMEM132E, FAM65B, MYO3A, CLIC5, HGF, USH2A, TBC1D24, MET, PCDH15, PTPRQ, OTOGL, MYO6, TNC, TRIOBP, BDP1, MYO15A, DIAPH1, MYH14, DFNA5, WFS1, EYA4, COL11A2, MYH9, ACTG1, SLC17A8, GRHL2, DSPP, P2RX2, MYO1A, GFI1, TYMP* and *GFI1B.* Accordingly, the composition of the invention may be suitable to effectively improve or even eliminate at least a symptom of a subject suffering from a genetically-induced form of hearing loss, e.g., it may restore hearing capabilities to a level similar to that of a healthy subject not suffering from genetically-induced hearing loss. In some embodiments, the composition may not be able to improve or eliminate a symptom associated with SNHL, but it may at least significantly slow down or even terminate disease progression, i.e. the symptom does not worsen over time.

The composition of the invention may also be used to treat or to prevent hearing loss in patients with haploinsufficiency or with compound heterozygous mutations.

The composition of the invention may however also effectively improve or completely eliminate at least a symptom of a subject suffering from a non-genetic form of hearing loss, e.g. by stimulating the regeneration of hair cells or auditory neurons damaged due to exposure to noise or ototoxic drugs. Alternatively, it may at least significantly slow down or even terminate disease progression. The composition may also be for use in preventing sensorineural hearing loss in a subject, e.g., by protecting cells of the inner ear from hazardous agents, such as ototoxic drugs (e.g., diuretics, non-steroidal anti-inflammatory drugs, antibiotics, chemotherapeutic agents, or quinine) or noise. Preventing includes reducing the severity of potential future sensorineural hearing loss or developing sensorineural hearing loss. The composition may also be for use in increasing survival of inner ear cells that have been damaged by such a hazardous agent. Accordingly, the composition of the invention may be for use in protecting from non-genetically-induced hearing loss, e.g., by delivery of a gene or multiple genes selected from the group comprising *BDNF, GDNF, NT-3, IGF1, IGF2, CNTF, ARTN, NRTN, ATP7B, MRP1, MRP2* and *MDR1.* In particular, *BDNF, GDNF, NT-3, IGF1, IGF2, CNTF, ARTN,* and/or *NRTN* may stimulate regeneration or increase survival. *ATP7B, MRP1, MRP2* and *MDR1* may prevent damage of inner ear cells upon therapy with ototoxic drugs.

The invention likewise provides a method for treating or preventing sensorineural hearing loss in a subject in need thereof, comprising the step of administering an effective amount of the composition according to the invention to said subject.

The composition of the invention comprises a 3^{rd} generation lentiviral vector. The term "lentivirus" refers to a genus of retroviruses comprising e.g. human immunodeficiency virus (HIV), simian immunodeficiency virus (SIV), feline immunodeficiency virus (FIV) or equine infectious anemia virus (EIAV). Like that of other retroviruses, the genome of lentiviruses consists of a single-stranded (ss) positive sense RNA. During replication, the lentiviral ssRNA genome is converted into double-stranded (ds) DNA by a process known as reverse transcription. The reverse transcribed lentiviral dsDNA is subsequently integrated into the host cell's genome, which in turn replicates and transcribes the integrated lentiviral genes along with its own genes to produce new viral particles.

The lentiviral vector according to the invention may thus be derived from a lentivirus selected from the group comprising HIV-1, HIV-2, SIV, FIV or EIAV. In a preferred embodiment, the lentiviral vector is derived from HIV-1.

The lentiviral, e.g., HIV-1, genome comprises three major structural genes: *gag, pol* and *env. gag* encodes the viral matrix (MA), capsid (CA) and nucleocapsid (NC), which collectively facilitate the assembly and release of the virus particles. The *pol* gene encodes the viral enzymes protease (PR), reverse transcriptase (RT) and integrase (IN), which govern viral replication. The HIV-1 *env* encodes the viral surface glycoprotein gp160, which is subsequently cleaved to form the surface protein gp120 and the transmembrane protein gp41 during viral maturation. In addition to the structural genes *gag, pol* and *env,* the HIV-1 genome furthermore comprises the two regulatory genes *tat* and *rev* as well as the four accessory genes *vif, vpr, vpu* and *nef*. Whereas *tat* encodes a transactivator required for viral transcription, rev encodes a protein that controls both splicing and export of viral transcripts. The four accessory genes are considered non-essential for viral replication, but are believed to increase its efficiency (German Advisory Committee Blood (Arbeitskreis Blut), Subgroup 'Assessment of Pathogens Transmissible by Blood', 2016, Human Immunodeficiency Virus (HIV). Transfus Med Hemother. 43(3), 203-222).

In 1996, Naldini *et al.* reported the use of an HIV-based vector for *in vivo* gene delivery and stable transduction of non-dividing cells (Naldini et al., 1996, In vivo gene delivery and stable transduction of nondividing cells by a lentiviral vector. Science 272(5259). 263-267). Use of HIV-1-derived lentiviral vectors for *in vivo* gene transfer was however associated with considerable health risks. To meet these biosafety concerns, the first generation of lentiviral vector systems split the viral genome into three separate plasmids to avoid the formation of replication-competent viruses. The first plasmid encoded the actual vector that was to be integrated into the host cell's genome. It comprised the transgene of interest functionally linked to a suitable promoter sequence as well as *cis*-elements necessary for polyadenylation, integration, initiation of reverse transcription and packaging (e.g., the long-terminal repeats, the packaging signal, the primer binding site, the polypurine tract or the Rev-responsive element). The second plasmid, known as packaging plasmid, comprised the genes encoding the viral proteins that contribute to packaging, reverse transcription and integration of the viral genome, i.e., *gag, pol,* the regulatory genes *tat* and *rev* as well as the four accessory genes *vif, vpu, vpr* and *nef.* The third plasmid (Env plasmid) expressed the viral glycoprotein for host cell receptor binding. Due to the physical separation of the packaging genes from the rest of the viral genome, this split genome design prevented viral replication after infection of the host cell.

To further improve the safety of lentiviral vectors, a second generation system was established by removing all accessory genes from the packaging plasmid, as they were found to constitute crucial virulence factors.

With the third generation of lentiviral vector systems, so-called self-inactivating (SIN) vectors were introduced. When lentiviral vectors are integrated into a host cell genome, the transgene cassette of the provirus comprising the gene of interest is flanked by two long terminal repeats (LTRs). The presence of these LTRs may promote the emergence of potentially harmful replication-competent recombinants. In addition, viral promoter/enhancer regions located in the LTRs could induce the expression of adjacent host genes, with potentially tumorigenic consequences. Moreover, the promoter/enhancer regions in these LTRs can transcriptionally interfere with the promoter driving the transgene as well as the neighboring genes. Therefore, promoter/enhancer sequences of the viral 3' LTR were removed by deleting a particular region inside the LTR (U3) from the DNA that was used to produce the viral RNA. Deletion of the U3 region effectively abolished the transcriptional activity of the LTR. Furthermore, *tat,* a regulatory gene driving viral transcription, was deleted from the packaging plasmid, whereas the second regulatory gene *rev* was provided from another separate, fourth, plasmid (Zufferey et al., 1998, Self-Inactivating Lentivirus Vector for Safe and Efficient in Vivo Gene Delivery. J Virol 72(12), 9873-9880; Schambach et al., 2013, Biosafety Features of Lentiviral Vectors. Human Gene Therapy 24, 132-142). In case the 5' promoter driving the viral genomic mRNA is not HIV-derived and substituted by a CMV or RSV promoter, an extra tat plasmid may be omitted.

Accordingly, the lentiviral vector according to the invention is replication incompetent due to the split packaging design and self-inactivating (SIN) due to a deletion in the U3 region of the 3' LTR. It further lacks the genes *vif, vpr, vpu, nef,* and, optionally, *tat.*

Therefore, the lentiviral vector according to the invention comprises the following features
a) a 5' LTR comprising a constitutively active heterologous promoter at the U3 position, a repeat region (R) and a U5 region,
b) a 5' UTR comprising a primer binding site (PBS), a splice donor site (SD), a packaging signal (ψ), a Rev-responsive element, and, optionally, a splice acceptor (SA) site,
c) an internal enhancer/promoter region operably linked to a cargo sequence,
d) RNA processing elements optionally comprising a Woodchuck hepatitis virus posttranscriptional regulatory element (PRE), and
e) a 3' LTR with a deleted (SIN) U3 region, a repeat region (R) and a U5 region.

The splice acceptor site is optional. The splice donor site is important, but vectors lacking it are functional. Presence of RNA processing elements such as a Woodchuck hepatitis virus posttranscriptional regulatory element (PRE) contribute to efficiency, and vectors without them are typically not generated in high titers. However, they can be concentrated.

Suitable 3^{rd} generation lentiviral vectors are e.g., known in the art and/or can be prepared by the skilled person. An exemplary lentiviral vector that can be used in the invention comprises a nucleic acid sequence of SEQ ID NO: 1. The lentiviral vector may also comprise a nucleic acid sequence having at least 80%, at least 85%, at least 90%, at least 95%, at least 96%, at least 97%, at least 98%, or at least 99% sequence identity to SEQ ID NO: 1. It may also consist of SEQ ID NO: 1. Alternatively, the lentiviral vector according to the invention may comprise a different promoter region than SEQ ID NO:1, e.g., another suitable promotor as taught herein.

Optionally, the viral enhancer/promoter regions located at the U3 position of the 5' LTR in the lentiviral vector may be replaced by a heterologous promoter to enhance expression of genomic RNA and to compensate for the loss of the lentiviral Tat protein. Therefore, the heterologous promoter preferably is a strong promoter, i.e., it drives constitutive expression of genes under its control. It may be selected from the group comprising a Rous sarcoma virus (RSV) promoter, a human cytomegalovirus (CMV) promoter, an HIV promoter (in case a lentiviral vector is used that is not derived from HIV-1 or HIV-2) or a eukaryotic promoter such as e.g., EF1a, PGK1, UBC, or human beta actin. It may also be an inducible promoter that only drives gene expression in the presence or absence of a certain stimulus, in particular, when expression of the vector would be toxic for the packaging cell. It may, e.g., be a Tet-regulated promoter, i.e., it may comprise a tetracycline response element (TRE) that can be bound by a tetracycline transactivator (tTA) protein in the presence of tetracycline or an analogue thereof, e.g. doxycycline. Preferably, the heterologous promoter is a constitutively active promoter derived from RSV or CMV.

The 5' UTR packaging signal (ψ) interacts with the nucleocapsid (NC) of the assembling Gag proteins to mediate packaging. Optimal packaging furthermore requires the trans-activation response element (TAR), U5, the primer binding site (PBS) and the group-specific antigen (Gag). The Rev protein additionally enhances the encapsidation of RNA containing the Rev-responsive element (RRE). The RRE also allows for higher expression of the viral genome and may suppress aberrant splice events (Schambach *et al.,* 2013). The 5' UTR may, optionally, further comprise multiple splice sites. In particular, the lentiviral vector may comprise a 5'UTR splice donor (SD) site and, optionally, a splice acceptor site (SA). In an optional embodiment, the 5' UTR may further comprise a polypurine tract (PPT) region, preferably downstream of the RRE. The PPT region is thought to increase the efficiency of reverse transcription and may even mediate nuclear entry of the lentiviral pre-integration complex, which forms after uncoating of the viral particle upon entry into the host cell (Schambach *et al.,* 2013).

The internal promoter/enhancer region is preferably a strong promoter, i.e., it drives constitutive expression of the cargo sequence under its control. It may be a promoter selected from the group comprising a viral promoter, a cellular promoter, a cell-specific promoter, an inducible promoter or a synthetic promoter.

It may, e.g., be a viral promoter selected from the group comprising a promoter derived from CMV, spleen focus-forming virus (SFFV), myeloproliferative sarcoma virus (MPSV), murine embryonal stem cell virus (MESV), murine leukemia virus (MLV) and simian virus 40 (SV40). Preferably, the internal promoter is a promoter derived from CMV or SFFV, most preferably from CMV. In another preferred embodiment, the promoter may also be a CAG promoter, i.e. a composite construct consisting of the CMV enhancer fused to the chicken beta-actin promoter and the rabbit beta-Globin splice acceptor site. The internal promoter/enhancer region may alternatively be a cellular promoter selected from the group comprising EF1a, PGK1 and UBC. It may also be a promoter that enables expression of the cargo sequence in a cell-specific manner. It may, e.g., be a hair cell-specific promoter selected from the group comprising POU4F3, POU3F4, ATOH1, Prestin, Pendrin and MYO7A or an SGN-specific promoter selected from the group comprising e.g. MAP1B, SYN, and NSE.

The internal promoter/enhancer may also be an inducible promoter, especially, if expression of the cargo sequence may lead to cytotoxicity. The promoter may thus be selected from the group comprising a TET-inducible promoter, a Cumate-inducible promoter, an electrosensitive promoter and an optogenetic switch. Electrosensitive promoters or optogenetic switches may be especially useful when expression of the cargo sequence is to be controlled by a cochlea implant.

The internal promoter/enhancer region may also be a hybrid or a synthetic promoter selected from the group comprising CAG and MND. A hybrid promoter is composed of several regulatory elements from different promoters/enhancers, which are joined to build a new promoter combining the desired features. In contrast, a synthetic promoter essentially consists of an array of transcription factor binding sites fused to a minimal promoter. A preferred hybrid promoter used for driving cargo sequence expression is CAG.

Finally, the internal enhancer/promoter region may comprise a chromatin opening element, i.e., a DNA sequence consisting of methylation-free CpG islands that either encompasses a housekeeping gene promoter or is operably linked to a heterologous promoter to confer reproducible, stable transgene expression.

The 3' UTR of the lentiviral vector according to the invention may comprise an RNA processing element, e.g., a Woodchuck hepatitis virus posttranscriptional regulatory element (PRE). The PRE may enhance cargo sequence expression by up to 10-fold and contributes to high virus titers. It also improves mRNA stability as well as translation efficiency and reduces read-through transcription into adjacent cellular sequences (Schambach *et al.,* 2013).

To further prevent read-through transcription, the 3' end of the lentiviral vector may comprise upstream sequence elements (USE) that can be inserted into the 3' U3 region to improve 3' end processing and transcriptional termination. Preferably, the USE is a recombinant direct repeat of the USE derived from SV40 (2xSV USE). The 3' UTR may, optionally, further comprise target sites for differentiation-specific cellular miRNAs to enable post-transcriptional targeting of the vector mRNA.

Upon random or semi-random integration of a lentiviral vector into or next to a host cell's gene, the vector may act as a mutagen that dysregulates the gene's expression. In an optional embodiment, the space created by deletion of the U3 region in the 3'LTR of the lentiviral vector may therefore comprise an insulator sequence. Insulator sequences constitute barriers between regulator gene regions and thus may prevent vector-driven dysregulation of adjacent proto-oncogenes, i.e., cellular genes whose erroneous expression may provoke tumorigenesis (Schambach *et al.,* 2013).

To further increase biosafety of the composition according to the invention, the packaging plasmid encoding for Gag-Pol may be designed to comprise an inhibitory mutation in the *pol* region encoding for the integrase protein to render the lentiviral vector integration-deficient. Possible mutations may alter a triad of amino acid residues at the catalytic core of the integrase enzyme, e.g., D64, D116 and E152. Alternatively, amino acid changes may be introduced into the integrase DNA attachment site e.g. by mutating nucleotides within a 12 base-pair (bp) region of the vector 5' LTR U3 region or an 11 bp region of the 3'LTR U5 region (Shaw and Cornetta, 2014, Design and Potential of Non-Integrating Lentiviral Vectors. Biomedicines 2(1), 14-35). Therefore, in an optional embodiment, the lentiviral vector according to the invention is a non-integrating lentiviral vector.

The lentiviral vector of the composition for use according to the invention furthermore comprises a cargo sequence, wherein expression of said cargo sequence in said cell of the inner ear is capable of reducing, eliminating or preventing at least a symptom of sensorineural hearing loss in the subject. The cargo sequence is operably linked to the internal promoter/enhancer region. It comprises a nucleic acid selected from the group comprising a protein-coding gene, a miRNA, an shRNA or a IncRNA.

Preferably, the cargo sequence is a protein-coding gene, wherein a reduced or erroneous expression of said gene is associated with the development of the sensorineural hearing loss in the subject. Accordingly, the cargo sequence may be an intact copy of any gene known to drive SNHL when being mutated. It may, for instance, be a gene such as *MYO7A, GJB2, OTOF, ATOH1, SLC26A4, TMC1, TMPRSS3, GIPC3, USH1C, RDX, CLDN14, WHRN, ESPN, ILDR1, SERPINB6, KCNQ4, POU4F3, SIX1, CCDC50, MIR96, POU3F4, EYA1, KCNQ1, GJB6, STRC, TECTA, GJB3, PRPS1, CDH23, GRXCR2, CABP2, GRXCR1, SYNE4, COL11A2, LRTOMT, BSND, PJVK, CIB2, CEACAM16, TMIE, NARS2, LHFPL5, MARVELD2, SLC22A4, KARS, SLC26A5, TPRN, ELMOD3, GPSM2, COCH, ESRRB, ADCY1, EPS8L2, CD164, OTOA, S1PR2, LOXHD1, TSPEAR, EPS8, CDC14A, MSRB3, DCDC2, PNPT1, TMEM132E, FAM65B, MYO3A, CLIC5, HGF, USH2A, TBC1D24, MET, PCDH15, PTPRQ, OTOGL, MYO6, TNC, TRIOBP, BDP1, MYO15A, DIAPH1, MYH14, DFNA5, WFS1, EYA4, COL11A2, MYH9, ACTG1, SLC17A8, GRHL2, DSPP, P2RX2, MYO1A, GFI1, TYMP,* or *GFI1B.*

The gene may, e.g., be a transcription factor or a combination of several different transcription factors that induce(s) transdifferentiation of a given cell into a cell required for hearing. For example, expression of one or more transcription factors in the inner ear may induce the differentiation of glial cells within the organ of Corti into auditory neurons. Alternatively, expression of one or more transcription factor(s) such as, e.g., Atoh1, Gfi1, Pou4f3 or Pou3f4 may induce differentiation of non-sensory auditory epithelial cells into new functional hair cells. Similarly, expression of the transcription factors Sox2, NeuroG1 or NeuroD1 may drive the differentiation of cells into SGN. For this purpose, the composition of the invention may furthermore comprise growth factors, e.g. a neurotrophic factor such as BDNF, GDNF, NT3 or IGF. Alternatively, the cargo sequence may encode said growth factors.

Under certain circumstances, it may be beneficial to inactivate the translated protein encoded by the cargo sequence after a given time, e.g., due to arising cytotoxicity. Therefore, the coding sequence may be designed to further encode an instable degradation or destabilization domain. The cargo sequence may for instance encode a rapidly degradable E. *coli-*derived dihydrofolate reductase (ecDHFR)-domain fused to the protein of interest. The ecDHFR-domain can be stabilized in a reversible and dose-dependent manner by the small antibiotic ligand trimethoprim (TMP). Upon withholding TMP from the transduced cell, the instability of the ecDHFR-domain is conferred to the fused protein of interest, causing rapid degradation of the entire fusion protein (Iwamoto et al., 2010, A general chemical method to regulate protein stability in the mammalian central nervous system. Chem Biol. 17(9), 981-988). A person skilled in the art may select another destabilization domain, e.g. that has been engineered in the past (derived, e.g., from DHFR, FRB or FKBP12) and that may be similarly used to control the stability of the protein encoded by the cargo sequence (Levy et al., 1999, Analysis of a conditional degradation signal in yeast and mammalian cells. Eur J Biochem. 259(1-2), 244-252; Stankunas et al., 2003, Conditional protein alleles using knockin mice and a chemical inducer of dimerization. Mol Cell. 12(6), 1615-1624).

The cargo sequence preferably is a complementary DNA (cDNA) that has been generated via reverse transcription from messenger RNA (mRNA) transcribed from the gene of interest and that lacks introns normally present in the gene of interest. Alternatively, it may be the complete gene of interest comprising introns and, optionally, regulatory regions provided that the length of the cargo sequence does not exceed the packaging limit of the lentiviral vector. The cargo sequence may alternatively be synthetically engineered, it may, e.g., be codon-optimized to ensure efficient expression of the gene of interest in the subject, e.g., codon-optimized for expression in human cells. The cargo sequence may, optionally, further comprise an internal ribosomal entry site (IRES), i.e. an RNA element that mediates mRNA translation in the absence of a 5' cap and allows for co-expression of multiple genes under the control of a single promoter.

In an alternative embodiment, the cargo sequence may be transcribed into a regulatory RNA selected from the group comprising a microRNA, a short hairpin RNA or a long non-coding RNA. Preferably, the cargo sequence is transcribed into a microRNA (miRNA), i.e., a regulatory RNA molecule that is originally transcribed as a hairpin stem loop before being processed into a short single-stranded 20-25 nt RNA molecule capable of post-transcriptionally silencing gene expression. Endogenously transcribed miRNAs are estimated to regulate more than 60 % of all protein coding genes and to modulate virtually every cellular pathway (Catalanotto et al., 2016, MicroRNA in Control of Gene Expression: An Overview of Nuclear Functions. International journal of molecular sciences, 17(10), 1712). Therefore, the cargo regulatory RNA, e.g., miRNA, according to the invention may be an RNA whose reduced or erroneous expression is associated with the development of SNHL in a subject. Alternatively, the cargo regulatory RNA, e.g., miRNA, according to the invention may also be an RNA that targets a gene whose elevated or erroneous expression is associated with the development of SNHL in a subject.

In yet another embodiment, the cargo sequence of the invention may be transcribed into a short hairpin RNA (shRNA). shRNAs are synthetic RNA molecules that, similar to miRNAs, can silence gene expression via RNA interference. The cargo shRNA according to the invention may target a gene whose elevated or erroneous expression is associated with the development of the sensorineural hearing loss in the subject.

Alternatively, the cargo sequence may also be transcribed into a long non-coding RNA (IncRNA). LncRNAs are non-coding transcripts larger than 200 nt that may fulfill diverse functions, e.g., in regulation of gene transcription, splicing, translation or epigenetic regulation. The cargo IncRNA according to the invention may be a IncRNA whose reduced or erroneous expression is associated with the development of the sensorineural hearing loss in the subject, or wherein said IncRNA may regulate expression of a gene whose elevated or erroneous expression is associated with the development of the sensorineural hearing loss in the subject.

In yet another embodiment, the cargo sequence may encode a Cas, e.g., Cas9 protein, and further comprise a single guide RNA (sgRNA) for efficient CRISPR/Cas delivery to inner ear cells. The clustered regularly interspaced palindromic repeats (CRISPR)/CRISPR associated protein (Cas), e.g., Cas9 system may be used for targeted genome-editing. The expressed sgRNA finds and binds to a specific sequence in the host cell's DNA and the Cas enzyme subsequently introduces double or single stranded cuts into specific adjacent DNA regions. Repair of these cuts may result in target gene knock-out due to introduction of insertions or deletions. Alternatively, the system may be used to introduce a stretch of DNA into the cutting site, e.g. an intact copy of a mutated gene.

In one embodiment, expression of the cargo sequence in a cell of the inner ear may also treat or prevent hearing loss arising from a non-genetic cause. Cargo sequence expression in a cell of the inner ear may, for instance, compensate for the loss of hair cells due to infection or exposure to loud noise, ototoxic drugs or mechanical trauma. The cargo sequence may, e.g., be a gene or multiple genes selected from the group comprising *BDNF, GDNF, NT-3, IGF1, IGF2, CNTF, ARTN,* and *NRTN.* It may also be a regulatory RNA (e.g., a miRNA) that cell-specifically silences the expression of a gene that normally prevents regeneration of sensory hair cells in the organ of Corti. It may, e.g., be a miRNA that targets the retinoblastoma gene (*Rb1*)*,* which was reported to maintain hair cells and supporting cells in a post-mitotic state. Previous reports have demonstrated that targeted deletion of *Rb1* may trigger proliferation of functional hair cells *in vivo* (Sage et al., 2005, Proliferation of functional hair cells in vivo in the absence of the retinoblastoma protein. Science 307(5712), 1056-1058.). The cargo sequence may also encode, e.g., a transcription factor that induces transdifferentiation of cells into sensory hair cells or SGNs, a growth factor, or a molecular drug pump / transporter that actively exports and thereby removes a hazardous agent, e.g., an ototoxic drug from a cell of the inner ear, e.g., a gene or multiple genes selected from the group comprising *ATP7B, MRP1, MRP2* and *MDR1.*

Preferably, expression of the cargo sequence according to the invention in a cell of the inner ear is capable of reducing, eliminating or preventing at least a symptom of sensorineural hearing loss in the subject. In the context of the invention, "reducing a symptom" means that the severity of a symptom may be alleviated, e.g. upon administration of the composition, the condition of a subject with severe hearing impairments may be ameliorated to an extent to which his/her hearing capabilities have been significantly improved compared to the hearing capabilities prior to administration. In another embodiment, a symptom of sensorineural hearing loss may even be completely eliminated in a subject upon expression of the cargo sequence, i.e., the subject suffering from SNHL may have his/her hearing capabilities fully restored upon administration of the composition according to the invention. In yet another embodiment, the expression of the cargo sequence may prevent a symptom of SNHL in a subject, e.g., completely prevent or reduce the incidence or severity of said symptom. For instance, the composition according to the invention may be administered to a fetus that has been diagnosed with a genetic deficiency associated with a later on-set development of SNHL. Expression of the cargo sequence may compensate for the genetic defects and thus prevent the emergence of SNHL in later life. Preventing a symptom of SNHL may also refer to protecting a subject from the hazardous effects of external stimuli. Preventing includes reducing the severity and/or incidence of potential future sensorineural hearing loss or developing sensorineural hearing loss. Expression of the cargo sequence in a sensory hair cell or auditory neuronal cell may, e.g., confer resistance to specific ototoxic drugs that are employed during chemotherapeutic treatment of a cancer patient.

The lentiviral vector according to the invention is pseudotyped with a viral envelope glycoprotein capable of binding to a receptor expressed in a cell of the inner ear.

The term pseudotyping refers to the generation of viral vectors that carry foreign viral envelope proteins on their surface. Viral surface glycoproteins modulate viral entry into the host cell by interacting with particular cellular receptors to induce membrane fusion. For instance, the native HIV envelope glycoprotein specifically binds the receptor CD4 as well as co-receptors CXCR4 or CCR5, which effectively limits the potential target cells of HIV to CD4⁺ T-cells and monocytes. To change or broaden the selection of cells that may be transduced, lentiviral vectors are therefore usually pseudotyped with heterologous envelope glycoproteins both from related and unrelated viruses. Dependent on the choice of glycoprotein used for pseudotyping, the tropism of the virus, i.e., the range of host cells that can be infected by the lentivirus, can thus be either expanded or directed to particular cell types of interest.

Preferably, in the context of the invention, the lentiviral vector is pseudotyped with a viral envelope glycoprotein capable of binding to a receptor selected from the group consisting of the LDL-receptor and LDL-R family members, the SLC1A5-receptor, the Pit1/2-receptor and the PIRYV-G-receptor.

Most preferably, said receptor is an LDL-receptor or LDL-R family member, e.g., an LDL-receptor.

The evolutionary conserved Low-Density Lipoprotein (LDL) receptor is a cell surface receptor that mediates endocytosis of cholesterol-rich LDL. It is ubiquitously expressed in all nucleated cells, in particular inside the liver where it removes the majority of LDL from the circulation. In addition, the LDL-receptor serves as an entry site for a wide variety of viruses, including Hepatitis C virus (Agnello et al., 1999, Hepatitis C virus and other flaviviridae viruses enter cells via a low density lipoprotein receptor. Proc Natl Acad Sci 96(22), 12766-12771). In 2013, Finkelshtein *et al.* identified the LDL-receptor as the major entry port of vesicular stomatitis virus (VSV), which explained why viruses and viral vectors pseudotyped with the VSV envelope glycoprotein G (VSV-G) displayed an extraordinarily broad tropism (Finkelshtein et al., 2013, LDL receptor and its family members serve as the cellular receptors for vesicular stomatitis virus. PNAS 110(18), 7306-7311). The LDL-receptor family member megalin was found to be expressed in various cell types of the cochlea. However, no evidence for megalin expression was found in SGNs or inner and outer hair cells or supporting cells in the organ of Corti (Hosokawa et al., 2018, Immunohistochemical localization of megalin and cubilin in the human inner ear. Brain Res. 1701, 153-160). The present inventors unexpectedly found that the LDL receptor is expressed in inner ear cells, for example, in inner and outer hair cells as well as in SGNs. Accordingly, these cells can be transduced by viruses pseudotyped with VSV-G.

The viral envelope glycoprotein capable of binding the LDL-receptor or LDL-R family members may be, e.g., MARAV-G, COCV-G, VSV-G or VSV-G ts.

Lentiviral vectors are most commonly pseudotyped with vesicular stomatitis virus envelope glycoprotein (VSV-G). As the broadly expressed LDL-receptor and other LDL-receptor family members serve as entry sites for VSV, the VSV-G envelope confers a very broad tropism to viral vectors. Upon binding of VSV-G to the LDL-receptor, the virion is taken up by the host cell via clathrin-mediated endocytosis. Besides providing high tropism, VSV-G furthermore significantly increases the stability of the lentiviral vector, which allows for efficient concentration of viral titers by centrifugation, another essential feature for effective, virus-mediated gene transfer. Accordingly, pseudotyping of lentiviral vectors with VSV-G allows for robust transduction into a wide range of different cell types. Using the composition of the invention, the inventors were able to transduce the auditory, hair-cell-like cell line HEI-OC1 as well as primary dissociated SGNs with VSV-G-pseudotyped lentiviral vectors. The inventors could further prevent progressive hearing loss in a MYO7A-deficient shaker-1 mouse model by injecting a VSV-G-pseudotyped lentiviral vector into the vestibular labyrinth of the inner ear. Subsequent analysis revealed, for the first time, successful transduction of cochlear cells, including inner and outer hair cells and SGNs with lentiviral vectors. Therefore, in one embodiment, the lentiviral vector of the invention is pseudotyped with VSV-G, e.g., wild-type VSV-G or a VSV-G derivative capable of binding to the LDL-receptor or LDL-R family members. Preferably, the wild type VSV-G is a glycoprotein derived from the Indiana VSV serotype and has an amino acid sequence having at least 80%, preferably at least 85%, at least 90%, at least 95%, at least 96%, at least 97%, at least 98%, at least 99% or 100% sequence identity to SEQ ID NO: 21. Optionally, it is encoded by a nucleic acid sequence having at least 80%, preferably at least 85%, at least 90%, at least 95%, at least 96%, at least 97%, at least 98%, at least 99% or 100% sequence identity to SEQ ID NO: 20. It may also be derived from the New Jersey VSV serotype and have an amino acid sequence having at least 80%, preferably at least 85%, at least 90%, at least 95%, at least 96%, at least 97%, at least 98%, at least 99% or 100% sequence identity to SEQ ID NO: 23. Optionally, it is encoded by a nucleic acid sequence having at least 80%, preferably at least 85%, at least 90%, at least 95%, at least 96%, at least 97%, at least 98%, at least 99% or 100% sequence identity to SEQ ID NO: 22. To achieve higher particle stability upon *in-vivo* administration and to evade potential recognition by the host's complement system, a thermostable and complement-resistant VSV-G glycoprotein (VSV-G ts) may alternatively be used, which may have an amino acid sequence having at least 80%, preferably at least 85%, at least 90%, at least 95%, at least 96%, at least 97%, at least 98%, at least 99% or 100% sequence identity to SEQ ID NO: 3. It may, optionally, be encoded by a nucleic acid sequence having at least 80%, preferably at least 85%, at least 90%, at least 95%, at least 96%, at least 97%, at least 98%, at least 99% or 100% sequence identity to SEQ ID NO: 2, and capable of binding to the LDL-R or LDL-R family members.

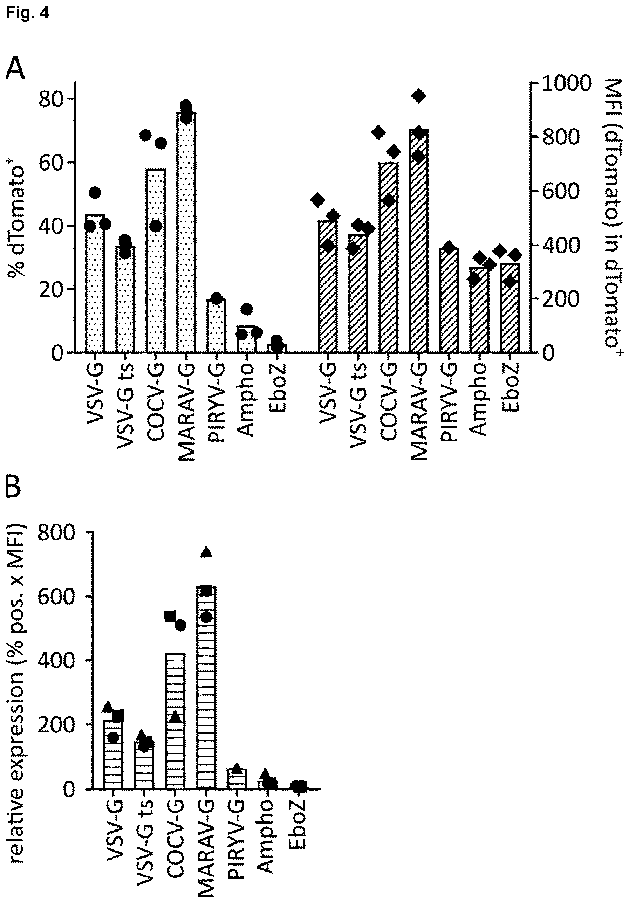

However, in a preferred embodiment, the lentiviral vector according to the invention is pseudotyped with a COCV-G glycoprotein, i.e., a glycoprotein derived from Cocal virus. COCV-G is capable of binding to the LDL-receptor and it may, e.g., have an amino acid sequence having at least 80%, preferably at least 85%, at least 90%, at least 95%, at least 96%, at least 97%, at least 98%, at least 99% or 100% sequence identity to SEQ ID NO: 5. Optionally, COCV-G may be encoded by a nucleic acid sequence having at least 80%, preferably at least 85%, at least 90%, at least 95%, at least 96%, at least 97%, at least 98%, at least 99% or 100% sequence identity to SEQ ID NO: 4. In 2010, Trobridge *et al.* addressed some of the disadvantages of VSV-G-pseudotyped lentiviral vectors, including the aforementioned cytotoxicity associated with constitutive VSV-G expression, but also the inactivation of VSV-G-pseudotyped vectors by human serum complement. The authors of this study demonstrated that lentiviral vectors pseudotyped with COCV-G glycoprotein derived from Cocal virus could be produced at titers as high as VSV-G-pseudotyped vectors, and exhibited both broad tropism and high stability. Importantly, pseudotyping with COCV-G also confers resistance to viral inactivation by human serum (Trobridge et al., 2010, Cocal-pseudotyped Lentiviral Vectors Resist Inactivation by Human Serum and Efficiently Transduce Primate Hematopoietic Repopulating Cells. Molecular Therapy 18(4), 725-733). The present inventors made the surprising discovery that lentiviral vectors pseudotyped with COCV-G transduced the murine inner-ear-derived hair-cell-like cell line HEI-OC1 with even higher efficiency than vectors pseudotyped with VSV-G (Fig. 4).

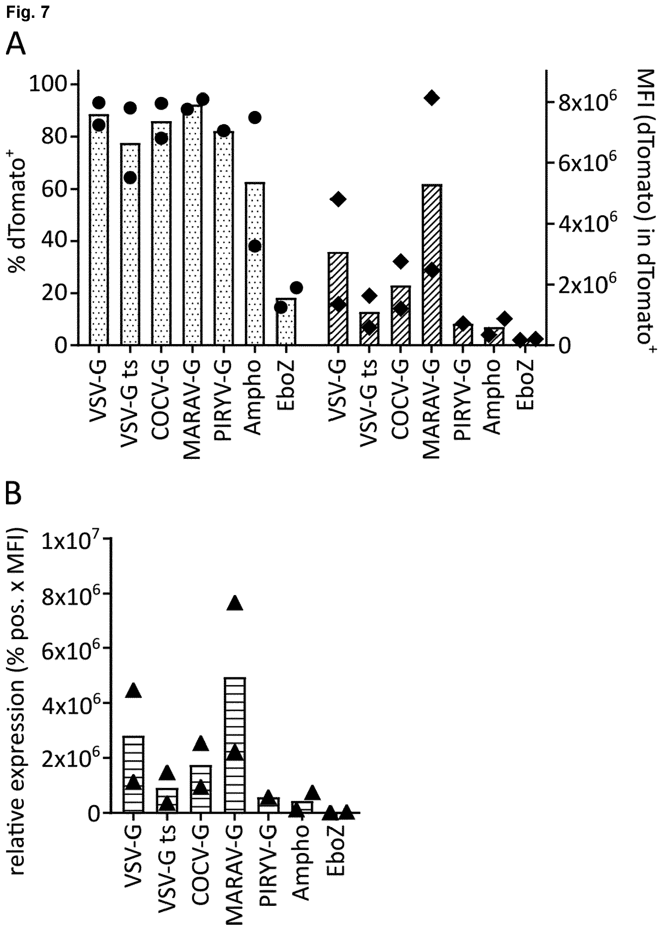

In a particularly preferred embodiment, the glycoprotein used for pseudotyping the lentiviral vector of the invention capable of binding to the LDL-receptor is MARAV-G. MARAV-G may, e.g., have an amino acid sequence having at least 80%, preferably at least 85%, at least 90%, at least 95%, at least 96%, at least 97%, at least 98%, at least 99% or 100% sequence identity to SEQ ID NO: 7. Optionally, it may be encoded by a nucleic acid sequence having at least 80%, preferably at least 85%, at least 90%, at least 95%, at least 96%, at least 97%, at least 98%, at least 99% or 100% sequence identity to SEQ ID NO: 6. MARAV-G is an envelope glycoprotein derived from Maraba virus. Maraba virus is a rhabdovirus best known for displaying broad tropism for both human and murine cancer cells. The inventors could show that the use of lentiviral vectors pseudotyped with MARAV-G allows for an unexpectedly efficient transduction of both HEI-OC1 cells as well as dissociated primary SGNs. In fact, in both cell types, MARAV-G-pseudotyped lentiviral vectors performed significantly better than their VSV-G- or COCV-G-pseudotyped counterparts (Fig. 4 and Fig. 7).

Alternatively, the lentiviral vector according to the invention may also be pseudotyped with a viral envelope glycoprotein capable of binding the SLC1A5-receptor. SLC1A5 was originally identified as a neutral amino acid transporter that exchanges glutamine with other neutral amino acids such as serine, asparagine or threonine in a Na⁺-dependent manner (Scalise et al., 2018, The Human SLC1A5 (ASCT2) Amino Acid Transporter: From Function to Structure and Role in Cell Biology. Front Cell Dev Biol.). Expression of SLC1A5 was reported for lung, skeletal muscle, large intestine, kidney, testis, T-cells, brain and adipose tissue (Scalise *et al.,* 2018). SLC1A5 is, however, also used as a common entry port by a group of retroviruses, including the feline endogenous virus (RD114) and baboon endogenous virus (BaEV) (Marin et al., 2003, N-Linked Glycosylation and Sequence Changes in a Critical Negative Control Region of the ASCT1 and ASCT2 Neutral Amino Acid Transporters Determine Their Retroviral Receptor Functions. Journal of Virology 77(5), 2936-2945).

Accordingly, the lentiviral vector of the invention may also be pseudotyped with a viral envelope glycoprotein derived from RD114 glycoprotein (GP) that is capable of binding the SLC1A5-receptor. Such a glycoprotein may have an amino acid sequence having at least 80%, preferably at least 85%, at least 90%, at least 95%, at least 96%, at least 97%, at least 98%, at least 99% or 100% sequence identity to SEQ ID NO: 9. Optionally, it may be encoded by a nucleic acid sequence having at least 80%, preferably at least 85%, at least 90%, at least 95%, at least 96%, at least 97%, at least 98%, at least 99% or 100% sequence identity to SEQ ID NO: 8. It thus may also be RD114 GP. A suitable RD114 GP derivative may, e.g., be RD114\TR, which has been modified to comprise a cytoplasmic tail (designated TR) of amphotropic murine leukemia virus (MLV-A) glycoprotein in its intracytoplasmic domain (Sandrin et al., 2002, Lentiviral vectors pseudotyped with a modified RD114 envelope glycoprotein show increased stability in sera and augmented transduction of primary lymphocytes and CD34+ cells derived from human and nonhuman primates. Blood 100(3), 823-32). It may also be a glycoprotein derived from BaEV GP that is capable of binding the SLC1A5-receptor. Such a glycoprotein may, e.g., have an amino acid sequence having at least 80%, preferably at least 85%, at least 90%, at least 95%, at least 96%, at least 97%, at least 98%, at least 99% or 100% sequence identity to SEQ ID NO: 11. Optionally, it may be encoded by a nucleic acid sequence having at least 80%, preferably at least 85%, at least 90%, at least 95%, at least 96%, at least 97%, at least 98%, at least 99% or 100% sequence identity to SEQ ID NO: 10. It thus may also be BaEV GP. Suitable BaEV GP derivatives may, e.g., be BaEV\TR or BaEV\Rless. The latter harbors a specific deletion of the R peptide sequence in its cytoplasmic tail (Girard-Gagnepain et al., 2014, Baboon envelope pseudotyped LVs outperform VSV-G-LVs for gene transfer into early-cytokine-stimulated and resting HSCs. Blood 124(8), 1221-1231).

The lentiviral vector of the invention may also be pseudotyped with a viral envelope glycoprotein capable of binding the Pit1/2-receptor. Pit1 and Pit2 are sodium-dependent phosphate transporters that play a vital role in phosphate transport to ensure normal cellular function. Pit1 and Pit2 serve also as receptors for the gibbon ape leukemia virus (GALV) and the amphotropic murine leukemia virus (A-MuLV), respectively (Leverett et al., 1998, Entry of amphotropic murine leukemia virus is influenced by residues in the putative second extracellular domain of its receptor, Pit2. J Virol. 72(6), 4956-61). Therefore, the viral envelope glycoprotein may be derived from GALV. GALV GP is capable of binding the Pit1/2-receptor, and it may, e.g., have an amino acid sequence having at least 80%, preferably at least 85%, at least 90%, at least 95%, at least 96%, at least 97%, at least 98%, at least 99% or 100% sequence identity to SEQ ID NO: 13. Optionally, it may be encoded by a nucleic acid sequence having at least 80%, preferably at least 85%, at least 90%, at least 95%, at least 96%, at least 97%, at least 98%, at least 99% or 100% sequence identity to SEQ ID NO: 12. The viral glycoprotein may be alternatively derived from A-MuLV/Ampho. Such an Ampho GP is capable of binding the Pit1/2-receptor, and it may, e.g., have an amino acid sequence having at least 80%, preferably at least 85%, at least 90%, at least 95%, at least 96%, at least 97%, at least 98%, at least 99% or 100% sequence identity to SEQ ID NO: 15. Optionally, it may be encoded by a nucleic acid sequence having at least 80%, preferably at least 85%, at least 90%, at least 95%, at least 96%, at least 97%, at least 98%, at least 99% or 100% sequence identity to SEQ ID NO: 14. It may also be pseudotyped with a glycoprotein capable of binding the Pit1/2-receptor and derived from 10A1 MLV having an amino acid sequence having at least 80%, preferably at least 85%, at least 90%, at least 95%, at least 96%, at least 97%, at least 98%, at least 99% or 100% sequence identity to SEQ ID NO: 17. Thus, the glycoprotein may, optionally, be encoded by a nucleic acid sequence having at least 80%, preferably at least 85%, at least 90%, at least 95%, at least 96%, at least 97%, at least 98%, at least 99% or 100% sequence identity to SEQ ID NO: 16.

The lentiviral vector of the invention may be alternatively pseudotyped with PIRYV-G having an amino acid sequence having at least 80%, preferably at least 85%, at least 90%, at least 95%, at least 96%, at least 97%, at least 98%, at least 99% or 100% sequence identity to SEQ ID NO: 19. Optionally, it may be encoded by a nucleic acid sequence having at least 80%, preferably at least 85%, at least 90%, at least 95%, at least 96%, at least 97%, at least 98%, at least 99% or 100% sequence identity to SEQ ID NO: 18. The glycoprotein is thus capable of mediating entry into a host cell that can be entered by PIRYV-G having SEQ ID NO: 19. PIRYV-G is derived from the Piry virus, which, like VSV, MARAV and COCV, belongs to the genus vesiculovirus. Similar to COCV-G, PIRYV-G was shown to be more resistant to complement-mediated inactivation by mammalian sera than VSV-G (Tijani et al., 2018, Lentivector Producer Cell Lines with Stably Expressed Vesiculovirus Envelopes. Mol Ther Methods Clin Dev. 10, 303-312). However, the cellular surface receptor facilitating the entry of Piry virus into a host cell is currently unknown.

Optionally, the lentiviral vector may be pseudotyped with more than one type of heterologous glycoprotein. It may, e.g., be pseudotyped with two different glycoproteins targeting the same receptor protein, such as VSV-G and COCV-G. Alternatively, the lentiviral vector may be pseudotyped with a mixture of glycoproteins that bind to at least two, i.e. two, three or four different receptor proteins. It may, e.g., be simultaneously pseudotyped with COCV-G binding to the LDL receptor and RD114 binding to SLC1A5.

The composition of the invention is obtainable from packaging the lentiviral vector, preferably, in a process comprising providing at least four distinct expression plasmids. The lentiviral particles may be provided from a vector plasmid encoding the lentiviral vector genome itself as described above, a packaging plasmid coding for Gag and Pol, a plasmid encoding Rev and a plasmid encoding at least one of the herein mentioned envelope glycoproteins, e.g., as disclosed by Schambach et al., 2006 (Equal potency of gammaretroviral and lentiviral SIN vectors for expression of O6-methylguanine-DNA methyltransferase in hematopoietic cells. Mol. Ther. 13(2), 391-400). The vector plasmid may e.g. comprise a nucleic acid sequence of SEQ ID NO: 24 or having at least 80%, at least 85%, at least 90%, at least 95%, at least 96%, at least 97%, at least 98%, or at least 99% sequence identity thereto. Different promotor and/or enhancer and/or resistance elements may be used if desired. The packaging plasmid may e.g. comprise a nucleic acid sequence of SEQ ID NO: 25, or having at least 80%, at least 85%, at least 90%, at least 95%, at least 96%, at least 97%, at least 98%, or at least 99% sequence identity thereto. A different promotor may also be used. The Rev-encoding plasmid may e.g. comprise a nucleic acid sequence of SEQ ID NO: 26 or having at least 80%, at least 85%, at least 90%, at least 95%, at least 96%, at least 97%, at least 98%, or at least 99% sequence identity thereto. The Env-encoding plasmid may e.g. comprise a nucleic acid sequence of SEQ ID NO: 27 or having at least 80%, at least 85%, at least 90%, at least 95%, at least 96%, at least 97%, at least 98%, or at least 99% sequence identity thereto. A different promotor may also be used. Suitable promotors or other functional elements are e.g., taught herein.

Because the split genome design of lentiviral vectors effectively prevents the replication of lentiviral vectors, they need to be generated by co-expressing the different viral constituents in a single packaging cell. The at least four distinct expression plasmids are therefore co-transfected into a packaging cell to produce the lentiviral vector at sufficiently high titer. The packaging cell may be selected from the group comprising a HEK293T cell, a HEK293 cell, a NIH3T3 cell, a HeLa cell, an HT1080 cell, a COS-1 cell and an AGE1.CR cell. Preferably, the packaging cell is a human cell, most preferably a HEK293T or HEK293 cell. The ratio of the Gag/Pol encoding packaging construct, the Env plasmid, the Rev plasmid and the vector plasmid may be 10-20 : 1-10: 5-10: 5:10, preferably 13-16 : 1-7: 6-8 : 6-8. Especially the amount of Env-encoding plasmid may vary considerably depending on the selection of the glycoprotein for lentiviral vector pseudotyping. The medium comprising the produced lentiviral particles may be harvested 25-40 h, e.g. 30-36 h post-transfection of the packaging cells. Optionally, a second harvest of newly generated lentiviral particles may be performed 40-60 h, e.g. 48-54 h post-transfection. Preferably, the medium comprising the lentiviral particles is filtered using a 0.22 µm pore size filter. Media comprising virus particles obtained from subsequent harvests may be pooled and, optionally, stored, e.g., at -80 °C.

Co-expression of multiple plasmids in a single packaging cell such as a HEK293T cell according to the invention enables the production of lentiviral particles at high titers. Because the inner ear is a rather small and highly secluded organ that is difficult to access, only small volumes of the composition according to the invention can be applied to a subject (up to about 20 µL for humans and about 1 µL for mice). The efficiency of gene transfer and in turn of gene expression, however, largely depends on the total number of vector particles delivered per target cell (Zhang *et al,* 2001). The composition of the invention, therefore, needs to possess a virus titer high enough to deliver a sufficient amount of lentiviral particles to facilitate effective transduction of inner ear cells. The viral titer of a composition is most accurately provided as functional titer, i.e. it describes how many viral particles have actually infected a target cell. It is, therefore, also designated an infectious titer. It is commonly expressed as transduction units per mL (TU/mL) and can be assessed after transduction of a cell, e.g., by PCR-based methods or flow cytometry. The final virus titer of the present composition should be of a magnitude of at least 10⁶, preferably of at least 10⁷, most preferably of at least 10⁸ TU/mL. It may even be higher, e.g., at least 10⁹ TU/mL or even at least 10¹⁰ TU/mL.

To reach a functional titer of the above mentioned magnitude, the lentiviral vector titer of the composition according to the invention may be further concentrated after harvesting of the lentiviral particles from packaging cells, e.g., using ultracentifugation. In a preferred embodiment, the lentiviral vector titer is concentrated using ultracentrifugation, wherein the viral vector particles are centrifuged at at least 10,000 x g for at least 1 h, at least 1.5 h or, preferably, for at least 2 h, at least 2.5 h, at least 3 h, at least 3.5 h or at least 4 h (Zhang *et al.,* 2001,). The speed and duration of centrifugation depend on the respective pseudotype of the viral vector particle. VSV-G-pseudotyped lentiviral vector particles may, for instance, be centrifuged at 82,740 x g for 2 h. In contrast, vector particles pseudotyped with RD114, GALV or BaEV may be centrifuged at about 13,238 x g, but for longer time periods, e.g., at least 4 h, preferably overnight. Using ultracentrifugation, it is possible to concentrate the virus titer of the composition of the invention by at least 10 fold, by at least 20 fold, by at least 30 fold, by at least 40 fold, by at least 50 fold, by at least 60 fold, by at least 70 fold, by at least 80 fold, by at least 90 fold, by at least 100 fold, by at least 150 fold, by at least 200 fold, by at least 250 fold, or even by at least 300 fold. However, even though ultracentrifugation has proven highly effective for harvesting lentiviral vectors, the method is associated with certain draw-backs: The rotors that may be used at such speed usually have only a small volume capacity and the attainment of large volumes of high-titer viral vectors thus is very time-consuming (Zhang *et al.,* 2001). Ultracentrifugation further requires the virus particles to exhibit sufficient stability, e.g., due to pseudotyping of the viral envelope. Therefore, in another embodiment, the virus titer may also be concentrated by any other suitable purification method known from the state of the art, such as microfiltration, ultrafiltration, chromatography, density gradient ultracentrifugation, tangential flow microfiltration, or precipitation.

After concentrating the lentiviral particles of the invention by performing ultracentrifugation or any other suitable method from the state of the art, the viral particles have to be reconstituted in a suitable medium. The medium should have a physiological salt concentration and, preferably, a physiological pH. The inventors unexpectedly found that a defined mixture of phosphate-buffered saline (PBS) and 4-(2-hydroxyethyl)-1-piperazineethanesulfonic acid (HEPES) constituted a particularly beneficial medium for reconstituting and stabilizing virus particles. Therefore, the viral particles are preferably re-suspended in a medium comprising PBS (pH 7.2-7.4) and 1-50 mM, e.g., 1-10 mM, 20-30 mM, 30-40 mM, 40-50 mM, or, preferably, 10-20 mM HEPES. The present inventors found the addition of HEPES to be advantageous for maintaining the stability of the lentiviral particles of the invention, even after repeated Freeze-Thaw circles. Preferably, the medium used for reconstituting the concentrated viral particles does not contain any serum, hormones or cytokines to avoid any external biological stimulus on inner ear cells. Optionally, the medium may also be serum or blood. It may also be a lymphatic liquid of the inner ear, e.g., perilymph or endolymph.

The composition of the invention may be stored for up 1-4 weeks, e.g., 1 week, 2 weeks, 3 weeks or 4 weeks at temperatures around 4°C. Preferably, the composition is stored in a frozen state, most preferably at about -80°C. When frozen, the composition may be stored for at least 6, at least 12, at least 24, or at least 36 months prior to being administered to the subject.

Preferably, the cell of the inner ear that is to be transduced by the lentiviral vector according to the invention is a cell selected from the group consisting of cells of the organ of Corti, including inner and outer hair cells, spiral ganglion neurons and glial cells, cells of the stria vascularis and spiral ligament and lateral wall fibrocytes. Preferably, the cell is a hair cell or a spiral ganglion neuron. Hair cells constitute the sensory cells required for hearing and are located within the organ of Corti. They are arranged into one row of inner hair cells followed by three rows of outer hair cells. Hair cells possess apical modifications, so-called stereocilia, which are in contact with the fluid filling the scala media, the endolymph, and interdigitate with a variety of supporting cells. Unlike, e.g., birds or reptiles, mammals are incapable of naturally regenerating damaged or lost hair cells. Accordingly, hair cell degeneration may lead to permanent hearing loss if left untreated.

Spiral ganglion neurons (SGNs) are located within the modiolus, i.e., the central axis of the cochlea. Their dendrites form synapses with the base of hair cells and their axons run into the eighth cranial nerve, also known as the vestibulocochlear nerve. SGNs are the first neurons in the auditory systems that fire action potentials upon perception of sound. Glial cells are non-neuronal cells that do not produce electrical impulses and act as supporting cells of the nervous system. Glial cells have the potential to become reprogrammed into primary auditory neurons (PANs) through artificial expression of certain transcription factors (Meas *et al.,* 2018, Reprogramming Glia Into Neurons in the Peripheral Auditory System as a Solution for Sensorineural Hearing Loss: Lessons From the Central Nervous System. Front Mol Neurosci. 11: 77). Glial cells thus represent an attractive target for lentiviral gene transfer to compensate for the loss of neurons in the peripheral auditory system. The stria vascularis consists of epithelial cells and forms the outer wall of the scala media. It furthermore produces the endolymph of the scala media. The spiral ligament secures the membranous scalar media to the spiral canal of the cochlea.

The inner ear cell transduced according to the invention may alternatively be type I and II vestibular hair cells, vestibular supporting cells and vestibular ganglion neurons.The vestibular system is the sensory system of the inner ear that facilitates the sense of balance and spatial orientation for coordinated movement. Accordingly, the composition according to the invention may also be suitable to alleviate symptoms associated with vestibular disturbances.

In another embodiment, the cell of the inner ear may also be a supporting cell selected from the group comprising a Boettcher cell, a Claudius cell, a Deiters' cell, a Hensen's cell or a pillar cell. Boettcher cells are located on the basilar membrane in the lower turn of the cochlea where they project microvilli into the intercellular space and may perform both secretory and absorptive functions. Claudius cells are located immediately above the Boettcher cells and are in direct contact with the endolymph. The formation of tight junctions between adjacent Claudius cells prevents the leakage of endolymph out of the scala media. Deiters' cells are arranged in up to 5 rows and sit directly on the basilar membrane of the cochlea. They form long, apical cell extensions that extend to the reticular lamina of the inner ear. The tall Hensen's cells are located adjacent to the outer row of Deiters' cells within the organ of Corti where they may mediate ion metabolism. Hensen's cells are a particularly interesting target for gene therapy as they are believed to have retained a capacity to regenerate hair cells (Malgrange et al., 2002, Epithelial supporting cells can differentiate into outer hair cells and Deiters' cells in the cultured organ of Corti. Cellular and Molecular Life Sciences. 59, 1744-1757). For example, a cargo nucleic acid may stimulate the regeneration of hair cells by Hensen's cells. Pillar cells can be divided in outer and inner pillar cells. Both types form numerous cross-linked microtubules and actin filaments. Pillar cells obtained their name because the outer cells are free standing and form contacts to adjacent cells only at their bases and apices.

The composition of the invention may be administered to the subject by any method suitable for delivering a substance to cells of the inner ear. The method for delivering the composition to a subject may, for instance, be a method selected from the group comprising a cochlea implant route, round window injection, oval window injection, canalostomy, cochleostomy as well as injection into the endolymphatic sac. For round window injection, a long needle is inserted through the outer ear and punctures the eardrum to inject the composition of the invention through the round window located in the medial wall of the middle ear into the scala tympani. Oval window injection involves injection of the composition through the oval window into the scala vestibuli. Alternatively, the composition may be injected into the endolymphatic sac, a small pouch connected via the endolymphatic duct to the endolymph-containing scala media and, thus, the compartment harboring the organ of Corti. However, injection into the endolymphatic sac requires a mastoidectomy that is associated with a large decompression of the posterior fossa plate. Thus, there is a risk associated with injection into the endolymphatic sac (Blanc *et al.,* 2020). Cochleostomy is a method where the viral vector is injected directly into the cochlea. As previously described, direct injection into the cochlea harbors the risk of mechanically damaging sensory hair cells, leading to a decline in hearing function. During canalostomy, a hole is drilled into one of the semicircular canals of the vestibular organ and the composition is injected directly into the vestibular labyrinth. Mechanical damage to the cells of the inner ear, e.g., sensory hair cells, should be avoided in the context of the invention, especially in treatment of a human patient. Thus, the amount of the composition injected, as well as the pressure associated with the injection, should be minimized. Thus, a high titer and a high efficiency, which can be reached according to the invention, are of particular importance for treatment of humans.

The inventors could demonstrate that delivery of the composition into the mouse inner ear via canalostomy resulted in effective transduction of sensory hair cells and SGN. Therefore, if the subject according to the invention is a mouse or another rodent such as a guinea pig or a rat, the composition of the invention is preferably administered via canalostomy, in particular by injecting it into the posterior semicircular canal. Because a canalostomy does not disturb the middle ear during surgery and prevents injury of hair cells, it may also be suitable for vector delivery into the human inner ear (Blanc *et al.,* 2020). Nevertheless, the method is associated with the risk of opening the membranous labyrinth and of a post-operative obstruction of the canal by fibrotic tissue. Improvements in the surgical techniques used, e.g., via the help of robotic assistance, may greatly reduce some of the risks associated with this method (Blanc *et al.,* 2020). The surgical procedure conducted for canalostomy resembles that of a cochlear implant surgery (Blanc *et al.,* 2020).

A cochlea implant is a device that is surgically introduced into the inner ear. Conventional cochlear implants consist of an external and an internal part. The external part is comprised of a microphone, a sound processor and a battery. The internal, transplanted part directs the signals received by the external part via an array of electrodes into the cochlea to directly stimulate the SGNs, thus circumventing the need for sensory hair cells to perceive sound. The implant may further be equipped with a reservoir chamber comprised of the composition of the invention as well as drug delivery channels that transport the composition from the reservoir chamber to the tips of the electrodes for targeted delivery into the cochlea. The implant may optionally be designed to allow for sustained release of the composition. Several types of cochlea implants and other auditory prostheses capable of delivering drugs to the inner ear have been developed and may be suitable for administering the composition of the invention to a subject (Hendricks *et al.,* 2008, Localized Cell and Drug Delivery for Auditory Prostheses. Hear Res. 242(1-2), 117-131). Preferably, when the subject is a human, the composition is administered using a cochlear implant. In a further embodiment, the cochlear implant may also deliver a growth factor, e.g., a neurotrophic factor such as BDNF, GDNF, NT3 or IGF that may support, e.g., transdifferentiation of cells into sensory hair cells or SGNs.

The composition according to the invention may also be administered to the subject by other suitable methods, including known pump-catheter systems or round window membrane diffusion, the latter comprising the use of, e.g., a collagenase for partial digestion of the round window membrane. It is also possible that the composition of the invention is for use in administration to the subject via the systemic route. Intravenous injection of the composition under local anesthesia would constitute an atraumatic and easy alternative to the other methods discussed herein. Administration via the systemic route has already been successfully applied for delivery of AAVs into the murine inner ear (Shibata et al., 2017, Intravenous rAAV2/9 injection for murine cochlear gene delivery. Sci. Rep. 7, 9609). However, systemic delivery of the composition according to the invention would harbor the risk of off-target transduction and requires very high composition volumes and virus titers.

The inventors have shown that a high transduction efficiency or transduction rate can be obtained using the composition of the invention. Thus, preferably, the composition of the invention has a viral transduction efficiency in spiral ganglion neurons and hair cells of at least 10%, preferably, at least 30%. E.g., the transduction efficiency may be at least 35%, at least 40%, at least 50%, at least 55%, at least 60%, at least 65%.

The subject to be treated can be a human or non-human animal, preferably a vertebrate, e.g. a mammal such as a human, a primate, a dog, a cat, a rat, a guinea pig or a mouse. Preferably, the subject is a human being, e.g., a human having sensorineural hearing loss or in danger of developing sensorineural hearing loss.

In particular, the invention provides the following embodiments:
1. A composition for use in treating or preventing sensorineural hearing loss in a subject, wherein the composition has a titer of at least 10⁷ TU/mL and comprises a 3^{rd} generation lentiviral vector pseudotyped with a viral envelope glycoprotein capable of binding to a receptor expressed in a cell of the inner ear, wherein said composition is administered to the inner ear of the subject.
2. The composition for use of embodiment 1, wherein the lentiviral vector is pseudotyped with a viral envelope glycoprotein capable of binding to a receptor selected from the group consisting of the LDL-receptor and LDL-R family members, the SLC1A5-receptor, the Pit1/2-receptor and the PIRYV-G-receptor.
3. The composition for use of any of embodiments 1-2 wherein the lentiviral vector is pseudotyped with a viral envelope glycoprotein capable of binding the LDL-receptor or LDL-R family members, optionally, the LDL-receptor.
4. The composition for use of any of embodiments 1 or 3, wherein the viral envelope glycoprotein is selected from the group comprising MARAV-G, COCV-G, VSV-G and VSV-G ts.
5. The composition for use of embodiment 4, wherein the viral envelope glycoprotein is MARAV-G.
6. The composition for use of embodiment 4, wherein the viral envelope glycoprotein is COCV-G.
7. The composition for use of embodiment 6, wherein the viral envelope glycoprotein is VSV-G.
8. The composition for use of embodiment 4, wherein the viral envelope glycoprotein is VSV-G ts.
9. The composition for use of any of embodiments 1-2, wherein the lentiviral vector is pseudotyped with a viral envelope glycoprotein capable of binding the SLC1A5-receptor.
10. The composition for use of any of embodiments 1 or 9, wherein the viral envelope glycoprotein is selected from the group consisting of RD114 GP and derivatives thereof and BaEV GP and derivatives thereof.
11. The composition for use of embodiment 10, wherein the viral envelope glycoprotein is RD114 GP.
12. The composition for use of embodiment 10, wherein the viral envelope glycoprotein is derived from RD114 GP.
13. The composition for use of embodiment 10, wherein the viral envelope glycoprotein is BaEV GP.
14. The composition for use of embodiment 10, wherein the viral envelope glycoprotein is derived from BaEV GP.
15. The composition for use of any of embodiments 1-2, wherein the lentiviral vector is pseudotyped with a viral envelope glycoprotein capable of binding the Pit1/2-receptor.
16. The composition for use of any of embodiments 1 or 15, wherein the viral envelope glycoprotein is derived from a virus selected from the group consisting of GALV and 10A1 MLV.
17. The composition for use of embodiment 16, wherein the viral envelope glycoprotein is derived from GALV.
18. The composition for use of embodiment 16, wherein the viral envelope glycoprotein is derived from 10A1 MLV.
19. The composition for use of any of embodiments 1-2, wherein the lentiviral vector is pseudotyped with PIRYV-G capable of binding a PIRYV-G-receptor.
20. The composition for use of any of embodiments 1-19, wherein the lentiviral vector comprises a cargo sequence comprising a nucleic acid selected from the group comprising a protein-coding gene, a miRNA, an shRNA, a IncRNA and an sgRNA,
   wherein expression of said cargo sequence in said cell of the inner ear is capable of reducing, eliminating or preventing at least a symptom of sensorineural hearing loss in the subject.
21. The composition for use of embodiment 20, wherein the cargo sequence is a protein-coding gene, wherein a reduced or erroneous expression of said gene is associated with the development of the sensorineural hearing loss in the subject.
22. The composition for use of any of embodiments 20 or 21, wherein the cargo sequence is a protein-coding gene encoding a growth factor selected from the group comprising a neurotrophic factor such as BDNF, GDNF, NT3, IGF.
23. The composition for use of any of embodiments 20 to 22, wherein the cargo sequence is a protein-coding gene, wherein expression of said gene may compensate for a non-genetic cause of sensorineural hearing loss in the subject, optionally, by protecting inner ear cells from a hazardous agent or by increasing survival of inner ear cells that have been damaged by a hazardous agent, wherein the hazardous agent is selected from the group comprising noise and ototoxic drugs.
24. The composition for use of embodiment 20, wherein the cargo sequence is a miRNA, wherein a reduced or erroneous expression of said miRNA is associated with the development of the sensorineural hearing loss in the subject, or wherein said miRNA targets a gene whose elevated or erroneous expression is associated with the development of the sensorineural hearing loss in the subject.
25. The composition for use of embodiment 20, wherein the cargo sequence is an shRNA, wherein said shRNA targets a gene whose elevated or erroneous expression is associated with the development of the sensorineural hearing loss in the subject.
26. The composition for use of embodiment 20, wherein the cargo sequence is a IncRNA, wherein a reduced or erroneous expression of said IncRNA is associated with the development of the sensorineural hearing loss in the subject, or wherein said IncRNA regulates a gene whose elevated or erroneous expression is associated with the development of the sensorineural hearing loss in the subject.
27. The composition for use of embodiment 20, wherein the cargo sequence encodes a Cas9 protein and further comprises a single guide RNA for CRISPR/Cas9 delivery to a cell of the inner ear.
28. The composition for use of any of embodiments 1-27, wherein the hearing loss is caused by a mutation in a gene selected from the group comprising *MYO7A, GJB2, OTOF, ATOH1, SLC26A4, TMC1, TMPRSS3, GIPC3, USH1C, RDX, CLDN14, WHRN, ESPN, ILDR1, SERPINB6, KCNQ4, POU4F3, SIX1, CCDC50, MIR96, POU3F4, EYA1, KCNQ1, GJB6, STRC, TECTA, GJB3, PRPS1, CDH23, GRXCR2, CABP2, GRXCR1, SYNE4, COL11A2, LRTOMT, BSND, PJVK, CIB2, CEACAM16, TMIE, NARS2, LHFPL5, MARVELD2, SLC22A4, KARS, SLC26A5, TPRN, ELMOD3, GPSM2, COCH, ESRRB, ADCY1, EPS8L2, CD164, OTOA, S1PR2, LOXHD1, TSPEAR, EPS8, CDC14A, MSRB3, DCDC2, PNPT1, TMEM132E, FAM65B, MYO3A, CLIC5, HGF, USH2A, TBC1D24, MET, PCDH15, PTPRQ, OTOGL, MYO6, TNC, TRIOBP, BDP1, MYO15A, DIAPH1, MYH14, DFNA5, WFS1, EYA4, COL11A2, MYH9, ACTG1, SLC17A8, GRHL2, DSPP, P2RX2, MYO1A, GFI1, TYMP* and *GFI1B.*
29. The composition for use of any of embodiments 1-21, wherein the cargo sequence is a gene selected from the group comprising *MYO7A, GJB2, OTOF ATOH1, SLC26A4, TMC1, TMPRSS3, GIPC3, USH1C, RDX, CLDN14, WHRN, ESPN, ILDR1, SERPINB6, KCNQ4, POU4F3, SIX1, CCDC50, MIR96, POU3F4, EYA1, KCNQ1, GJB6, STRC, TECTA, GJB3, PRPS1, CDH23, GRXCR2, CABP2, GRXCR1, SYNE4, COL11A2, LRTOMT, BSND, PJVK, CIB2, CEACAM16, TMIE, NARS2, LHFPL5, MARVELD2, SLC22A4, KARS, SLC26A5, TPRN, ELMOD3, GPSM2, COCH, ESRRB, ADCY1, EPS8L2, CD164, OTOA, S1PR2, LOXHD1, TSPEAR, EPS8, CDC14A, MSRB3, DCDC2, PNPT1, TMEM132E, FAM65B, MYO3A, CLIC5, HGF, USH2A, TBC1D24, MET, PCDH15, PTPRQ, OTOGL, MYO6, TNC, TRIOBP, BDP1, MYO15A, DIAPH1, MYH14, DFNA5, WFS1, EYA4, COL11A2, MYH9, ACTG1, SLC17A8, GRHL2, DSPP, P2RX2, MYO1A, GFI1, TYMP, GFI1B, BDNF, GDNF, NT-3, IGF1, IGF2, CNTF, ARTN, NRTN, ATP7B, MRP1, MRP2* and *MDR1.*
30. The composition for use of any of embodiments 1-29, wherein the lentiviral vector is derived from a virus selected from the group comprising HIV-1, HIV-2, SIV, FIV or EIAV.
31. The composition for use of embodiment 30, wherein the lentiviral vector is derived from HIV-1.
32. The composition for use of embodiment 30, wherein the lentiviral vector is derived from HIV-2.
33. The composition for use of embodiment 30, wherein the lentiviral vector is derived from SIV.
34. The composition for use of embodiment 30, wherein the lentiviral vector is derived from FIV.
35. The composition for use of embodiment 30, wherein the lentiviral vector is derived from EIAV.
36. The composition for use of any of embodiments 1-35, wherein the composition is obtainable from packaging the lentiviral vector in a process comprising providing at least four distinct expression plasmids.
37. The composition for use of embodiment 36, obtainable from a method wherein the at least four distinct expression plasmids are co-transfected into a packaging cell to produce the lentiviral vector particles, wherein the packaging cell is selected from the group comprising a HEK293T cell, a HEK293 cell, an NIH3T3 cell, a HeLa cell, an HT1080 cell, a COS-1 cell and an AGE1.CR cell to produce the lentiviral vector, preferably, a human cell line.
38. The composition for use of any of embodiments 36 or 37, wherein the ratio of the Gag/Pol packaging construct, the Env plasmid, the Rev plasmid and the vector plasmid is 7-16:1-7:3-8:4-10.
39. The composition for use of any of embodiments 1-38, wherein the lentiviral vector is replication incompetent due to the split packaging design and self-inactivating due to a deletion in the U3 region of the 3' LTR.
40. The composition for use of any of embodiments 1-39, wherein the lentiviral vector lacks the genes vif, vpr, vpu, nef, and, optionally, tat.
41. The composition for use of any of embodiments 1-40, wherein the lentiviral vector comprises
   a) a 5' LTR comprising a constitutively active heterologous promoter at the U3 position, a repeat region (R) and a U5 region,
   b) a 5' UTR comprising a primer binding site (PBS), a splice donor site (SD), a packaging signal (ψ), a Rev-responsive element and, optionally, a splice acceptor (SA) site,
   c) an internal enhancer/promoter region operably linked to a cargo sequence,
   d) RNA processing elements optionally comprising a Woodchuck hepatitis virus post-transcriptional regulatory element (PRE), and
   e) a 3' LTR with a deleted (SIN) U3 region, a repeat region (R) and a U5 region.
42. The composition for use of embodiment 41, wherein the heterologous promoter at the U3 position of the 5' LTR is selected from the group comprising an RSV promoter, a CMV promoter, an HIV promoter, a eukaryotic promoter and a Tet-regulated promoter.
43. The composition for use of embodiment 41, wherein the internal enhancer/promoter region is selected from the group comprising a viral promoter, a cellular promoter, a cell-specific promoter, an inducible promoter, a hybrid promoter, and a synthetic promoter.
44. The composition for use of any of embodiments 41 or 43, wherein the internal enhancer/promoter region is a viral promoter selected from the group comprising SFFV, MPSV, MND, MESV, CMV, MLV and SV40.
45. The composition for use of any of embodiments 41 or 43, wherein the internal enhancer/promoter region is a cellular promoter selected from the group comprising EF1a, PGK1 and UBC.
46. The composition for use of any of embodiments 41 or 43, wherein the internal enhancer/promoter region is a hair cell-specific promoter and/or enhancer selected from the group comprising POU4F3, POU3F4, ATOH1, Prestin, Pendrin and MYO7A.
47. The composition for use of any of embodiments 41 or 43, wherein the internal enhancer/promoter region is an inducible promoter selected from the group comprising a TET-inducible promoter, a Cumate-inducible promoter, an electrosensitive promoter and an optogenetic switch.
48. The composition for use of any of embodiments 41 or 43, wherein the internal enhancer/promoter region is the hybrid promoter CAG.
49. The composition for use of any of embodiments 41 or 43, wherein the internal enhancer/promoter region is a synthetic promoter.
50. The composition for use of embodiment 41, wherein the internal enhancer/promoter region comprises a chromatin opening element.
51. The composition for use of any of embodiments 1-50, wherein the lentiviral vector is a non-integrating lentiviral vector.
52. The composition for use of any of embodiments 1-51, wherein the cell of the inner ear is a cell selected from the group consisting of cells of the organ of Corti, including supporting cells as well as inner and outer hair cells, spiral ganglion neurons and glial cells, cells of the stria vascularis and spiral ligament, lateral wall fibrocytes, type I and II vestibular hair cells, vestibular supporting cells and vestibular ganglion neurons.
53. The composition for use of embodiment 52, wherein the cell of the inner ear is a hair cell.
54. The composition for use of embodiment 52, wherein the cell of the inner ear is a spiral ganglion neuron.
55. The composition for use of embodiment 52, wherein the cell of the inner ear is a glial cell.
56. The composition for use of embodiment 52, wherein the cell of the inner ear is a cell of the stria vascularis.
57. The composition for use of embodiment 52, wherein the cell of the inner ear is a cell of the spiral ligament.
58. The composition for use of embodiment 52, wherein the cell of the inner ear is a lateral wall fibrocyte.
59. The composition for use of embodiment 52, wherein the cell of the inner ear is a type I or II vestibular hair cell.
60. The composition for use of embodiment 52, wherein the cell of the inner ear is a vestibular supporting cell.
61. The composition for use of embodiment 52, wherein the cell of the inner ear is a vestibular ganglion neuron.
62. The composition for use of any of embodiments 1-51, wherein the cell of the inner ear is a supporting cell selected from the group comprising a Boettcher cell, a Claudius cell, a Deiters' cell, a Hensen's cell or a pillar cell.
63. The composition for use of embodiment 62 wherein the cell of the inner ear is a Boettcher cell.
64. The composition for use of embodiment 62, wherein the cell of the inner ear is a Claudius cell.
65. The composition for use of embodiment 62, wherein the cell of the inner ear is a Deiters' cell.
66. The composition for use of embodiment 62, wherein the cell of the inner ear is a Hensen's cell.
67. The composition for use of embodiment 62, wherein the cell of the inner ear is a pillar cell.
68. The composition for use of any of embodiments 1-67, wherein the lentiviral vector titer is concentrated using ultracentifugation or other purification methods, incl. microfiltration, ultrafiltration, chromatography, density gradient ultracentrifugation, tangential flow microfiltration, or precipitation.
69. The composition for use of any of embodiments 1-68 that comprises a buffer having a physiological salt concentration and pH selected from the group comprising PBS with 10-20 mM HEPES.
70. The composition for use of any of embodiments 1-69, wherein the pharmaceutical composition is administered to the subject by a method selected from the group comprising a cochlea implant route, round window injection, oval window injection, canalostomy, cochleostomy as well as injection into the endolymphatic sac.
71. The composition for use of any of embodiments 1-70, wherein the composition is delivered to cells of the inner ear by injection into one of the semicircular canals of the vestibular labyrinth (canalostomy), wherein, preferably, the subject is a mouse.
72. The composition for use of any of embodiments 1-71, wherein the viral transduction efficiency in spiral ganglion neurons is at least 10%, optionally, at least 30%.
73. The composition for use of any of embodiments 1-72, wherein the subject is a human, a primate, a dog, a cat, a rat, a guinea pig or a mouse.
74. The composition for use of any of embodiments 1-73, wherein the subject is a human.
75. The composition for use of any of embodiments 1-74 which is for treating sensorineural hearing loss in a subject.
76. The composition for use of any of embodiments 1-74 which is for preventing sensorineural hearing loss in a subject.
77. A method for treating sensorineural hearing loss in a subject in need thereof, comprising the step of administering an effective amount of the composition of any of embodiments 1-76 to said subject.
78. A method for preventing sensorineural hearing loss in a subject in need thereof, comprising the step of administering an effective amount of the composition of any of embodiments 1-76 to said subject.

In summary, the invention teaches a composition comprising a third generation lentiviral vector for use in treating sensorineural hearing loss in a subject. The inventors were able, for the first time, to transduce sensory hair cells and SGNs in the inner ear of mice with efficiencies sufficiently high enough to result in correction of inner ear function or protection from progressive loss of inner ear function by combining an up-to-date lentiviral vector generation with an optimized production protocol and the reconstitution of viral vector in a defined-particle stabilizing medium. In doing so, the present invention demonstrates that lentiviral vectors have the potential to be successfully applied in gene therapy to treat sensorineural hearing loss in human patients.

Throughout the invention, the term "about" is intended to be understood as "+/- 10%". If "about" relates to a range, it refers to both lower and upper limits of the range. "A" is intended to mean "one or more", if not explicitly mentioned otherwise.

All literature cited herein is herewith fully incorporated. The present invention is further illustrated, but not limited, by the following examples.

### Brief description of the Drawings

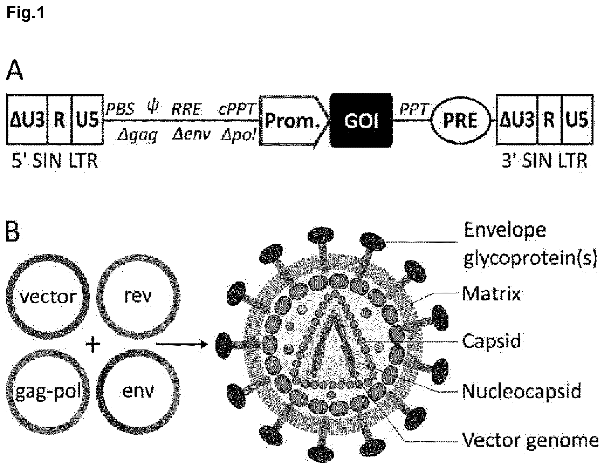

- **Fig. 1**: Third-generation lentiviral vector system with split-packaging design and self-inactivating vector architecture (A) Schematic depiction of the lentiviral vector architecture. SIN: self-inactivating; LTR: long terminal repeat, ΔU3: unique 3' region with self-inactivating deletion; R: repeat region; U5: unique 5' region; PBS: primer binding site; RRE: Rev-response element; cPPT: central polypurine tract; Prom.: promoter; GOI: gene of interest; PPT: polypurine tract; PRE: post-transcriptional regulatory element. (B) Four expression plasmids to provide the genomic vector information and all required structural and enzymatic (Gag-Pol, Env and Rev) components are transiently co-transfected into packaging cells, e.g., 293T cells, to produce vector particles.

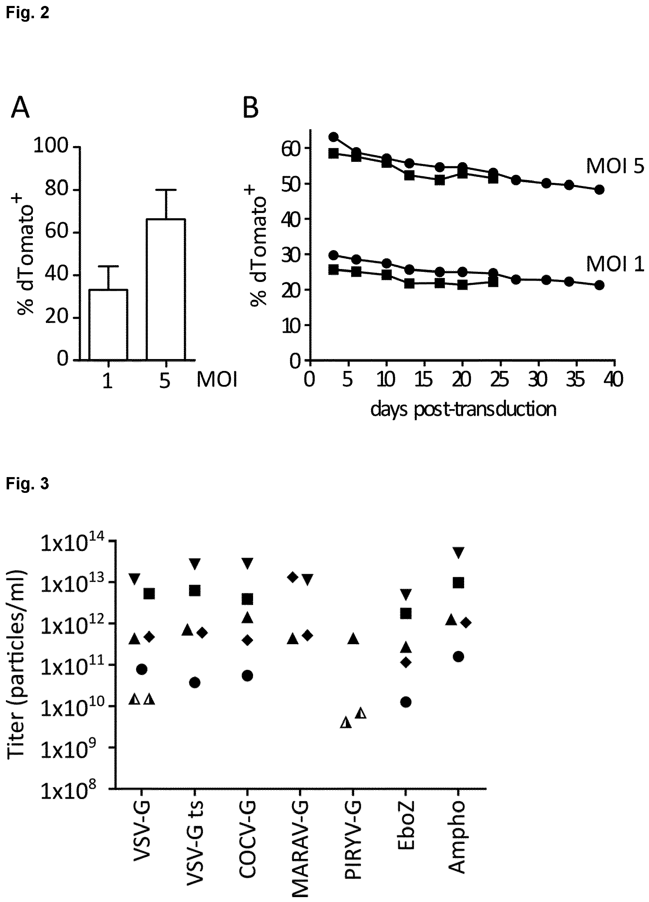

- **Fig. 2**: Efficient transduction of the HEI-OC1 cell line with lentiviral vectors pseudotyped with VSV-G. HEI-OC1 (a cochlear HC-like conditionally transformed cell line) were transduced with lentiviral vector preparations encoding for a dTomato reporter protein and analyzed by flow cytometry. (A) Two different multiplicities of infection (MOI) were tested. Analysis was performed on day 6 post-transduction, n=3 independent vector batches. Mean + SD. (B) Expression kinetics analysis. A great proportion of the successfully transduced, reporter-positive cells was stably maintained until the final time point of analysis (39 days). n=2 independent vector batches (dots and squares indicate separate batches).
- **Fig. 3**: Selected envelope glycoproteins efficiently pseudotype lentiviral vectors. Multiple batches were produced with each pseudotype to package a dTomato-reporter-encoding lentiviral vector. Titers were assessed as the number of particles present per milliliter of supernatant by RNA extraction followed by qRT-PCR. Symbols indicate different batches.
- **Fig. 4**: Lentiviral vectors encoding a dTomato reporter protein and pseudotyped with different envelope proteins transduce the auditory cell line HEI-OC1 with different efficiency. Analysis was carried out by flow cytometry to determine (A) the percentage of dTomato-positive cells as well as the mean fluorescence intensity (MFI) of dTomato within the population of dTomato-positive cells. (B) Calculation of the relative expression of dTomato combines the two relevant parameters transduction efficiency (percentage of dTomato-positive cells) and expression level (MFI) as a correlate of the number of integrated vector copies per cell.

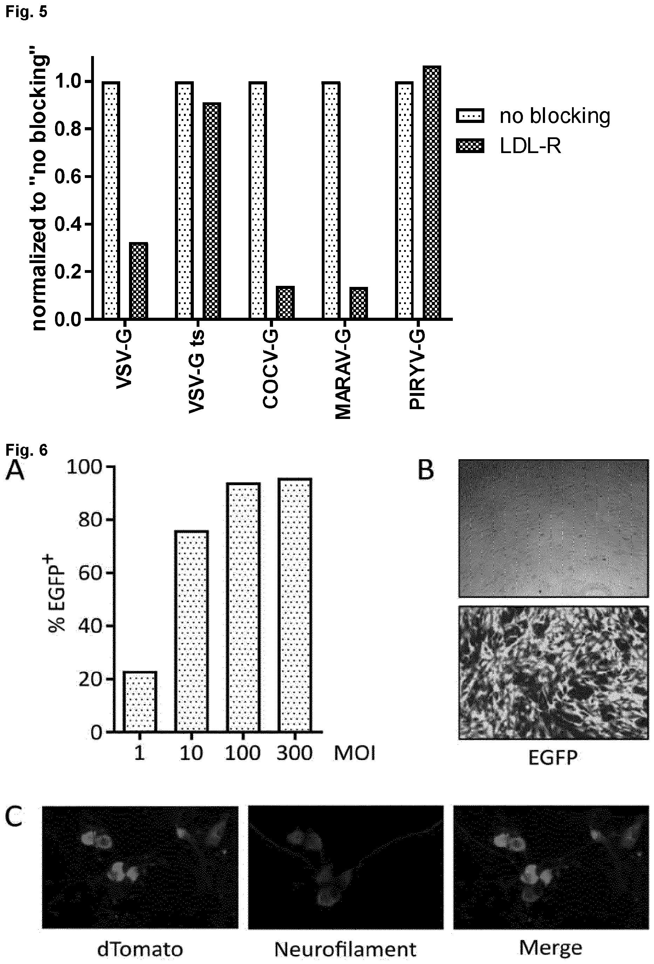

- **Fig. 5**: The pseudotypes VSV-G, VSV-G ts, COCV-G and MARAV-G use the LDL-receptor (LDL-R) for cell entry while PIRYV-G-mediated cell entry does not involve LDL-R. Soluble LDL-R protein (5µg/mL) was added to HEI-OC1 cells prior to transduction with a lentiviral vector encoding a dTomato reporter protein, packaged with different pseudotypes. Cell entry of the vector particles in the presence or absence (no blocking) of soluble LDL-R was monitored by flow-cytometry two days post-transduction, to determine the percentage of dTomato-positive cells. Values were normalized to the respective "no blocking" control.
- **Fig. 6**: Efficient transduction of dissociated primary SGN with lentiviral vector preparations pseudotyped with VSV-G. (A) Rat SGN were extracted, dissociated, seeded in *in-vitro* cultures and transduced at different multiplicities of infection (MOI) with a lentiviral vector carrying an EGFP marker gene. Flow cytometry revealed efficient transduction, with a low MOI of 1 already yielding more than 20% of EGFP-positive cells. (B) SGN culture transduced with a lentiviral vector expressing an EGFP reporter. Top: brightfield; bottom: EGFP channel. (C) Immunocytochemistry of SGN transduced with a lentiviral vector expressing a dTomato reporter gene. Neurofilament: marker for SGN.
- **Fig. 7**: Lentiviral vectors encoding a dTomato reporter protein and pseudotyped with different envelope proteins transduce primary dissociated SGN cultures with different efficiency. Analysis was carried out by flow cytometry to determine (A) the percentage of dTomato-positive cells as well as the mean fluorescence intensity (MFI) of dTomato within the population of dTomato-positive cells and to calculate (B) the relative expression of dTomato, combining the two relevant parameters transduction efficiency (percentage of dTomato-positive cells) and expression level (MFI) as a correlate of the number of integrated vector copies per cell.

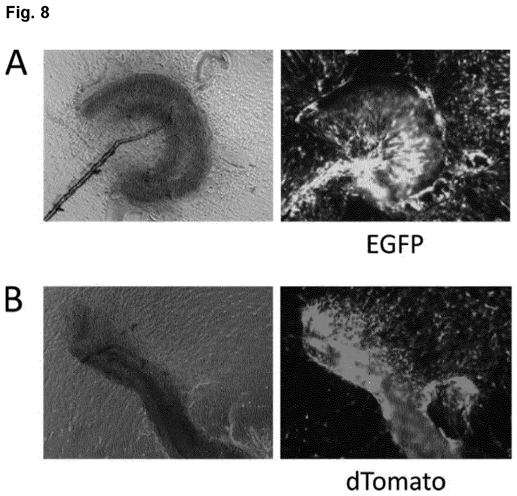

- **Fig. 8**: Efficient transduction of primary organ of Corti explants with VSV-G pseudotyped lentiviral vector preparations. Rat organs of Corti were explanted and cultured *in vitro.* Transduction of these cultures with (A) an EGFP-expressing or (B) a dTomato-expressing lentiviral vector yielded a high transduction efficiency throughout the explant.
- **Fig. 9**: Inner ear architecture and canalostomy. (A) The inner ear contains the labyrinth, including three semicircular canals, and the cochlea. Viral particles can be administered via canalostomy through a hole drilled into the posterior semicircular canal. (B) Cochlea cut in cross section. The organ of Corti (black box) sits on the basilar membrane, a membrane which separates the scala tympani from the scala media. (C) The organ of Corti contains inner and outer hair cells. Spiral ganglion neurons (SGN) project the generated electrical signal to the brain.

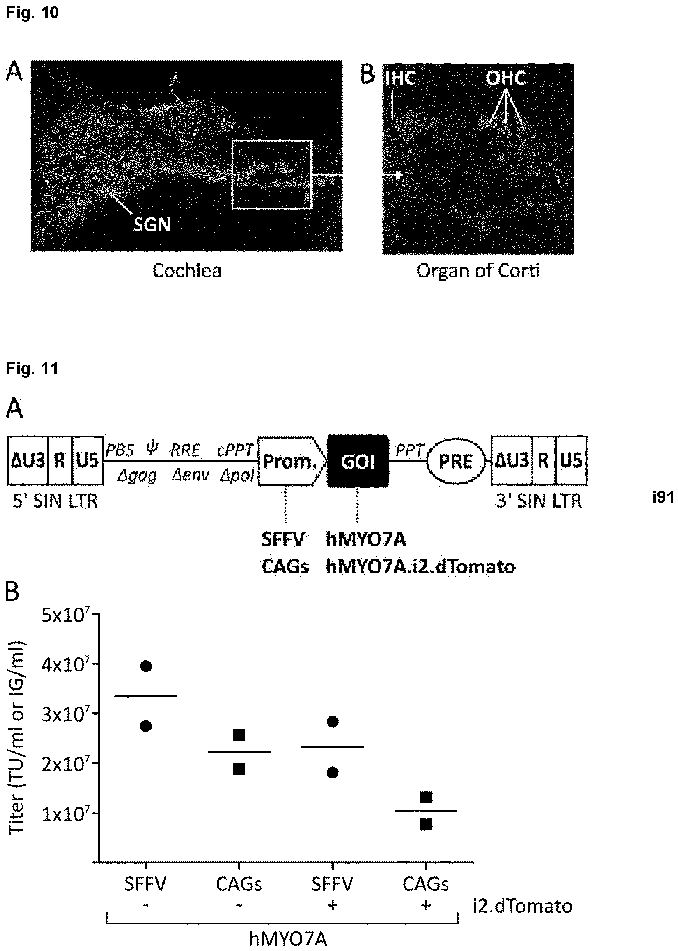

- **Fig. 10**: Efficient transduction of SGN and HC upon *in-vivo* lentiviral vector injection. A lentiviral vector encoding for a dTomato reporter protein was administered *in vivo* to adult mice through canalostomy. Animals were sacrificed on day 30. Epithelium, spiral ganglion neurons (SGN) and hair cells (HC) were highly positive for the marker protein. Red signal: dTomato; blue signal: DAPI. (A) Exemplary section of the cochlea. (B) Higher magnification of the section highlighted in A by a white box (the organ of Corti), to better identify inner (IHC) and outer (OHC) hair cells.
- **Fig. 11**: Lentiviral particles delivering a human Myosin VIIa transgene can be successfully produced. (A) Schematic depiction of the vector to treat hearing loss due to Myosin VIIa deficiency. Expression of the human Myosin VIIa (h*MYO7A*) cDNA sequence is driven by either an SFFV or a CAGs promoter. For initial tests, the transgene is combined with a dTomato reporter via an IRES sequence (i2.dTomato) to facilitate identification of transduced cells. (B) Titer of VSV-G pseudotyped particles produced with the vector variants shown in A. Titers were determined based on flow cytometry of transduced cells, expressed as transducing units per milliliter (TU/mL), for preparations including the dTomato reporter, and based on qPCR quantifying the number of vector copies in transduced cells, expressed as infectious genomes per milliliter (IG/mL), for preparations devoid of the dTomato reporter.

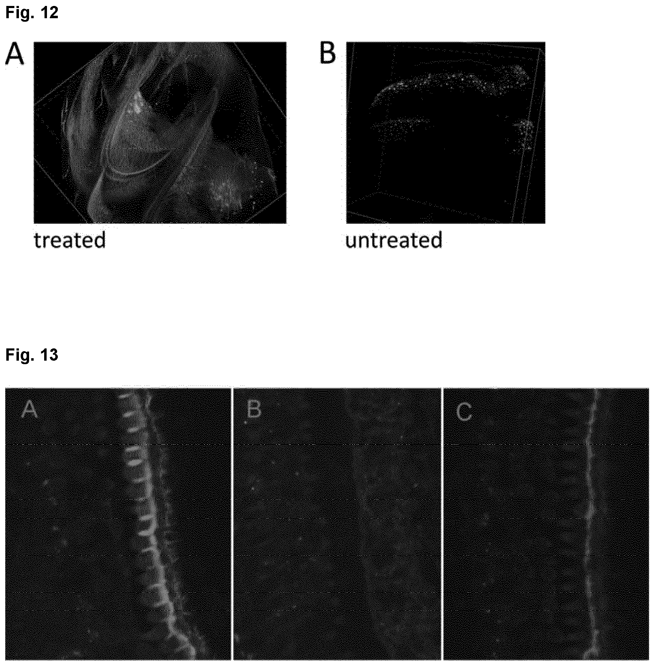

- **Fig. 12**: LV vector particles delivering MYO7A efficiently transduce the murine inner ear. (A) VSV-G pseudotyped particles delivering hMYO7A linked to a dTomato reporter, with their expression driven by the CAGs promoter (see figure 11), were administered via canalostomy. The cochleae were removed at 2 months post-delivery, cleared and expression of the dTomato protein determined to characterize the transduction pattern. Both inner and outer hair cells and SGN are dTomato-positive, indicating successful transduction. (B) Untreated control processed analogously to the cochleae of treated animals. Some false-positive cells are present in the stria vascularis.
- **Fig. 13**: Lentiviral delivery of MYO7A results in survival of hair cells in a murine *Myo7a* knock-out (KO) hearing loss mouse model. *Shaker-1* mice were injected with VSV-G pseudotyped lentiviral particles delivering hMYO7A at day 21 of age. Cochleae were removed at 2 months of age. (A-C) Confocal image of phalloidin staining of actin in inner hair cells of midturn whole mount cochleae. (A) *Shaker-1* ^{+/-} (heterozygous for Myosin VIIa KO); (B) *Shaker-1* ^{-/-} (homozygous KO), untreated: a complete loss of hair cells is seen; (C) *Shaker-1* ^{-/-} (homozygous KO), lentivirally treated:survival of hair cells upon LV therapy.

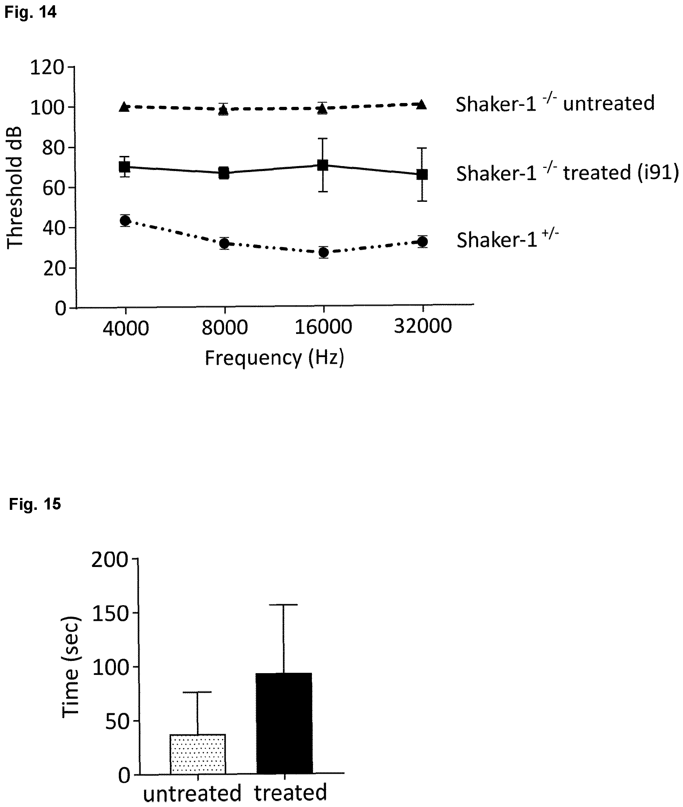

- **Fig. 14**: LV MYO7A gene therapy arrests hearing loss progression. *Shaker-1* mice were injected with VSV-G pseudotyped lentiviral particles delivering hMYO7A (i91) or left untreated. Auditory brainstem recordings (ABR) were performed to determine the threshold dB at different sound frequencies. Circles: heterozygous *Shaker-1* ^{+/-}, 1 month old; squares: homozygous KO *Shaker-1* ^{-/-} treated at 21 days of age, 1 month post-treatment; triangles: untreated homozygous KO *Shaker-1* ^{-/-}, 50 days old. n=3 within each group.
- **Fig. 15**: LV MYO7A gene therapy arrests loss of balance function. Homozygous KO *Shaker1* ^{-/-} mice were injected with VSV-G pseudotyped lentiviral particles delivering *hMYO7A* or left untreated. Balance was tested on a rotarod, expressed as the time (sec) animals managed to stay on the rotarod. Dotted bar: untreated at day 50 of age; black, solid bar: treated at day 21 of age and analyzed 1 month post-treatment. n=12 per group.

### Example

In the following example, the preparation of pseudotyped lentiviral vectors and their use for effective transduction of sensory hair cells and spiral ganglion neurons are demonstrated.

### Materials and Methods

### HEK293T cell culture

### Materials

| | |
|---|---|
| Basic culture medium | Dulbecco's Modified Eagle's Medium (DMEM) |
| Fetal bovine serum (FBS) | heat-inactivated (h.i.) for 30min @ 56°C |
| Penicillin/Streptomycin (Pen/Strep) | 10,000 U Penicillin; 10mg/ml Streptomycin |
| Sodium pyruvate | 100mM |
| Phosphate-buffered saline (PBS) | 1x |
| Trypsin / EDTA [10x] | 0.5% / 0.2% in PBS |
| | |
| Complete culture medium | DMEM+10% (v/v) h.i. FBS+1% (v/v) Pen/Strep +1% (v/v) sodium pyruvate |

### Procedure

- HEK293T cells are kept in complete culture medium at 37°C in the presence of 5% CO₂
- Cells are passaged every 2^{nd} - 3^{rd} day
   - cells are rinsed with 1xPBS
   - cells are detached by treatment with 1x trypsin/EDTA and incubation for min @ 37°C
   - the reaction is stopped with standard culture medium
   - the culture is split at the respective ratio (usually 1:8-1:12)

### Viral vector production

### Materials

| | |
|---|---|
| HEPES | 1M |
| Chloroquine | 25mM |
| HeBS (2x) | Ultra-pure water, 50mM HEPES, 280mM NaCl, 1.5mM Na₂HPO₄ |
| Calcium chloride (CaCl₂) | 2.5M |
| Lentiviral vector transfer plasmid | according to the vector to be packaged |
| Lentiviral Gag/Pol expression plasmid | pcDNA3.GP.4xCTE |
| Envelope glycoprotein expression plasmid | see list of Env GPs |
| Rev expression plasmid | pRSV-Rev |
| Filters | Millex-GP syringe filter (0.22 µm) |

| **Envelope glycoprotein (EnvGP)** | **Env G**P **packaging plasmid** |
|---|---|
| VSV-G | pMD.G |
| VSV-G ts | CMV.VSVg_thermos.IRES.Puro |
| COCV-G | CMV.MPMV.ASLV.CocaIV |
| MARAV-G | phCMV-Maraba-G |
| PIRYV-G | phCMV.PIRYV-G.iPuro 2 |
| Ampho | phCMV-A-MLVenv |
| BaEV | pcDNA3.BaEV |
| BaEV\TR | pcDNA3.BaEV-TR |
| RD114 | phCMV-RD114 |
| RD114\TR | phCMV-RD114-TR |

### Procedure

- Day 0: Cell seeding
   HEK293T cells are seeded on tissue-culture-grade plastic dishes in standard culture medium
- Day 1: Transient transfection of packaging components
   - All packaging components are combined in sterile water with the Gag/Pol packaging construct, the Env plasmid, the Rev plasmid and the vector plasmid in a ratio of 13-16 : 1-7 : 6-8 : 6-8
   - 10% (v/v) [250 mM] calcium chloride are added and mixed well
   - the DNA/CaCl₂ mixture is added dropwise to an equal volume of 2xHeBS while generating air bubbles with an electronic pipette filler, during addition of the DNA mix and for another 30 seconds
   - the mixture is incubated for 15-20 minutes at room temperature
   - meanwhile, the medium on the cultured cells is replaced by fresh complete culture medium supplemented with 1-2% (v/v) [10-20 mM] of HEPES and 0.1% (v/v) [25 µM] chloroquine
   - after the 20 min incubation, the transfection mix is dropwise added to the cells
   - the cells are cultured at 37°C and 5% CO₂
   - 6-16 hours post-transfection, the medium is replaced by fresh complete culture medium supplemented with 10-20 mM HEPES
- Day 3: Harvest I
   - 30-36 hours post-transfection, the medium containing produced lentiviral particles is harvested and passed through a 0.22 µM pore size filter
   - fresh complete culture medium supplemented with 10-20 mM HEPES is to the transfected cells for a second harvest
- Day 4: Harvest II
   - 48-54 hours post-transfection, the second harvest is accomplished as per the procedure of the first harvest
   - the two harvests (I and II) from each plate are pooled
   - the viral vector preparation is either immediately frozen at -80°C without further concentration or concentrated using ultracentrifugation

### Viral vector concentration and reconstitution

### Procedure

• The viral vector preparation is loaded into SW28 polyallomer ultracentrifuge tubes
• For concentration, the viral vector particles are spun at 4°C at different speeds and runtimes, depending on the pseudotype (see table)

| **Envelope glycoprotein (Env GP)** | **Env GP packaging plasmid** |
|---|---|
| VSV-G | pMD.G |
| VSV-G ts | CMV.VSVg_thermos.IRES.Puro |
| COCV-G | CMV.MPMV.ASLV.CocaIV |
| MARAV-G | phCMV-Maraba-G |
| PIRYV-G | phCMV.PIRYV-G.iPuro 2 |
| Ampho | phCMV-A-MLVenv |
| BaEV | pcDNA3.BaEV |
| BaEV/TR | pcDNA3.BaEV-TR |
| RD114 | phCMV-RD114 |
| RD114/TR | phCMV-RD114-TR |

• The supernatant is decanted off and the pellet containing the viral particles is resuspended in reconstitution buffer consisting of PBS supplemented with 1-2% 1 M HEPES
• The volume used for resuspension is usually chosen to concentrate the vector preparation 50-300 fold
• Aliquots are prepared and stored at -80°C until further usage

### In vivo application of lentiviral vector particles

### Procedure

Adult mice are anesthetized with an intraperitoneal (IP) injection of a mixture of ketamine (150 mg/kg), xylocaine (6 mg/kg) and acepromazine (2 mg/kg) in sodium chloride 0.9%. A dorsal postauricular incision is made, and the posterior semicircular canal exposed. Using a microdrill, a canalostomy is created, exposing the perilymphatic space. Subsequently, 1 µL of vector is injected using a Hamilton microsyringe with 0.1 µL graduations and a 36 gauge needle. The canalostomy is sealed with bone wax, and the animals are allowed to recover for 72 hours. The mice showed no signs of vestibular dysfunction or head tilt postoperatively.

### Potential human delivery:

Three main routes can be considered for delivery into the inner ear in humans. Vector can be delivered through the stapes footplate using standard stapedectomy approaches. This delivers to the vestibular organ and scala vestibuli. After elevating the tympanic membrane, the stapes footplate is visualized. Using a CO₂ laser, a 250 µm hole is created in the footplate. An infusion catheter is placed into the stapedotomy not exceeding a depth of 0.5 mm. Infusion of vector at speeds not exceeding 10 µL/min is carried out.

Injections into the round window can be accomplished using middle ear based or posterior tympanotomy approaches. This allows delivery into the scala tympani. For the middle ear approach, the tympanic membrane is elevated, and the bony round window overhang removed with a microdrill. The round window pseudo membrane is removed. At this point, the round window can be injected. For the round window, microinjection devices as well as a cochlear catheter developed by MedEI GmbH may be used.

Finally, in humans, delivery could also be carried out into the scala media by injecting into the endolymphatic sac. After completing a mastoidectomy, the facial nerve and posterior semicircular canal are identified. The endolymphatic sac is identified and can then be accessed. All of these approaches are based on standardized ontological surgical procedures and use standard operating room equipment and anaesthesia.

### Results

The improved lentiviral vector system illustrated in Figure 1 was used to efficiently transduce HEI-OC1 cells, which are a cochlear HC-like cell line (Figure 2). Transduction efficiencies of over 40% and 80% were achieved with low MOI (multiplicities of infection) and transgene expression was stable over at least 39 days (Figure 2).

Lentiviral vector particles can be efficiently pseudotyped with different envelope glycoproteins, including VSV-G, VSV-Gts, COCV-G, MARAV-G, PIRYV-G, EboZ and Ampho (Figure 3). Using the dTomato fluorescence protein as a reporter for cell transduction efficiency, lentiviral vector particles pseudotyped with the above named envelope glycoproteins were directly compared in HEI-OC1 cells. Flow cytometric analyses showed that lentiviral vector particles pseudotyped with different envelope glycoproteins differed in their transduction efficiencies, in descending order of cell transduction efficiency (% dTomato expressing cells) and transgene expression level (MFI of dTomato in dTomato⁺ cells): MARAV-G, COCV-G, VSV-G, VSV-Gts, PIRYV-G, Ampho and EboZ (Figure 4).

Cell entry blocking experiments demonstrated that lentiviral particles pseudotyped with VSV-G, VSV-G ts, COCV-G and MARAV-G use the LDL-receptor (LDL-R) for cell entry while PIRYV-G-mediated cell entry may not involve LDL-R (Figure 5).

Lentiviral vector particles engineered to express EGFP and pseudotyped with the VSV-G envelope glycoprotein efficiently transduced primary rat SGN *in vitro,* with transduction of over 20% of SGN at low MOI and over 80% at higher MOI as demonstrated by flow cytometric analyses of EGFP (Figure 6A). Furthermore, EGFP transgene expression in transduced SGN is clearly shown by immunofluorescence microscopy (Figure 6B). Immunohistochemical staining for the SGN marker neurofilament showed successful transduction of SGN with a lentiviral vector designed to express dTomato (Figure 6C).

Similarly to the data shown above in Figure 4, lentiviral vectors that encode the dTomato fluorescence protein as a reporter for cell transduction efficiency transduced primary SGN ex *vivo* cultures with different efficiency depending upon the envelope glycoprotein used to pseudotype the lentiviral vector particles (Figure 7). Flow cytometric analyses showed that lentiviral vector particles pseudotyped with different envelope glycoproteins had good transduction efficiencies, except for those pseudotyped with the EboZ glycoprotein, but resulted in different levels of transgene expression, with highest transgene expression with MARAV-G pseudotyped particles, followed by those pseudotyped with VSV-G, COCV-G, VSV-Gts, PIRYV-G, Ampho and EboZ envelope glycoproteins (Figure 7).

VSV-G pseudotyped lentiviral vectors containing either EGFP or dTomato as transgene efficiently transduced primary rat organ of Corti explants as shown by fluorescence microscopy photographs (Figure 8). Note that EGFP and dTomato are strongly expressed throughout the respective explants (Figure 8).

Efficient *in vivo* transduction of mouse SGN and HC was observed upon *in vivo* injection of a lentiviral vector encoding a dTomato reporter protein as transgene (Figure 10).

Lentiviral vector particles designed to deliver the human Myosin VIIa (*MYO7A*) as a transgene to treat hearing loss due to Myosin VIIa deficiency were successfully produced (Figure 11). Lentiviral vector titers ranged from 1 x 10⁷- 3x10⁷ TU/mL and 2 x 10⁷-4x10⁷ IG/mL (Figure 11).

Murine inner ear cells, including IHC and OHC, were efficiently transduced with lentiviral vector particles engineered to express *MYO7A* as a transgene with dTomato as a reporter (Figure 12). Lentiviral particles were administered *in vivo* via canalostomy and transgene expression was driven by the CAGs promoter. Lentiviral delivery of *MYO7A* led to increased survival of inner ear hair cells in the *Shaker-1* mouse model of hearing loss due to *Myo7a* knock-out (Figure 13), which further demonstrates the gene therapy potential of lentiviral vector particles. Amelioration of the *Shaker-1* mouse phenotype by lentiviral vector delivery of *MYO7A* was demonstrated by auditory brainstem recordings (ABR) to determine the hearing level (e.g. threshold dB) at various sound frequencies. A lower threshold dB indicates a better hearing capacity at a given frequency. *Shaker-1* mice treated with the MYO7A-expressing lentiviral vector (i91) had vastly better hearing as compared to the untreated controls, indicating that the gene transfer inhibited the progressive hearing loss common to these mice (Figure 14). Additionally, lentiviral vector gene therapy to deliver *MYO7A* arrested the loss of balance function as treated animals were able to balance on a rotarod approximately two times longer than untreated mice (Figure 15).

### References

Blanc et al. (2020) rAAV-Mediated Cochlear Gene Therapy: Prospects and Challenges for Clinical Application. J. Clin Med. 9(2), 589.
Izumikawa et al. (2005) Auditory hair cell replacement and hearing improvement by Atoh1 gene therapy in deaf mammals. Nature Medicine 11(3), 271-276.
Tao et al. (2018) Delivery of Adeno-Associated Virus Vectors in Adult Mammalian Inner-Ear Cell Subtypes Without Auditory Dysfunction. Human Gene Therapy 29(4), 492-506.
Dong et al. (1996) Quantitative Analysis of the Packaging Capacity of Recombinant Adeno-Associated Virus. Human Gene Therapy 7, 2101-2112.
Milone and O'Doherty (2018) Clinical use of lentiviral vectors. Leukemia 32, 1529-1542. Kumar et al. (2001) Systematic Determination of the Packaging Limit of Lentiviral Vectors. Human Gene Threapy 12, 1893-1905.
Schambach et al. (2013) Biosafety Features of Lentiviral Vectors. Human Gene Therapy 24, 132-142.
Zhang et al. (2001) A highly efficient and consistent method for harvesting large volumes of high-titre lentiviral vectors. Gene Therapy 8, 1745-1751.
Hashimoto et al. (2007) Lentiviral gene replacement therapy of retinas in a mouse model for Usher syndrome type 1B. Gene Therapy 14, 584-594.
Han et al. (1999) Transgene Expression in the Guinea Pig Cochlea Mediated by a Lentivirus-Derived Gene Transfer Vector. Human Gene Therapy 10, 1867-1873.
Pietola et al. (2009) HOX-GFP and WOX-GFP lentivirus vectors for inner ear gene transfer. Acta Oto-Laryngologica, 128, 613-620.
Yan Wei et al. (2013) Effect of lentiviruses carrying enhanced green fluorescent protein injected into the scala media through a cochleostomy in rats. American Journal of Otolaryngology-Head and Neck Medicine and Surgery 34, 301-307.
Morgan et al. (2020) Gene therapy as a possible option to treat hereditary hearing loss. Medizinische Genetik.
ACMG Working Group on Update of Genetics Evaluation Guidelines for the Etiologic Diagnosis of Congenital Hearing Loss; for the Professional Practice and Guidelines Committee (2014) American College of Medical Genetics and Genomics guideline for the clinical evaluation and etiologic diagnosis of hearing loss. Genetics in Medicine 16, 347-355.
Cabanillas et al. (2018) Comprehensive genomic diagnosis of non-syndromic and syndromic hereditary loss in Spanish patients. BMC Medical Genomics 11, 58.
German Advisory Committee Blood (Arbeitskreis Blut), Subgroup 'Assessment of Pathogens Transmissible by Blood' (2016) Human Immunodeficiency Virus (HIV). Transfus Med Hemother. 43(3), 203-222.
Naldini et al., (1996) In vivo gene delivery and stable transduction of nondividing cells by a lentiviral vector. Science 272(5259). 263-267.
Zufferey et al. (1998) Self-Inactivating Lentivirus Vector for Safe and Efficient in Vivo Gene Delivery. J Virol 72(12), 9873-9880.
Iwamoto et al. (2010) A general chemical method to regulate protein stability in the mammalian centralnervous system. Chem Biol. 17(9), 981-988.
Levy et al. (1999) Analysis of a conditional degradation signal in yeast and mammalian cells. Eur J Biochem. 259(1-2), 244-252.
Stankunas et al. (2003) Conditional protein alleles using knockin mice and a chemical inducer of dimerization. Mol Cell. 12(6), 1615-1624.
Catalanotto et al. (2016) MicroRNA in Control of Gene Expression: An Overview of Nuclear Functions. International journal of molecular sciences, 17(10), 1712.
Sage et al. (2005) Proliferation of functional hair cells in vivo in the absence of the retinoblastoma protein. Science 307(5712) 1056-1058.
Shaw and Cornetta (2014) Design and Potential of Non-Integrating Lentiviral Vectors. Biomedicines 2(1), 14-35.
Agnello et al. (1999) Hepatitis C virus and other flaviviridae viruses enter cells via a low density lipoprotein receptor. Proc Natl Acad Sci 96(22), 12766-12771.
Finkelshtein et al. (2013) LDL receptor and its family members serve as the cellular receptors for vesicular stomatitis virus. PNAS 110(18), 7306-7311.
Hosokawa et al. (2018) Immunohistochemical localization of megalin and cubilin in the human inner ear. Brain Res. 1701, 153-160.
Trobridge et al. (2010) Cocal-pseudotyped Lentiviral Vectors Resist Inactivation by Human Serum and Efficiently Transduce Primate Hematopoietic Repopulating Cells. Molecular Therapy 18(4), 725-733.
Scalise et al. (2018) The Human SLC1A5 (ASCT2) Amino Acid Transporter: FROM Function to Structure and Role in Cell Biology. Front Cell Dev Biol. 6(96).
Marin et al. (2003) N-Linked Glycosylation and Sequence Changes in a Critical Negative Control Region of the ASCT1 and ASCT2 Neutral Amino Acid Transporters Determine Their Retroviral Receptor Functions. Journal of Virology 77(5), 2936-2945.
Sandrin et al. (2002) Lentiviral vectors pseudotyped with a modified RD114 envelope glycoprotein show increased stability in sera and augmented transduction of primary lymphocytes and CD34+ cells derived from human and nonhuman primates. Blood 100(3), 823-32.
Girard-Gagnepain et al. (2014) Baboon envelope pseudotyped LVs outperform VSV-G-LVs for gene transfer into early-cytokine-stimulated and resting HSCs. Blood 124(8), 1221-1231.
Tijani et al. (2018) Lentivector Producer Cell Lines with Stably Expressed Vesiculovirus Envelopes. Mol Ther Methods Clin Dev. 10, 303-312.
Schambach et al. (2006) Equal potency of gammaretroviral and lentiviral SIN vectors for expression of O6-methylguanine-DNA methyltransferase in hematopoietic cells. Mol. Ther. 13(2), 391-400.
Meas et al. (2018) Reprogramming Glia Into Neurons in the Peripheral Auditory System as a Solution for Sensorineural Hearing Loss: Lessons From the Central Nervous System. Front Mol Neurosci. 11: 77.
Malgrange et al. (2002) Epithelial supporting cells can differentiate into outer hair cells and Deiters' cells in the cultured organ of Corti. Cellular and Molecular Life Sciences 59, 1744-1757.
Hendricks et al. (2008) Localized Cell and Drug Delivery for Auditory Prostheses. Hear Res. 242(1-2), 117-131.
Shibata et al. (2017) Intravenous rAAV2/9 injection for murine cochlear gene delivery. Sci. Rep. 7, 9609.

## Claims

1. A composition for use in treating sensorineural hearing loss in a subject, wherein the composition comprises a 3^{rd} generation lentiviral vector pseudotyped with a viral envelope glycoprotein capable of binding to a receptor expressed in a cell of the inner ear, wherein said composition has a titer of at least 10⁷TU/mL and is administered to the inner ear of the subject.

2. The composition for use of claim 1, wherein the lentiviral vector is pseudotyped with a viral envelope glycoprotein capable of binding to a receptor selected from the group consisting of the LDL-receptor and LDL-R family members, the SLC1A5-receptor, the Pit1/2-receptor and the PIRYV-G-receptor.

3. The composition for use of any of claims 1-2 wherein the lentiviral vector is pseudotyped with a viral envelope glycoprotein capable of binding the LDL-receptor, wherein the viral envelope glycoprotein is selected from the group comprising MARAV-G, COCV-G, VSV-G and VSV-G ts.

4. The composition for use of claim 3, wherein the viral envelope glycoprotein is MARAV-G.

5. The composition for use of claim 3, wherein the viral envelope glycoprotein is COCV-G.

6. The composition for use of claim 3, wherein the viral envelope glycoprotein is VSV-G, such as wild-type VSV-G or VSV-G ts.

7. The composition for use of any of the preceding claims, wherein the lentiviral vector comprises a cargo sequence comprising a nucleic acid selected from the group comprising a protein-coding gene, a miRNA, an shRNA, a IncRNA, or an sgRNA,
wherein expression of said cargo sequence in said cell of the inner ear is capable of reducing or eliminating at least a symptom of sensorineural hearing loss in the subject.

8. The composition for use of any of the preceding claims, wherein the cargo sequence is a gene selected from the group comprising *MYO7A, GJB2, OTOF, ATOH1, SLC26A4, TMC1, TMPRSS3, GIPC3, USH1C, RDX, CLDN14, WHRN, ESPN, ILDR1, SERPINB6, KCNQ4, POU4F3, SIX1, CCDC50, MIR96, POU3F4, EYA1, KCNQ1, GJB6, STRC, TECTA, GJB3, PRPS1, CDH23, GRXCR2, CABP2, GRXCR1, SYNE4, COL11A2, LRTOMT, BSND, PJVK, CIB2, CEACAM16, TMIE, NARS2, LHFPL5, MARVELD2, SLC22A4, KARS, SLC26A5, TPRN, ELMOD3, GPSM2, COCH, ESRRB, ADCY1, EPS8L2, CD164, OTOA, S1PR2, LOXHD1, TSPEAR, EPS8, CDC14A, MSRB3, DCDC2, PNPT1, TMEM132E, FAM65B, MYO3A, CLIC5, HGF, USH2A, TBC1D24, MET, PCDH15, PTPRQ, OTOGL, MYO6, TNC, TRIOBP, BDP1, MYO15A, DIAPH1, MYH14, DFNA5, WFS1, EYA4, COL11A2, MYH9, ACTG1, SLC17A8, GRHL2, DSPP, P2RX2, MYO1A, GFI1, TYMP, GFI1B, BDNF, GDNF, NT-3, IGF1, IGF2, CNTF, ARTN, NRTN, ATP7B, MRP1, MRP2* and *MDR1.*

9. The composition for use of any of the preceding claims, wherein the lentiviral vector is derived from a virus selected from the group comprising HIV-1, HIV-2, SIV, FIV and EIAV, wherein, preferably, the lentiviral vector is derived from HIV-1.

10. The composition for use of any of the preceding claims, wherein the lentiviral vector comprises
a) a 5' LTR comprising a constitutively active heterologous promoter at the U3 position, a repeat region (R) and a U5 region,
b) a 5' UTR comprising a primer binding site (PBS), a splice donor site (SD), a packaging signal (ψ), a Rev-responsive element, and, optionally, a splice acceptor (SA) site.
c) an internal enhancer/promoter region operably linked to a cargo sequence,
d) RNA processing elements optionally comprising a Woodchuck hepatitis virus posttranscriptional regulatory element (PRE), and
e) a 3' LTR with a deleted U3 (SIN) region, a repeat region (R) and a U5 region.

11. The composition for use of any of the preceding claims, wherein the cell of the inner ear is a cell selected from the group consisting of cells of the organ of Corti including supporting cells as well as inner and outer hair cells, spiral ganglion neurons and glial cells, cells of the stria vascularis and spiral ligament, lateral wall fibrocytes, type I and II vestibular hair cells, vestibular supporting cells and vestibular ganglion neurons.

12. The composition for use of any of the preceding claims, wherein the lentiviral vector titer is concentrated using ultracentifugation or other purification methods, incl. microfiltration, ultrafiltration, chromatography, density gradient ultracentrifugation, tangential flow microfiltration, and precipitation.

13. The composition for use of any of the preceding claims, wherein the composition is administered to the subject by a method selected from the group comprising a cochlea implant route, round window injection, oval window injection, canalostomy, cochleostomy and injection into the endolymphatic sac.

14. The composition for use of any of the preceding claims, wherein the lentiviral vector is a non-integrating lentiviral vector.

15. A method for treating sensorineural hearing loss in a subject in need thereof, comprising the step of administering an effective amount of the composition of any of claims 1-14 to said subject.
